# EUROPEAN PATENT SPECIFICATION

(11) **EP 4 045 150 B1**
(45) Date of publication and mention of the grant of the patent: **26.11.2025**
(21) Application number: 20793633.7
(22) Date of filing: 16.10.2020
(51) Int. Cl.: A61P 35/00, A61K 39/00, A61K 35/17

(54) **IMMUNOGENIC COMPOUNDS FOR TREATMENT OF ADRENAL CANCER**
IMMUNOGENE VERBINDUNGEN ZUR BEHANDLUNG VON NEBENNIERENKREBS
COMPOSÉS IMMUNOGÈNES POUR LE TRAITEMENT DU CANCER DES SURRÉNALES

(30) Priority: 16.10.2019 EP 19306351
(43) Date of publication of application: 24.08.2022
(73) Proprietor: Enterome S.A., 75011 Paris (FR)
(72) Inventor: CHENE, Laurent, 45170 Neuville aux Bois (FR); FAGERBERG, Jan, 75012 Paris (FR)
(74) Representative: Regimbeau
(86) International application number: PCT/EP2020/079226
(87) International publication number: WO 2021/074389

(56) References cited:
- WO-A1-2013/148147
- WO-A1-2017/203526
- WO-A2-2019/072871
- CLAUDIA PAPEWALIS ET AL: "Chromogranin A as potential target for immunotherapy of malignant pheochromocytoma", MOLECULAR AND CELLULAR ENDOCRINOLOGY, ELSEVIER IRELAND LTD, IE, vol. 335, no. 1, 29 May 2010 (2010-05-29), pages 69 - 77, XP028144098, ISSN: 0303-7207, [retrieved on 20100622], DOI: 10.1016/J.MCE.2010.05.021

## Description

The present invention relates to the field of cancer immunotherapy. In particular, the present invention relates to antigen-based immunotherapy targeting interleukin-13 receptor α2 (IL13RA2) and BIRC5 for reducing, ameliorating, preventing or treating adrenal cancer or for reducing or preventing its recurrence.

Adrenal cancers are cancers of the adrenal glands. The adrenal glands are small glands located on top of each kidney (suprarenal) and each adrenal gland has two parts, the cortex (outer part) and the medulla (inner part). Cancers of the cortex of the adrenal glands are also referred to as "adrenocortical carcinoma" (ACC). About two out of three adrenal cortical carcinomas cause symptoms by producing high levels of one of the adrenal cortex hormones, such as glucocorticoids, mineralocorticoids, and sex hormones. Cancers of the adrenal medulla include neuroblastomas of the adrenal medulla and pheochromocytomas. Pheochromocytomas are catecholamine-producing neuroendocrine tumors arising from chromaffin cells of the adrenal medulla. As the adrenal glands are responsible for hormone production with the adrenal cortex producing glucocorticoids, mineralocorticoids, and sex hormones and the adrenal medulla producing catecholamines, such as epinephrine, norepinephrine and dopamine, adrenal cancers often cause imbalances in hormone production, which can result in severe conditions, such as Cushing's syndrome, Conn's syndrome (aldosteronism), arrhythmias, hypertensive crises and anxiety.

While adrenal cancers are rare, they are highly aggressive and potentially deadly malignancies. Adrenal cancers are diagnosed in approximately 1-2 people per 1 million population each year, most commonly in young adults or in children under the age of 6. Surgical resection of the tumor is of utmost importance in the treatment of adrenal cancers. When the tumor is found at the early stage and can be removed surgically, the five-year survival rate is 50-60 percent. However, in more than two-thirds of the cases, the tumor has spread beyond the adrenal gland by the time it is discovered, thereby dampening the prognosis. The prognosis for adrenal cancers that have spread to nearby or distant organs is much less favorable, with only 10-20 percent surviving five years. For patients with locally advanced or metastatic adrenal cancer, not amenable to surgery, mitotane and cytotoxic chemotherapy (with etoposide, doxorubicin and cisplatin - EDP scheme) are the systemic treatments currently in use. However, no effective second-line therapies are available for patients with disease progression to EDP and mitotane. Moreover, more than half of the patients, who have undergone complete removal of the tumor are destined to have a relapse, often with metastases. The need for new therapies for adrenal cancer is strongly motivated by the aggressive behavior and the high recurrence rate of adrenal cancers.

In the recent years, cancer immunotherapy emerged as powerful tool to combat cancers. However, while cancer immunotherapy was shown to be successful for various kinds of cancers and resulted in approval of respective medicaments by the competent authorities, the success of cancer immunotherapy for adrenal cancer was very limited so far (Cosentini D, Grisanti S, Dalla Volta A, Laganà M, Fiorentini C, Perotti P, Sigala S, Berruti A. Immunotherapy failure in adrenocortical cancer: where next? Endocr Connect. 2018 Nov 1;7(12):E5-E8. doi: 10.1530/EC-18-0398; Fiorentini C, Grisanti S, Cosentini D, Abate A, Rossini E, Berruti A, Sigala S. Molecular Drivers of Potential Immunotherapy Failure in Adrenocortical Carcinoma. J Oncol. 2019 Apr 1;2019:6072863. doi: 10.1155/2019/6072863). Papewalis et al. studied peptides derived from chromogranin A for their use in treating malignant phaeochromocytoma (Papewalis et al., Molecular and Cellular Endocrinology 2010, 335(1), 69-77). WO2017203526 reports the frequent presence of high expression levels of various antigens found in testicular cancer, including IL13RA2, in other tumors, and the use of these 'testicular cancer antigens' (TCA) as a signature in cancer diagnostic methods. WO2013148147 discloses the sequences of certain genes frequently methylated in a particular way (methylation of the cytosine residue on the 'CpG' sites) in the case of adrenal gland neoplasms, and the use of this methylation profile in methods for diagnosing adrenocortical gland neoplasms. IL13RA2 is mentioned among other genes with a particularly high level of CpG methylation in adrenal gland neoplasms. WO 2019/072871 teaches microbiota variant peptides related to human tumor antigens, including IL13RA2.

Accordingly, there is a need for new immunotherapies for adrenal cancer. For example, in view of the immunosuppressive effects of increased glucocorticoid release often observed in adrenal cancers, there is a need for compounds exhibiting increased immunogenicity.

This object is achieved by means of the subject-matter set out below, in particular in the items provided by the present invention and in the appended claims.

The invention is described in more detail below.

### DEFINITIONS

Unless otherwise defined herein, scientific and technical terms used in the present application shall have the meanings that are commonly understood by those of ordinary skill in the art. Further, unless otherwise required by context, nomenclatures used herein, and techniques of cell and tissue culture are those well-known and commonly used in the art.

Such techniques are fully explained in the literature, such as Owen et al. (Kuby Immunology, 7th, edition, 2013 - W. H. Freeman) and Sambrook et al. (Molecular cloning: A laboratory manual 4th edition, Cold Spring Harbor Laboratory Press - Cold Spring Harbor, NY, USA, 2012).

Nevertheless, with respect to the use of different terms throughout the current specification, the following definitions more particularly apply.

The term "(poly)peptide" as used herein refers to a peptide and/or to a polypeptide. The terms "peptide", "polypeptide", "protein" and variations of these terms refer to peptides, oligopeptides, polypeptides, or proteins comprising at least two amino acids joined to each other preferably by a normal peptide bond, or, alternatively, by a modified peptide bond, such as for example in the cases of isosteric peptides. In particular, the terms "peptide", "polypeptide" and "protein" refer to a sequential chain of amino acids of any length linked together via peptide bonds (-NHCO-).

Peptides, polypeptides and proteins can play a structural and/or functional role in a cell *in vitro* and/or *in vivo.* The terms "peptide", "polypeptide", "protein" preferably encompass amino acids chains in size ranging from 2 to at least about 1000 amino acid residues. The term "peptide" preferably encompasses herein amino acid chains in size of less than about 30 amino acids, while the terms "polypeptide" and "protein" preferably encompass amino acid chains in size of at least 30 amino acids. The terms "polypeptide" and "protein" are used herein interchangeably. In a preferred embodiment, the terms "peptide", "polypeptide", "protein" also include "peptidomimetics" which are defined as peptide analogs containing non-peptidic structural elements, which peptides are capable of mimicking or antagonizing the biological action(s) of a natural parent peptide. A peptidomimetic lacks classical peptide characteristics such as enzymatically scissile peptide bonds. In particular, a peptide, polypeptide or protein can comprise amino acids other than the 20 amino acids defined by the genetic code in addition to these amino acids, or it can be composed of amino acids other than the 20 amino acids defined by the genetic code. In particular, a peptide, polypeptide or protein in the context of the present invention can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends. In particular, a peptide or polypeptide can be branched following an ubiquitination or be cyclic with or without branching. This type of modification can be the result of natural or synthetic post-translational processes that are well known to a person skilled in the art. The terms "peptide", "polypeptide", "protein" in the context of the present invention in particular also include modified peptides, polypeptides and proteins. For example, peptide, polypeptide or protein modifications can include acetylation, acylation, ADP-ribosylation, amidation, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, glycosylation including pegylation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York ; Post-translational Covalent Modifications of Proteins (1983) B. C. Johnson, Ed., Academic Press, New York ; Seifter et al. (1990) Analysis for protein modifications and nonprotein cofactors, Meth. Enzymol. 182: 626-646 and Rattan et al., (1992) Protein Synthesis: Post-translational Modifications and Aging, Ann NY Acad Sci, 663: 48-62). Accordingly, the terms "peptide", "polypeptide", "protein" preferably include for example lipopeptides, lipoproteins, glycopeptides, glycoproteins and the like.

In a preferred embodiment, a (poly)peptide or protein is a "classical" (poly)peptide or protein, whereby a "classical" (poly)peptide or protein is typically composed of amino acids selected from the 20 amino acids defined by the genetic code, linked to each other by a normal peptide bond.

As well-known in the art, peptides, polypeptides and proteins can be encoded by nucleic acids. The terms "nucleic acid", "nucleic acid molecule", "nucleic acid sequence", "polynucleotide", "nucleotide sequence" are used herein interchangeable and refer to a precise succession of natural nucleotides (e.g., A, T, G, C and U), or synthetic nucleotides, i.e. to a chain of at least two nucleotides. In particular, the terms "nucleic acid", "nucleic acid molecule", "nucleic acid sequence", "polynucleotide", "nucleotide sequence" refer to DNA or RNA. Nucleic acids preferably comprise single stranded, double stranded or partially double stranded DNA or RNA, preferably selected from genomic DNA, cDNA, ribosomal DNA, and the transcription product of said DNA, such as RNA. Preferred examples of nucleic acids include rRNA, mRNA; antisense DNA, antisense RNA; complimentary RNA and/or DNA sequences, ribozyme, (complementary) RNA/DNA sequences with or without expression elements, a vector; a mini-gene, gene fragments, regulatory elements, promoters, and combinations thereof. Further preferred examples of nucleic acid (molecules) and/or polynucleotides include, e.g., a recombinant polynucleotide, a vector, an oligonucleotide, an RNA molecule such as an rRNA, an mRNA, or a tRNA, or a DNA molecule as described above. It is thus preferred that the nucleic acid (molecule) is a DNA molecule or an RNA molecule; preferably selected from genomic DNA; cDNA; rRNA; mRNA; antisense DNA; antisense RNA; complementary RNA and/or DNA sequences; RNA and/or DNA sequences with or without expression elements, regulatory elements, and/or promoters; a vector; and combinations thereof. It is within the skill of the person in the art to determine nucleotide sequences which can encode a specific amino acid sequence.

The (poly)peptides and/or nucleic acids according to the invention may be prepared by any known method in the art including, but not limited to, any synthetic method, any recombinant method, any *ex vivo* generation method and the like, and any combination thereof. Such techniques are fully explained in the literature as mentioned above.

As used herein, a "sequence variant" is similar, but contains at least one alteration, in comparison to a reference sequence. A "sequence variant" is as defined herein may have an (amino acid) sequence which is at least 70%, preferably at least 75%, more preferably at least 80%, even more preferably at least 85%, still more preferably at least 90%, and most preferably at least 95% identical to a reference sequence. In some embodiments, the sequence variant exhibits one or two mutations, e.g. substitutions, deletions or additions (of one or two amino acids) as compared to the reference sequence. With regard to the peptides of the invention, it is preferred in a sequence variant that the core sequence as defined herein below is maintained (i.e., that mutations occur only outside the core sequence). A sequence variant may preserve the specific function of the reference sequence. In the context of the present invention, this function may be the functionality as an "epitope", i.e. it can be recognized by the immune system, in particular by antibodies, T cell receptors, and/or B cell receptors and, preferably, it can elicit an immune response. Accordingly, is preferred that the (poly)peptide for use according to the present invention is immunogenic. In other words, the (poly)peptide is preferably capable of eliciting an immune response.

The term "immunogenic compound" refers to a compound comprising a (poly)peptide according to the present invention. An "immunogenic compound" is able to induce, increase or maintain an immunological response against said (poly)peptide in a subject to whom it is administered. In some embodiments, immunogenic compounds comprise at least one (poly)peptide, or alternatively at least one compound comprising such an (poly)peptide, linked to a protein, which encompasses a carrier protein.

A "carrier protein" is usually a protein, which is able to transport a cargo, such as the (poly)peptide according to the present invention. For example, the carrier protein may transport its cargo across a membrane. In the context of the present invention, a carrier protein in particular (also) encompasses a peptide or a polypeptide that is able to elicit an immune response against the (poly)peptide that is linked thereto. Carrier proteins are known in the art.

Alternatively such carrier peptide or polypeptide may be co-administered in the form of immune adjuvant.

Preferably, the (poly)peptide as described herein may be co-administrated or linked, for example by covalent or non-covalent bond, to a protein/peptide having immuno-adjuvant properties, such as providing stimulation of CD4+ Th1 cells. While the (poly)peptide as described herein preferably binds to MHC class I, CD4+ helper epitopes may be additionally used to provide an efficient immune response. Th1 helper cells are able to sustain efficient dendritic cell (DC) activation and specific CTL activation by secreting interferon-gamma (IFN-y), tumor necrosis factor-alpha (TNF-α) and interleukine-2 (IL-2) and enhancing expression of costimulatory signal on DCs and T cells (Galaine et al., Interest of Tumor-Specific CD4 T Helper 1 Cells for Therapeutic Anticancer Vaccine. Vaccines (Basel). 2015 Jun 30;3(3):490-502).

For example, the adjuvant peptide/protein may preferably be distinct from the (poly)peptide according to the present invention. Preferably, the adjuvant peptide/protein is capable of recalling immune memory or provides a non-specific help or could be a specific helper peptide. Several helper peptides have been described in the literature for providing a nonspecific T cell help, such as tetanus helper peptide, keyhole limpet hemocyanin peptide or PADRE peptide (Adotévi et al., Targeting antitumor CD4 helper T cells with universal tumor-reactive helper peptides derived from telomerase for cancer vaccine. Hum Vaccin Immunother. 2013 May;9(5):1073-7, Slingluff CL, The present and future of peptide vaccines for cancer: single or multiple, long or short, alone or in combination? Cancer J. 2011 Sep-Oct;17(5):343-50). Accordingly, tetanus helper peptide, keyhole limpet hemocyanin peptide and PADRE peptide are preferred examples of such adjuvant peptide/proteins. In particular, the antigenic peptide as described herein, or a polypeptide comprising the said antigenic peptide, may be linked, for example by covalent or non-covalent bond, to the HHD-DR3 peptide of sequence MAKTIAYDEEARRGLERGLN (SEQ ID NO: 266). This peptide represents another example of a helper peptide (having immuno-adjuvant properties), which is preferred in the context of the present invention. Another preferred example is h-pAg T13L (sequence: TPPAYRPPNAPIL; SEQ ID NO: 280; Bhasin M, Singh H, Raghava GP (2003) MHCBN: a comprehensive database of MHC binding and non-binding peptides. Bioinformatics 19: 665-666). Further examples of preferred helper peptides include the UCP2 peptide (for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2) and the BIRC5 peptide (for example as described in EP2119726 A1 or in Widenmeyer M, Griesemann H, Stevanović S, Feyerabend S, Klein R, Attig S, Hennenlotter J, Wernet D, Kuprash DV, Sazykin AY, Pascolo S, Stenzl A, Gouttefangeas C, Rammensee HG: Promiscuous survivin peptide induces robust CD4+ T-cell responses in the majority of vaccinated cancer patients. Int J Cancer. 2012 Jul 1;131(1):140-9. doi: 10.1002/ijc.26365. Epub 2011 Sep 14). The most preferred helper peptide is the UCP2 peptide (amino acid sequence: KSVWSKLQSIGIRQH; SEQ ID NO: 281, for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2).

A composition as defined herein, which further comprises one or more immuno-adjuvant substances, may also be termed an "immunogenic composition" or in some embodiments a "vaccine composition" in the present specification. As used herein, the term "immunogenic composition" refers to a composition that is able to induce or maintain an immune response, in particular which induces an immune response, when it is administered to a mammal, and especially when it is administered to a human individual.

By "pharmaceutically acceptable excipient", it is meant herein a compound of pharmaceutical grade which improves the delivery, stability or bioavailability of an active agent, and can be metabolized by, and is non-toxic to, a subject to whom it is administered. Preferred excipients according to the invention include any of the excipients commonly used in pharmaceutical products, such as, for example, water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable excipients may further comprise minor amounts of auxiliary substances such as wetting or emulsifying agents, or preservatives.

By "vaccine", it is meant herein a composition capable of stimulating the immune system of a living organism so that protection against a harmful antigen is provided, either through prophylaxis or through therapy. Prophylactic vaccines are preferred.

According to the different aspects and embodiments of the invention described herein, a "subject" or "host" preferably refers to a mammal, and most preferably to a human being. Said subject may have, been suspected of having, or be at risk of developing an adrenal cancer.

As used herein, the expression "prevention and/or treatment of an adrenal cancer" refers to ameliorating, reducing, preventing and/or treating an adrenal cancer or to reducing or preventing the recurrence of an adrenal cancer. Accordingly, the expression "prevention and/or treatment of an adrenal cancer" refers to prophylactic as well as therapeutic settings.

Prophylactic settings generally mean to avoid or minimize the onset, development or recurrence of a disease or condition before its onset or after it was "healed" (e.g., after complete surgical resection of the tumor), while therapeutic settings usually encompass reducing, ameliorating or curing a disease or condition (or symptoms of a disease or condition) after its onset. In particular, the term "preventing" encompasses "reducing the likelihood of occurrence of" or "reducing the likelihood of reoccurrence".

An "effective amount" or "effective dose" as used herein is an amount which provides the desired effect. For therapeutic purposes, an effective amount is an amount sufficient to provide a beneficial or desired clinical result. The preferred effective amount for a given application can be easily determined by the skilled person taking into consideration, for example, the size, age, weight of the subject, the type of disease/disorder to be prevented or treated, and the amount of time since the disease/disorder began. In the context of the present invention, in terms of prevention or treatment, an effective amount of the composition is an amount that is sufficient to induce a humoral and/or cell-mediated immune response directed against the disease/disorder.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 10%.

Additional definitions are provided throughout the specification.

The present invention may be understood more readily by reference to the following detailed description, including preferred embodiments of the invention, and examples included herein.

### (Poly)peptide comprising an IL13RA2 epitope or a sequence variant thereof

The present disclosure provides a (poly)peptide, not encompassed by the wording of the claims, comprising or consisting of an epitope of IL13RA2 or a sequence variant thereof having at least 70% (preferably at least 75%, more preferably at least 80%, even more preferably at least 85%, and most preferably at least 90%) sequence identity for use in prevention and/or treatment of an adrenal cancer. Accordingly, the present disclosure also provides a method, not encompassed by the wording of the claims, for ameliorating, reducing, preventing and/or treating an adrenal cancer or for reducing or preventing its recurrence in a subject, comprising administering to the subject a (poly)peptide comprising an epitope of IL13RA2 or a sequence variant thereof having at least 70% (preferably at least 75%, more preferably at least 80%, even more preferably at least 85%, and most preferably at least 90%) sequence identity.

The term "epitope", as used herein, refers to a peptide, which can be recognized by the immune system. In general, an "epitope" (also known as "antigenic determinant"), is the part (or fragment) of an antigen that is recognized by the immune system, in particular by antibodies, T cell receptors, and/or B cell receptors. Thus, one antigen has at least one epitope, i.e. a single antigen has one or more epitopes. An "antigen" typically serves as a target for the receptors of an adaptive immune response, in particular as a target for antibodies, T cell receptors, and/or B cell receptors. In the context of the present invention the antigen may be interleukin-13 receptor alpha 2 (IL13RA2). Other antigens, which may optionally be of interest in the context of the present invention, include BIRC5 (Baculoviral IAP Repeat Containing 5) and FOXM1 (Forkhead Box M1). With regard to IL13RA2, human interleukin-13 receptor alpha 2 (IL13RA2) is preferred. The sequence of human IL13RA2 is shown in the following:

MAFVCLAIGCLYTFLISTTFGCTSSSDTEIKVNPPQDFEIVDPGYLGYLYLQWQPPLSLDHFKECTVEYELKYRNIG
SETWKTIITKNLHYKDGFDLNKGIEAKIHTLLPWQCTNGSEVQSSWAETTYWISPQGIPETKVQDMDCVYYNW
QYLLCSWKPGIGVLLDTNYNLFYWYEGLDHALQCVDYIKADGQNIGCRFPYLEASDYKDFYICVNGSSENKPIRS
SYFTFQLQNIVKPLPPVYLTFTRESSCEIKLKWSIPLGPIPARCFDYEIEIREDDTTLVTATVENETYTLKTTNETRQL
CFVVRSKVNIYCSDDGIWSEWSDKQCWEGEDLSKKTLLRFWLPFGFILILVIFVTG LLLRKPNTYPKMIPEFFCDT
[SEQ ID NO: 275]

In a particular instance not encompassed by the wording of the claims, a preferred (poly)peptide for use as disclosed herein comprises an epitope of human IL13RA2. In an instance not encompassed by the wording of the claims, a preferred (poly)peptide for use as disclosed herein may comprise an amino acid sequence according to SEQ ID NO: 275 or a fragment thereof comprising or consisting of an epitope or a sequence variant of such a fragment. As used herein, a "fragment" of an antigen comprises at least 5 consecutive amino acids of the antigen, preferably at least 6 consecutive amino acids of the antigen, more preferably at least 7 consecutive amino acids of the antigen, even more preferably at least 8 consecutive amino acids of the antigen and most preferably at least 9 consecutive amino acids of the antigen. A "sequence variant" is as defined herein, namely a sequence variant has an (amino acid) sequence which is at least 70% (preferably at least 75%, more preferably at least 80%, even more preferably at least 85%, most preferably at least 90%) identical to the reference sequence. A "functional" sequence variant means in the context of an antigen/antigen fragment/epitope, that the function of the epitope(s), e.g. comprised by the antigen (fragment), is not impaired or abolished.

In the context of the present invention, the term "epitope" is mainly used to designate T cell epitopes, which are presented on the surface of an antigen-presenting cell, where they are bound to Major Histocompatibility Complex (MHC). T cell epitopes presented by MHC class I molecules are typically, but not exclusively, peptides between 8 and 12 amino acids in length, whereas MHC class II molecules present longer peptides, generally, but not exclusively, between 12 and 25 amino acids in length. Preferably, the epitope of IL13RA2 or the sequence variant thereof has a length of 8 - 12 amino acids, more preferably of 8 - 10 amino acids and most preferably of 9 or 10 amino acids.

Several epitopes of IL13Ralpha2 are known to the skilled person and can be identified by using cancer/tumor epitope databases, e.g. from van der Bruggen P, Stroobant V, Vigneron N, Van den Eynde B. Peptide database: T cell-defined tumor antigens. Cancer Immun 2013; URL: http://www.cancerimmunity.org/peptide/, wherein human tumor antigens recognized by CD4+ or CD8+ T cells are classified into four major groups on the basis of their expression pattern, or from the database "Tantigen" (TANTIGEN version 1.0, Dec 1, 2009; developed by Bioinformatics Core at Cancer Vaccine Center, Dana-Farber Cancer Institute; URL: http://cvc.dfci.harvard.edu/tadb/).

Exemplified epitopes of IL13RA2 not encompassed by the wording of the claims, having an amino acid sequence as set forth in any one of SEQ ID NOs 243 - 265, 276 - 278 and 331 - 334 are shown in Table 1 below.

**Table 1: Examples of human IL13RA2 epitopes, not encompassed by the wording of the claims.**

| **SEQ ID NO.** | **IL13 RA2 epitope sequence** |
|---|---|
| 243 | AIGCLYTFL |
| 244 | ASDYKDFYI |
| 245 | CLYTFLIST |
| 246 | CSDDGIWSE |
| 247 | EASDYKDFY |
| 248 | ETWKTIITK |
| 249 | FLISTTFGC |
| 250 | FVTGLLLRK |
| 251 | GLDHALQCV |
| 252 | ILVIFVTGL |
| 253 | KVQDMCVYY |
| 254 | LDTNYNLFY |
| 255 | LLCSWKPGI |
| 256 | LQWQPPLSL |
| 257 | NIVKPLPPV |
| 258 | NLFYWYEGL |
| 259 | QSSWAETTY |
| 260 | RNIGSETWK |
| 261 | VCLAIGCLY |
| 262 | VENETYTLK |
| 263 | WLPFGFILI |
| 264 | WQYLLCSWK |
| 265 | WSDKQCWEG |
| 276 | WLPFGFILIL |
| 277 | VLLDTNYNL |
| 278 | GLDHALQCV |
| 331 | FLISTTFGCT |
| 332 | YLYLQWQPPL |
| 333 | GVLLDTNYNL |
| 334 | FQLQNIVKPL |

Of those exemplified IL13RA2 epitopes not encompassed by the wording of the claims, any one of amino acid sequences as set forth in SEQ ID NOs 245, 249, 251, 252, 255, 257, 263, 276 - 278 and 331- 334 is more preferred. The most preferred examples of epitopes of IL13RA2 have an amino acid sequence as set forth in SEQ ID NO: 263 or 276. Accordingly, an epitope of IL13RA2 having an amino acid sequence as set forth in SEQ ID NO: 263 is particularly preferred. Moreover, an epitope of IL13RA2 having an amino acid sequence as set forth in SEQ ID NO: 276 is particularly preferred.

In instance not encompassed by the wording of the claims the (poly)peptide for use as disclosed herein comprises or consists of an epitope of IL13RA2 having an amino acid sequence as set forth in any one of SEQ ID NOs 243 - 265, 276 - 278 and 331 - 334, preferably as set forth in SEQ ID NOs 245, 249, 251, 252, 255, 257, 263, 276 - 278 and 331 - 334, and more preferably as set forth in SEQ ID NO: 263 or 276.

In instance not encompassed by the wording of the claims the (poly)peptide for use as disclosed herein comprises or consists of a sequence variant of a (human) IL13RA2 epitope as described herein. For example the (poly)peptide for use as disclosed herein may comprise or consist of a sequence variant of an amino acid as set forth in any one of SEQ ID NOs 243 - 265, 276 - 278 and 331 - 334, preferably as set forth in SEQ ID NOs 245, 249, 251, 252, 255, 257, 263, 276 - 278 and 331 - 334, and most preferably as set forth in SEQ ID NO: 263 or 276.

As used herein, a "sequence variant" is similar, but contains at least one alteration, in comparison to the reference sequence, in particular a (human) reference epitope, such as a (human) IL13RA2 epitope. A "sequence variant" may be a recombinant sequence variant (which does not occur in nature), for example which is designed *in vitro,* e.g. by mutating the reference sequence, in particular a (human) reference epitope, such as a (human) IL13RA2 epitope. A "sequence variant" may also be a naturally occurring sequence variant, such as a naturally occurring peptide or a fragment of a naturally occurring protein (for example it may be found in a species other than human, such as a microbiota sequence variant as described below), which shares sequence identity with the reference sequence, such as a (human) IL13RA2 epitope. Such a naturally occurring protein or peptide (which comprises a sequence variant of an epitope of a tumor-associated antigen, such as IL13RA2 may be a homologue of the tumor-associated antigen or it may be unrelated to the tumor-associated antigen. For example, a (human) IL13RA2 epitope may be an IL13RA2 homologue or it may be unrelated to IL13RA2. Preferably, the sequence variant has a length of at least 5 amino acids, more preferably at least 6 amino acids, even more preferably at least 7 amino acids, and most preferably at least 8 amino acids. For example, the "sequence variant" may have a length of 9 or 10 amino acids. Preferably, the sequence variant has a length of 8 - 12 amino acids, more preferably the sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the sequence variant has a length of 13 - 24 amino acids. Peptides having such a length can bind to MHC (major histocompatibility complex) class II (MHC II), which is crucial for a CD4+ T-cell (T helper cell) response.

In general, the term "sequence variant", as used herein, i.e. throughout the present application, refers to a sequence which is similar (meaning in particular at least 50% sequence identity, see below), but not (100%) identical, to a reference sequence (such as (human) IL13RA2 or an epitope or fragment thereof). Accordingly, a sequence variant contains at least one alteration in comparison to a reference sequence. For example, in a sequence variant one or more of the amino acids or nucleotides of the reference sequence is deleted or substituted, or one or more amino acids or nucleotides are inserted into the sequence of the reference sequence. Therefore, the "sequence variant" is similar, but contains at least one alteration, in comparison to its reference sequence, such as an IL13RA2 epitope sequence. Preferably, a sequence variant shares, in particular over the whole length of the sequence, at least 60%, preferably at least 70%, more preferably at least 75%, more preferably at least 80%, even more preferably at least 85%, and most preferably at least 90% sequence identity with a reference sequence (such as the IL13RA2 epitope sequence).

A sequence variant may preserve the specific function of the reference sequence. In a particular instance not encompassed by the wording of the claims , this function may be the functionality as an "epitope", i.e. it can be recognized by the immune system, in particular by antibodies, T cell receptors, and/or B cell receptors and, preferably, it can elicit an immune response. Accordingly, in such instance not encompassed by the wording of the claims, it is preferred that the (poly)peptide for use as disclosed herein in particular the epitope of IL13RA2 or the sequence variant thereof, is immunogenic. In other words, the (poly)peptide, in particular the epitope of IL13RA2 or the sequence variant thereof, is preferably capable of eliciting an immune response.

The term "sequence variant" includes nucleotide sequence variants and amino acid sequence variants. For example, an amino acid sequence variant has an altered sequence in which one or more of the amino acids is deleted or substituted in comparison to the reference sequence, or one or more amino acids are inserted in comparison to the reference amino acid sequence. As a result of the alterations, the amino acid sequence variant has an amino acid sequence which is at least 50%, preferably at least 60%, more preferably at least 70%, more preferably at least 75%, even more preferably at least 80%, even more preferably at least 85%, and most preferably at least 90% identical to the reference sequence. For example, variant sequences which are at least 90% identical have no more than 10 alterations (i.e. any combination of deletions, insertions or substitutions) per 100 amino acids of the reference sequence.

In the context of the present invention, an amino acid sequence "sharing a sequence identity" of at least, for example, 70% to a query amino acid sequence of the present invention, is intended to mean that the sequence of the subject amino acid sequence is identical to the query sequence except that the subject amino acid sequence may include up to three amino acid alterations per each 10 amino acids of the query amino acid sequence. In other words, to obtain an amino acid sequence having a sequence of at least 70% identity to a query amino acid sequence, up to 30% (3 of 10) of the amino acid residues in the subject sequence may be inserted or substituted with another amino acid or deleted, preferably within the above definitions of variants or fragments. The same, of course, also applies similarly to nucleic acid sequences.

For (amino acid or nucleic acid) sequences without exact correspondence, a "% identity" of a first sequence (e.g., the sequence variant) may be determined with respect to a second sequence (e.g., the reference sequence). In general, the two sequences to be compared may be aligned to give a maximum correlation between the sequences. This may include inserting "gaps" in either one or both sequences, to enhance the degree of alignment. A % identity may then be determined over the whole length of each of the sequences being compared (so-called "global alignment"), that is particularly suitable for sequences of the same or similar length, or over shorter, defined lengths (so-called "local alignment"), that is more suitable for sequences of unequal length.

Methods for comparing the identity (sometimes also referred to as "similarity" or "homology") of two or more sequences are well known in the art. The percentage to which two (or more) sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

Preferably, the sequence variant not encompassed by the wording of the claims differs from the reference sequence, in particular a (human) reference epitope, such as the (human) IL13RA2 epitope, (only) in primary and/or secondary anchor residues for MHC molecules. More preferably, the sequence variant differs from the reference sequence, in particular a (human) reference epitope, such as the (human) IL13RA2 epitope, (only) in that it comprises amino acid substitutions (only) in primary and/or secondary anchor residues for MHC molecules. Anchor residues for the HLA subtypes are known in the art, and can be defined by large throughput analysis of structural data of existing p-HLA complexes in the Protein Data Bank. Moreover, anchor motifs for MHC subtypes can also be found in IEDB (URL: www.iedb.org; browse by allele) or in SYFPEITHI (URL: http://www.syfpeithi.de/). For example, for a 9 amino acid size HLA.A2.01 peptide, the peptide primary anchor residues, providing the main contact points, are located at residue positions P1, P2 and P9.

In a particular instance not encompassed by the wording of the claims, it is preferred that the core sequence of the sequence variant not encompassed by the wording of the claims is identical with the core sequence of the reference sequence, in particular a (human) reference epitope, such as the (human) IL13RA2 epitope, wherein the "core sequence" consists of all amino acids except the (at least) three most N-terminal and the (at least) three most C-terminal amino acids of the (human) reference epitope, such as the (human) IL13RA2 epitope. Accordingly, it is preferred that any alterations in the sequence variant in comparison to the (human) reference epitope, such as the (human) IL13RA2 epitope, are preferably located within the three N-terminal and/or within the three C-terminal amino acids of the (human) reference epitope, such as the (human) IL13RA2 epitope, but not in the "core sequence" of the (human) reference epitope, such as the (human) IL13RA2 epitope (amino acids in the middle of the of the (human) reference epitope sequence, e.g. in the middle of the (human) IL13RA2 epitope sequence). This does not mean that all three N-terminal and/or C-terminal amino acids of a (human) reference epitope, such as a (human) IL13RA2 epitope, must be altered, but only that those are the preferred amino acid positions, where an amino acid may optionally be altered. For example, in a (human) reference epitope, such as an IL13RA2 epitope, of nine amino acids, the three middle amino acids may represent the core sequence and alterations may preferably only occur at any of the three N-terminal and the three C-terminal amino acid positions.

In a particular instance not encompassed by the wording of the claims , the core sequence of the (human) reference epitope, such as the (human) IL13RA2 epitope, consists of all amino acids except the two most N-terminal and the two most C-terminal amino acids of the (human) reference epitope, such as the (human) IL13RA2 epitope. For example, in a (human) reference epitope, such as a (human) IL13RA2 epitope, of nine amino acids, the five middle amino acids may represent the core sequence and alterations may preferably only occur at any of the two N-terminal and the two C-terminal amino acid positions of the (human) reference epitope, such as the (human) IL13RA2 epitope.

In a particular instance not encompassed by the wording of the claims the core sequence of the (human) reference epitope, such as the (human) IL13RA2 epitope, consists of all amino acids except the most N-terminal and the most C-terminal amino acid of the (human) reference epitope, such as the (human) IL13RA2 epitope. For example, in a (human) reference epitope, such as a (human) IL13RA2 epitope, of nine amino acids, the seven middle amino acids may represent the core sequence and alterations may preferably only occur at the N-terminal position (P1) and the C-terminal amino acid position (P9) of the (human) reference epitope, such as the (human) IL13RA2 epitope.

In a particular instance not encompassed by the wording of the claims, the core sequence of the (human) reference epitope, such as the (human) IL13RA2 epitope, consists of all amino acids except the two most N-terminal amino acids and the most C-terminal amino acid of the (human) reference epitope, such as the (human) IL13RA2 epitope. For example, in a (human) reference epitope, such as a (human) IL13RA2 epitope, of nine amino acids, the six middle amino acids may represent the core sequence and alterations may preferably only occur at any of the two N-terminal positions (P1 and P2) and the C-terminal amino acid position (P9) of the (human) reference epitope, such as the (human) IL13RA2 epitope.

In a particular instance not encompassed by the wording of the claims the sequence variant, e.g. having a length of nine amino acids, comprises at position 1 (P1; the most N-terminal amino acid position) a phenylalanine (F) or a lysine (K). In a particular instance not encompassed by the wording of the claims the sequence variant, e.g. having a length of nine amino acids, comprises at position 2 (P2) a leucine (L) or a methionine (M). In a particular instance not encompassed by the wording of the claims the sequence variant, e.g. having a length of nine amino acids, comprises at position 9 (P9) a valine (V) or a leucine (L). In a particular instance not encompassed by the wording of the claims the sequence variant, e.g. having a length of nine amino acids, comprises at position 1 (P1; the most N-terminal amino acid position) a phenylalanine (F) or a lysine (K), at position 2 (P2) a leucine (L) or a methionine (M) and/or at position 9 (P9) a valine (V) or a leucine (L).

Moreover, amino acid substitutions, in particular at positions other than the anchor position(s) for MHC molecules (e.g., P1, P2 and P9 for MHC-I subtype HLA.A2.01), are preferably conservative amino acid substitutions. Examples of conservative substitutions include substitution of one aliphatic residue for another, such as Ile, Val, Leu, or Ala for one another; or substitutions of one polar residue for another, such as between Lys and Arg; Glu and Asp; or Gln and Asn. Other such conservative substitutions, for example, substitutions of entire regions having similar hydrophobicity properties, are well known (Kyte and Doolittle, 1982, J. Mol. Biol. 157(1):105- 132). Examples of conservative amino acid substitutions are presented in Table 2 below:

**(Table 2)**

| **Original residues** | **Examples of substitutions** |
|---|---|
| Ala (A) | Val, Leu, Ile, Gly |
| Arg (R) | His, Lys |
| Asn (N) | Gln |
| Asp (D) | Glu |
| Cys (C) | Ser |
| Gln (Q) | Asn |
| Glu (E) | Asp |
| Gly (G) | Pro, Ala |
| His (H) | Lys, Arg |
| Ile (I) | Leu, Val, Met, Ala, Phe |
| Leu (L) | Ile, Val, Met, Ala, Phe |
| Lys (K) | Arg, His |
| Met (M) | Leu, Ile, Phe |
| Phe (F) | Leu, Val, Ile, Tyr, Trp, Met |
| Pro (P) | Ala, Gly |
| Ser (S) | Thr |
| Thr (T) | Ser |
| Trp (W) | Tyr, Phe |
| Tyr (Y) | Trp, Phe |
| Original residues | Examples of substitutions |
| Val (V) | Ile, Met, Leu, Phe, Ala |

Preferred examples of sequence variants not encompassed by the wording of the claims are sequence variants of a (human) IL13RA2 epitope having an amino acid sequences as set forth in any one of SEQ ID NOs 243 - 265, 276 - 278 and 331 - 334.

In a particular instance not encompassed by the wording of the claims as such the (poly)peptide for use as herein disclosed comprises an amino acid sequence as set forth in any one of SEQ ID NOs 1- 242, 267 - 274, 279 and 335 - 344. The amino acid sequences as set forth in any one of SEQ ID NOs 1- 242, 267 - 274, 279 and 335 - 344 represent preferred examples of sequence variants of human IL13RA2 epitopes. As shown in the examples herein, the exemplified sequence variants allow the raise of a strong immune response against themselves, and most importantly, allow the raise of a strong immune response against peptides having amino acid similarity therewith which are comprised in the IL13RA2 antigen.

Preferred examples of sequence variants not encompassed by the wording of the claims as such are listed in Table 3 below, which also provides information regarding the corresponding reference sequence in human IL13RA2, and HLA class. The sequence IDs SEQ ID NO: 1 to 242, 267 - 274, 279 and 335 - 344 refer to the sequence variants.

**Table 3: Examples of preferred sequence variants of human IL13RA2 epitopes not encompassed by the wording of the claims as such.**

| **SEQ ID NO. sequence variant** | **Sequence variant** | **Reference sequence in human IL13RA2** | **HLA** |
|---|---|---|---|
| **1** | ALGCLYTFV | AIGCLYTFL | HLA-A*02:01 |
| **2** | AMGCLYTFI | AIGCLYTFL | HLA-A*02:01 |
| **3** | AMGCLYTFV | AIGCLYTFL | HLA-A*02:01 |
| **9** | FIGCLYTFI | AIGCLYTFL | HLA-A*02:01 |
| **10** | FIGCLYTFL | AIGCLYTFL | HLA-A*02:01 |
| **11** | FIGCLYTFV | AIGCLYTFL | HLA-A*02:01 |
| **24** | FLGCLYTFI | AIGCLYTFL | HLA-A*02:01 |
| **25** | FLGCLYTFL | AIGCLYTFL | HLA-A*02:01 |
| **26** | FLGCLYTFV | AIGCLYTFL | HLA-A*02:01 |
| **57** | FMGCLYTFI | AIGCLYTFL | HLA-A*02:01 |
| **58** | FMGCLYTFL | AIGCLYTFL | HLA-A*02:01 |
| **59** | FMGCLYTFV | AIGCLYTFL | HLA-A*02:01 |
| **111** | WLGCLYTFI | AIGCLYTFL | HLA-A*02:01 |
| **112** | WLGCLYTFL | AIGCLYTFL | HLA-A*02:01 |
| **113** | WLGCLYTFV | AIGCLYTFL | HLA-A*02:01 |
| **130** | WMGCLYTFI | AIGCLYTFL | HLA-A*02:01 |
| **131** | WMGCLYTFL | AIGCLYTFL | HLA-A*02:01 |
| **132** | WMGCLYTFV | AIGCLYTFL | HLA-A*02:01 |
| **158** | YIGCLYTFI | AIGCLYTFL | HLA-A*02:01 |
| **159** | YIGCLYTFL | AIGCLYTFL | HLA-A*02:01 |
| **160** | YIGCLYTFV | AIGCLYTFL | HLA-A*02:01 |
| **171** | YLGCLYTFI | AIGCLYTFL | HLA-A*02:01 |
| **172** | YLGCLYTFL | AIGCLYTFL | HLA-A*02:01 |
| **173** | YLGCLYTFV | AIGCLYTFL | HLA-A*02:01 |
| **204** | YMGCLYTFI | AIGCLYTFL | HLA-A*02:01 |
| **205** | YMGCLYTFL | AIGCLYTFL | HLA-A*02:01 |
| **206** | YMGCLYTFV | AIGCLYTFL | HLA-A*02:01 |
| **89** | FSDYKDFYI | ASDYKDFYI | HLA-A*01:01 |
| **90** | FSDYKDFYL | ASDYKDFYI | HLA-A*01:01 |
| **91** | FSDYKDFYV | ASDYKDFYI | HLA-A*01:01 |
| **153** | WSDYKDFYI | ASDYKDFYI | HLA-A*01:01 |
| **154** | WSDYKDFYL | ASDYKDFYI | HLA-A*01:01 |
| **155** | WSDYKDFYV | ASDYKDFYI | HLA-A*01:01 |
| **237** | YSDYKDFYI | ASDYKDFYI | HLA-A*01:01 |
| **238** | YSDYKDFYL | ASDYKDFYI | HLA-A*01:01 |
| **239** | YSDYKDFYV | ASDYKDFYI | HLA-A*01:01 |
| **4** | CMYTFLISV | CLYTFLIST | HLA-A*02:01 |
| **42** | FLYTFLISI | CLYTFLIST | HLA-A*02:01 |
| **43** | FLYTFLISL | CLYTFLIST | HLA-A*02:01 |
| **44** | FLYTFLIST | CLYTFLIST | HLA-A*02:01 |
| **45** | FLYTFLISV | CLYTFLIST | HLA-A*02:01 |
| **78** | FMYTFLISI | CLYTFLIST | HLA-A*02:01 |
| **79** | FMYTFLISL | CLYTFLIST | HLA-A*02:01 |
| **80** | FMYTFLIST | CLYTFLIST | HLA-A*02:01 |
| **81** | FMYTFLISV | CLYTFLIST | HLA-A*02:01 |
| **123** | WLYTFLISV | CLYTFLIST | HLA-A*02:01 |
| **144** | WMYTFLISI | CLYTFLIST | HLA-A*02:01 |
| **145** | WMYTFLISL | CLYTFLIST | HLA-A*02:01 |
| **146** | WMYTFLISV | CLYTFLIST | HLA-A*02:01 |
| **190** | YLYTFLISI | CLYTFLIST | HLA-A*02:01 |
| **191** | YLYTFLISL | CLYTFLIST | HLA-A*02:01 |
| **192** | YLYTFLIST | CLYTFLIST | HLA-A*02:01 |
| **193** | YLYTFLISV | CLYTFLIST | HLA-A*02:01 |
| **226** | YMYTFLISI | CLYTFLIST | HLA-A*02:01 |
| **227** | YMYTFLISL | CLYTFLIST | HLA-A*02:01 |
| **228** | YMYTFLIST | CLYTFLIST | HLA-A*02:01 |
| **229** | YMYTFLISV | CLYTFLIST | HLA-A*02:01 |
| **5** | CSDDGIWSI | CSDDGIWSE | HLA-A*01:01 |
| **6** | CSDDGIWSL | CSDDGIWSE | HLA-A*01:01 |
| **7** | CSDDGIWSV | CSDDGIWSE | HLA-A*01:01 |
| **16** | FLDDGIWSI | CSDDGIWSE | HLA-A*01:01 |
| **17** | FLDDGIWSL | CSDDGIWSE | HLA-A*01:01 |
| **18** | FLDDGIWSV | CSDDGIWSE | HLA-A*01:01 |
| **49** | FMDDGIWSI | CSDDGIWSE | HLA-A*01:01 |
| **50** | FMDDGIWSL | CSDDGIWSE | HLA-A*01:01 |
| **51** | FMDDGIWSV | CSDDGIWSE | HLA-A*01:01 |
| **83** | FSDDGIWSI | CSDDGIWSE | HLA-A*01:01 |
| **84** | FSDDGIWSL | CSDDGIWSE | HLA-A*01:01 |
| **85** | FSDDGIWSV | CSDDGIWSE | HLA-A*01:01 |
| **107** | WLDDGIWSI | CSDDGIWSE | HLA-A*01:01 |
| **108** | WLDDGIWSL | CSDDGIWSE | HLA-A*01:01 |
| **109** | WLDDGIWSV | CSDDGIWSE | HLA-A*01:01 |
| **125** | WMDDGIWSI | CSDDGIWSE | HLA-A*01:01 |
| **126** | WMDDGIWSL | CSDDGIWSE | HLA-A*01:01 |
| **127** | WMDDGIWSV | CSDDGIWSE | HLA-A*01:01 |
| **147** | WSDDGIWSI | CSDDGIWSE | HLA-A*01:01 |
| **148** | WSDDGIWSL | CSDDGIWSE | HLA-A*01:01 |
| **149** | WSDDGIWSV | CSDDGIWSE | HLA-A*01:01 |
| **163** | YLDDGIWSI | CSDDGIWSE | HLA-A*01:01 |
| **164** | YLDDGIWSL | CSDDGIWSE | HLA-A*01:01 |
| **165** | YLDDGIWSV | CSDDGIWSE | HLA-A*01:01 |
| **196** | YMDDGIWSI | CSDDGIWSE | HLA-A*01:01 |
| **197** | YMDDGIWSL | CSDDGIWSE | HLA-A*01:01 |
| **198** | YMDDGIWSV | CSDDGIWSE | HLA-A*01:01 |
| **231** | YSDDGIWSI | CSDDGIWSE | HLA-A*01:01 |
| **232** | YSDDGIWSL | CSDDGIWSE | HLA-A*01:01 |
| **233** | YSDDGIWSV | CSDDGIWSE | HLA-A*01:01 |
| **8** | FASDYKDFY | EASDYKDFY | HLA-A*01:01 |
| **32** | FLSDYKDFY | EASDYKDFY | HLA-A*01:01 |
| **66** | FMSDYKDFY | EASDYKDFY | HLA-A*01:01 |
| **105** | WASDYKDFY | EASDYKDFY | HLA-A*01:01 |
| **117** | WLSDYKDFY | EASDYKDFY | HLA-A*01:01 |
| **137** | WMSDYKDFY | EASDYKDFY | HLA-A*01:01 |
| **157** | YASDYKDFY | EASDYKDFY | HLA-A*01:01 |
| **180** | YLSDYKDFY | EASDYKDFY | HLA-A*01:01 |
| **214** | YMSDYKDFY | EASDYKDFY | HLA-A*01:01 |
| **37** | FLWKTIITK | ETWKTIITK | HLA-A*03:01 |
| **73** | FMWKTIITK | ETWKTIITK | HLA-A*03:01 |
| **93** | FTWKTIITK | ETWKTIITK | HLA-A*03:01 |
| **140** | WMWKTIITK | ETWKTIITK | HLA-A*03:01 |
| **185** | YLWKTIITK | ETWKTIITK | HLA-A*03:01 |
| **221** | YMWKTIITK | ETWKTIITK | HLA-A*03:01 |
| **241** | YTWKTIITK | ETWKTIITK | HLA-A*03:01 |
| **27** | FLISTTFGI | FLISTTFGC | HLA-A*02:01 |
| **28** | FLISTTFGL | FLISTTFGC | HLA-A*02:01 |
| **29** | FLISTTFGV | FLISTTFGC | HLA-A*02:01 |
| **60** | FMISTTFGI | FLISTTFGC | HLA-A*02:01 |
| **61** | FMISTTFGL | FLISTTFGC | HLA-A*02:01 |
| **62** | FMISTTFGV | FLISTTFGC | HLA-A*02:01 |
| **114** | WLISTTFGL | FLISTTFGC | HLA-A*02:01 |
| **115** | WLISTTFGV | FLISTTFGC | HLA-A*02:01 |
| **133** | WMISTTFGI | FLISTTFGC | HLA-A*02:01 |
| **134** | WMISTTFGL | FLISTTFGC | HLA-A*02:01 |
| **135** | WMISTTFGV | FLISTTFGC | HLA-A*02:01 |
| **174** | YLISTTFGI | FLISTTFGC | HLA-A*02:01 |
| **175** | YLISTTFGL | FLISTTFGC | HLA-A*02:01 |
| **176** | YLISTTFGV | FLISTTFGC | HLA-A*02:01 |
| **207** | YMISTTFGI | FLISTTFGC | HLA-A*02:01 |
| **208** | YMISTTFGL | FLISTTFGC | HLA-A*02:01 |
| **209** | YMISTTFGV | FLISTTFGC | HLA-A*02:01 |
| **67** | FMTGLLLRK | FVTGLLLRK | HLA-A*03:01 |
| **215** | YMTGLLLRK | FVTGLLLRK | HLA-A*03:01 |
| **19** | FLDHALQCV | GLDHALQCV | HLA-A*02:01 |
| **52** | FMDHALQCV | GLDHALQCV | HLA-A*02:01 |
| **166** | YLDHALQCV | GLDHALQCV | HLA-A*02:01 |
| **199** | YMDHALQCV | GLDHALQCV | HLA-A*02:01 |
| **69** | FMVIFVTGV | ILVIFVTGL | HLA-A*02:01 |
| **217** | YMVIFVTGV | ILVIFVTGL | HLA-A*02:01 |
| **65** | FMQDMCVYY | KVQDMCVYY | HLA-A*01:01 |
| **94** | FVQDMCVYY | KVQDMCVYY | HLA-A*01:01 |
| **156** | WVQDMCVYY | KVQDMCVYY | HLA-A*01:01 |
| **179** | YLQDMCVYY | KVQDMCVYY | HLA-A*01:01 |
| **213** | YMQDMCVYY | KVQDMCVYY | HLA-A*01:01 |
| **242** | YVQDMCVYY | KVQDMCVYY | HLA-A*01:01 |
| **33** | FLTNYNLFY | LDTNYNLFY | HLA-A*01:01 |
| **68** | FMTNYNLFY | LDTNYNLFY | HLA-A*01:01 |
| **95** | LLTNYNLFY | LDTNYNLFY | HLA-A*01:01 |
| **97** | LMTNYNLFY | LDTNYNLFY | HLA-A*01:01 |
| **118** | WLTNYNLFY | LDTNYNLFY | HLA-A*01:01 |
| **138** | WMTNYNLFY | LDTNYNLFY | HLA-A*01:01 |
| **181** | YLTNYNLFY | LDTNYNLFY | HLA-A*01:01 |
| **216** | YMTNYNLFY | LDTNYNLFY | HLA-A*01:01 |
| **13** | FLCSWKPGI | LLCSWKPGI | HLA-A*02:01 |
| **14** | FLCSWKPGL | LLCSWKPGI | HLA-A*02:01 |
| **15** | FLCSWKPGV | LLCSWKPGI | HLA-A*02:01 |
| **46** | FMCSWKPGI | LLCSWKPGI | HLA-A*02:01 |
| **47** | FMCSWKPGL | LLCSWKPGI | HLA-A*02:01 |
| **48** | FMCSWKPGV | LLCSWKPGI | HLA-A*02:01 |
| **106** | WLCSWKPGV | LLCSWKPGI | HLA-A*02:01 |
| **124** | WMCSWKPGV | LLCSWKPGI | HLA-A*02:01 |
| **162** | YLCSWKPGV | LLCSWKPGI | HLA-A*02:01 |
| **194** | YMCSWKPGI | LLCSWKPGI | HLA-A*02:01 |
| **195** | YMCSWKPGV | LLCSWKPGI | HLA-A*02:01 |
| **38** | FLWQPPLSI | LQWQPPLSL | HLA-A*02:01 |
| **39** | FLWQPPLSL | LQWQPPLSL | HLA-A*02:01 |
| **40** | FLWQPPLSV | LQWQPPLSL | HLA-A*02:01 |
| **74** | FMWQPPLSI | LQWQPPLSL | HLA-A*02:01 |
| **75** | FMWQPPLSL | LQWQPPLSL | HLA-A*02:01 |
| **76** | FMWQPPLSV | LQWQPPLSL | HLA-A*02:01 |
| **82** | FQWQPPLSV | LQWQPPLSL | HLA-A*02:01 |
| **96** | LLWQPPLSV | LQWQPPLSL | HLA-A*02:01 |
| **98** | LMWQPPLSI | LQWQPPLSL | HLA-A*02:01 |
| **99** | LMWQPPLSL | LQWQPPLSL | HLA-A*02:01 |
| **100** | LMWQPPLSV | LQWQPPLSL | HLA-A*02:01 |
| **120** | WLWQPPLSI | LQWQPPLSL | HLA-A*02:01 |
| **121** | WLWQPPLSL | LQWQPPLSL | HLA-A*02:01 |
| **122** | WLWQPPLSV | LQWQPPLSL | HLA-A*02:01 |
| **141** | WMWQPPLSI | LQWQPPLSL | HLA-A*02:01 |
| **142** | WMWQPPLSL | LQWQPPLSL | HLA-A*02:01 |
| **143** | WMWQPPLSV | LQWQPPLSL | HLA-A*02:01 |
| **186** | YLWQPPLSI | LQWQPPLSL | HLA-A*02:01 |
| **187** | YLWQPPLSL | LQWQPPLSL | HLA-A*02:01 |
| **188** | YLWQPPLSV | LQWQPPLSL | HLA-A*02:01 |
| **222** | YMWQPPLSI | LQWQPPLSL | HLA-A*02:01 |
| **223** | YMWQPPLSL | LQWQPPLSL | HLA-A*02:01 |
| **224** | YMWQPPLSV | LQWQPPLSL | HLA-A*02:01 |
| **230** | YQWQPPLSV | LQWQPPLSL | HLA-A*02:01 |
| **12** | FIVKPLPPV | NIVKPLPPV | HLA-A*02:01 |
| **34** | FLVKPLPPI | NIVKPLPPV | HLA-A*02:01 |
| **35** | FLVKPLPPL | NIVKPLPPV | HLA-A*02:01 |
| **36** | FLVKPLPPV | NIVKPLPPV | HLA-A*02:01 |
| **70** | FMVKPLPPI | NIVKPLPPV | HLA-A*02:01 |
| **71** | FMVKPLPPL | NIVKPLPPV | HLA-A*02:01 |
| **72** | FMVKPLPPV | NIVKPLPPV | HLA-A*02:01 |
| **119** | WLVKPLPPV | NIVKPLPPV | HLA-A*02:01 |
| **139** | WMVKPLPPV | NIVKPLPPV | HLA-A*02:01 |
| **161** | YIVKPLPPV | NIVKPLPPV | HLA-A*02:01 |
| **182** | YLVKPLPPI | NIVKPLPPV | HLA-A*02:01 |
| **183** | YLVKPLPPL | NIVKPLPPV | HLA-A*02:01 |
| **184** | YLVKPLPPV | NIVKPLPPV | HLA-A*02:01 |
| **218** | YMVKPLPPI | NIVKPLPPV | HLA-A*02:01 |
| **219** | YMVKPLPPL | NIVKPLPPV | HLA-A*02:01 |
| **220** | YMVKPLPPV | NIVKPLPPV | HLA-A*02:01 |
| **21** | FLFYWYEGI | NLFYWYEGL | HLA-A*02:01 |
| **22** | FLFYWYEGL | NLFYWYEGL | HLA-A*02:01 |
| **23** | FLFYWYEGV | NLFYWYEGL | HLA-A*02:01 |
| **54** | FMFYWYEGI | NLFYWYEGL | HLA-A*02:01 |
| **55** | FMFYWYEGL | NLFYWYEGL | HLA-A*02:01 |
| **56** | FMFYWYEGV | NLFYWYEGL | HLA-A*02:01 |
| **110** | WLFYWYEGV | NLFYWYEGL | HLA-A*02:01 |
| **129** | WMFYWYEGV | NLFYWYEGL | HLA-A*02:01 |
| **168** | YLFYWYEGI | NLFYWYEGL | HLA-A*02:01 |
| **169** | YLFYWYEGL | NLFYWYEGL | HLA-A*02:01 |
| **170** | YLFYWYEGV | NLFYWYEGL | HLA-A*02:01 |
| **201** | YMFYWYEGI | NLFYWYEGL | HLA-A*02:01 |
| **202** | YMFYWYEGL | NLFYWYEGL | HLA-A*02:01 |
| **203** | YMFYWYEGV | NLFYWYEGL | HLA-A*02:01 |
| **92** | FSSWAETTY | QSSWAETTY | HLA-A*01:01 |
| **240** | YSSWAETTY | QSSWAETTY | HLA-A*01:01 |
| **101** | RLIGSETWK | RNIGSETWK | HLA-A*03:01 |
| **102** | RMIGSETWK | RNIGSETWK | HLA-A*03:01 |
| **210** | YMLAIGCLY | VCLAIGCLY | HLA-A*01:01 |
| **30** | FLNETYTLK | VENETYTLK | HLA-A*03:01 |
| **63** | FMNETYTLK | VENETYTLK | HLA-A*03:01 |
| **103** | VLNETYTLK | VENETYTLK | HLA-A*03:01 |
| **104** | VMNETYTLK | VENETYTLK | HLA-A*03:01 |
| **116** | WLNETYTLK | VENETYTLK | HLA-A*03:01 |
| **136** | WMNETYTLK | VENETYTLK | HLA-A*03:01 |
| **177** | YLNETYTLK | VENETYTLK | HLA-A*03:01 |
| **211** | YM N ETYTLK | VENETYTLK | HLA-A*03:01 |
| **31** | FLPFGFILV | WLPFGFILI | HLA-A*02:01 |
| **64** | FMPFGFILV | WLPFGFILI | HLA-A*02:01 |
| **178** | YLPFGFILV | WLPFGFILI | HLA-A*02:01 |
| **212** | YMPFGFILV | WLPFGFILI | HLA-A*02:01 |
| **267** | YLPFGFILV | WLPFGFILI | HLA-A*02:01 |
| **268** | VLPFGFILV | WLPFGFILI | HLA-A*02:01 |
| **269** | NLPFGFILV | WLPFGFILI | HLA-A*02:01 |
| **270** | RLPFGFILV | WLPFGFILI | HLA-A*02:01 |
| **271** | SLPFGFILV | WLPFGFILI | HLA-A*02:01 |
| **272** | TLPFGFILV | WLPFGFILI | HLA-A*02:01 |
| **273** | CLPFGFILV | WLPFGFILI | HLA-A*02:01 |
| **274** | LLPFGFILV | WLPFGFILI | HLA-A*02:01 |
| **41** | FLYLLCSWK | WQYLLCSWK | HLA-A*03:01 |
| **77** | FMYLLCSWK | WQYLLCSWK | HLA-A*03:01 |
| **189** | YLYLLCSWK | WQYLLCSWK | HLA-A*03:01 |
| **225** | YMYLLCSWK | WQYLLCSWK | HLA-A*03:01 |
| **20** | FLDKQCWEV | WSDKQCWEG | HLA-A*01:01 |
| **53** | FMDKQCWEV | WSDKQCWEG | HLA-A*01:01 |
| **86** | FSDKQCWEI | WSDKQCWEG | HLA-A*01:01 |
| **87** | FSDKQCWEL | WSDKQCWEG | HLA-A*01:01 |
| **88** | FSDKQCWEV | WSDKQCWEG | HLA-A*01:01 |
| **128** | WMDKQCWEV | WSDKQCWEG | HLA-A*01:01 |
| **150** | WSDKQCWEI | WSDKQCWEG | HLA-A*01:01 |
| **151** | WSDKQCWEL | WSDKQCWEG | HLA-A*01:01 |
| **152** | WSDKQCWEV | WSDKQCWEG | HLA-A*01:01 |
| **167** | YLDKQCWEV | WSDKQCWEG | HLA-A*01:01 |
| **200** | YMDKQCWEV | WSDKQCWEG | HLA-A*01:01 |
| **234** | YSDKQCWEI | WSDKQCWEG | HLA-A*01:01 |
| **235** | YSDKQCWEL | WSDKQCWEG | HLA-A*01:01 |
| **236** | YSDKQCWEV | WSDKQCWEG | HLA-A*01:01 |
| **279** | FLPFGFILPV | WLPFGFILI(L) | HLA-A*02:01 |
| **335** | FMPFGFILPI | WLPFGFILI(L) | HLA-A*02:01 |
| **336** | FMPFGFILGV | WLPFGFILI(L) | HLA-A*02:01 |
| **337** | FLISTTFTIN | FLISTTFGCT | |
| **338** | FMISTTFMRL | FLISTTFGCT | |
| **339** | QMISTTFGNV | FLISTTFGCT | |
| **340** | WLYLQWQPSV | YLYLQWQPPL | |
| **341** | FVLLDTNYEI | GVLLDTNYNL | |
| **342** | FILLDTNYEI | GVLLDTNYNL | |
| **343** | YELQNIVLPI | FQLQNIVKPL | |
| **344** | FMLQNIVKNL | FQLQNIVKPL | |

Those 265 sequence variants can be further defined based on the sequence of reference epitope in human IL13RA2. Accordingly, those 265 sequence variants may be categorized in a plurality of distinct families according to their reference epitope in IL13RA2:
- Family « AIGCLYTFL » (SEQ ID NO: 243), which family includes the amino acid sequences of SEQ ID NO: 1-3, 9-11, 24-26, 57-59, 111-113, 130-132, 158-160, 171-173, 204-206;
- Family « ASDYKDFYI » (SEQ ID NO: 244) which family includes the amino acid sequences of SEQ ID NO: 89-91, 153-155, 237-239 ;
- Family « CLYTFLIST » (SEQ ID NO: 245) which family includes the amino acid sequences of SEQ ID NO: 4, 42-45, 78-81, 123, 144-146, 190-193, 226-229 ;
- Family « CSDDGIWSE » (SEQ ID NO: 246) which family includes the amino acid sequences of SEQ ID NO: 5-7, 16-18, 49-51, 83-85, 107-109, 125-127, 147-149, 163-165, 197-198, 231-233 ;
- Family « EASDYKDFY » (SEQ ID NO: 247) which family includes the amino acid sequences of SEQ ID NO: 8, 32, 66, 105, 117, 137, 157, 180, 214 ;
- Family « ETWKTIITK» (SEQ ID NO: 248) which family includes the amino acid sequences of SEQ ID NO: 37, 73, 93, 140, 185, 221, 241 ;
- Family « FLISTTFGC » (SEQ ID NO: 249) which family includes the amino acid sequences of SEQ ID NO: 27-29, 60-62, 114-115, 133-135, 174-176, 207-209 ;
- Family « FVTGLLLRK » (SEQ ID NO: 250) which family includes the amino acid sequences of SEQ ID NO: 67, 215 ;
- Family « GLDHALQCV » (SEQ ID NO: 251) which family includes the amino acid sequences of SEQ ID NO: 19, 52, 166, 199 ;
- Family « ILVIFVTGL » (SEQ ID NO: 252) which family includes the amino acid sequences of SEQ ID NO: 69, 217 ;
- Family « KVQDMCVYY » (SEQ ID NO: 253) which family includes the amino acid sequences of SEQ ID NO: 65, 94, 156, 179, 213, 242 ;
- Family « LDTNYNLFY » (SEQ ID NO: 254) which family includes the amino acid sequences of SEQ ID NO: 33, 68, 95, 97, 118, 138, 181, 216 ;
- Family « LLCSWKPGI » (SEQ ID NO: 255) which family includes the amino acid sequences of SEQ ID NO: 13-15, 46-48, 106, 124, 162, 194-195;
- Family « LQWQPPLSL » (SEQ ID NO: 256) which family includes the amino acid sequences of SEQ ID NO: 38-40, 74-76, 82, 96-100, 120-122, 141-143, 186-188, 222-224, 230 ;
- Family « NIVKPLPPV » (SEQ ID NO: 257) which family includes the amino acid sequences of SEQ ID NO: 12, 34-36, 70-72, 119, 139, 161, 182-184, 218-220 ;
- Family « NLFYWYEGL » (SEQ ID NO: 258) which family includes the amino acid sequences of SEQ ID NO: 21-23, 54-56, 110, 129, 168-170, 201-203 ;
- Family « QSSWAETTY » (SEQ ID NO: 259) which family includes the amino acid sequences of SEQ ID NO: 92, 240 ;
- Family « RNIGSETWK » (SEQ ID NO: 260) which family includes the amino acid sequences of SEQ ID NO: 101, 102 ;
- Family « VCLAIGCLY » (SEQ ID NO: 261) including sequence SEQ ID NO: 210 ;
- Family « VENETYTLK » (SEQ ID NO: 262) which family includes the amino acid sequences of SEQ ID NO: 30, 63, 103-104, 116, 136, 177, 211 ;
- Family « WLPFGFILI » (SEQ ID NO: 263) which family includes the amino acid sequences of SEQ ID NO: 31, 64, 178, 212, 267 - 274, 279, 335 and 336;
- Family « WQYLLCSWK» (SEQ ID NO: 264) which family includes the amino acid sequences of SEQ ID NO: 41, 77, 189, 225 ;
- Family « WSDKQCWEG » (SEQ ID NO: 265) which family includes the amino acid sequences of SEQ ID NO: 20, 53, 86-88, 128, 150-152, 167, 200, 234-236;
- Family « WLPFGFILIL » (SEQ ID NO: 276) which family includes the amino acid sequences of SEQ ID NO: 279, 335 and 336;
- Family « FLISTTFGCT » (SEQ ID NO: 331) which family includes the amino acid sequences of SEQ ID NO: 337 - 339;
- Family « YLYLQWQPPL » (SEQ ID NO: 332) which family includes the amino acid sequence of SEQ ID NO: 340;
- Family « GVLLDTNYNL » (SEQ ID NO: 333) which family includes the amino acid sequences of SEQ ID NO: 341 and 342; or
- Family « FQLQNIVKPL » (SEQ ID NO: 334) which family includes the amino acid sequences of SEQ ID NO: 343 and 344.

In an instance not encompassed by the wording of the claims, a sequence variant may belong to the Family « AIGCLYTFL » (SEQ ID NO: 243). In an instance not encompassed by the wording of the claims, a preferred sequence variant comprises or consists of an amino acid sequence as set forth in any one of SEQ ID NO: 1-3, 9-11, 24-26, 57-59, 111-113, 130-132, 158-160, 171-173, 204-206. In an instance not encompassed by the wording of the claims, a preferred sequence variant belongs to the Family « ASDYKDFYI » (SEQ ID NO: 244). In an instance not encompassed by the wording of the claims, a preferred sequence variant comprises or consists of an amino acid sequence as set forth in any one of SEQ ID NO: 89-91, 153-155, 237-239. In an instance not encompassed by the wording of the claims, a preferred sequence variant belongs to the Family « CLYTFLIST » (SEQ ID NO: 245). In an instance not encompassed by the wording of the claims, a preferred sequence variant comprises or consists of an amino acid sequence as set forth in any one of SEQ ID NO: 4, 42-45, 78-81, 123, 144-146, 190-193, 226-229. In an instance not encompassed by the wording of the claims a preferred sequence variant belongs to the Family « CSDDGIWSE » (SEQ ID NO: 246). In an instance not encompassed by the wording of the claims, a preferred sequence variant comprises or consists of an amino acid sequence as set forth in any one of SEQ ID NO: 5-7, 16-18, 49-51, 83-85, 107-109, 125-127, 147-149, 163-165, 197-198, 231-233. In an instance not encompassed by the wording of the claims, a preferred sequence variant belongs to the Family « EASDYKDFY » (SEQ ID NO: 247). In an instance not encompassed by the wording of the claims, a preferred sequence variant comprises or consists of an amino acid sequence as set forth in any one of SEQ ID NO: 8, 32, 66, 105, 117, 137, 157, 180, 214. In an instance not encompassed by the wording of the claims, a preferred sequence variant belongs to the Family « ETWKTIITK» (SEQ ID NO: 248). In an instance not encompassed by the wording of the claims, a preferred sequence variant comprises or consists of an amino acid sequence as set forth in any one of SEQ ID NO: 37, 73, 93, 140, 185, 221, 241. In an instance not encompassed by the wording of the claims, a preferred sequence variant belongs to the Family « FLISTTFGC » (SEQ ID NO: 249). In an instance not encompassed by the wording of the claims, a preferred sequence variant comprises or consists of an amino acid sequence as set forth in any one of SEQ ID NO: 27-29, 60-62, 114-115, 133-135, 174-176, 207-209. In an instance not encompassed by the wording of the claims, a preferred sequence variant belongs to the Family « FVTGLLLRK » (SEQ ID NO: 250). In an instance not encompassed by the wording of the claims, a preferred sequence variant comprises or consists of an amino acid sequence as set forth in any one of SEQ ID NO: 67, 215. In an instance not encompassed by the wording of the claims, a preferred sequence variant belongs to the Family « GLDHALQCV » (SEQ ID NO: 251). In an instance not encompassed by the wording of the claims, a preferred sequence variant comprises or consists of an amino acid sequence as set forth in any one of SEQ ID NO: 19, 52, 166, 199. In an instance not encompassed by the wording of the claims, a preferred sequence variant belongs to the Family « ILVIFVTGL » (SEQ ID NO: 252). In an instance not encompassed by the wording of the claims, a preferred sequence variant comprises or consists of an amino acid sequence as set forth in any one of SEQ ID NO: 69, 217. In an instance not encompassed by the wording of the claims, a preferred sequence variant belongs to the Family « KVQDMCVYY » (SEQ ID NO: 253). In an instance not encompassed by the wording of the claims, a preferred sequence variant comprises or consists of an amino acid sequence as set forth in any one of SEQ ID NO: 65, 94, 156, 179, 213, 242. In an instance not encompassed by the wording of the claims, a preferred sequence variant belongs to the Family « LDTNYNLFY » (SEQ ID NO: 254). In an instance not encompassed by the wording of the claims, a preferred sequence variant comprises or consists of an amino acid sequence as set forth in any one of SEQ ID NO: 33, 68, 95, 97, 118, 138, 181, 216. In an instance not encompassed by the wording of the claims, a preferred sequence variant belongs to the Family « LLCSWKPGI » (SEQ ID NO: 255). In an instance not encompassed by the wording of the claims, a preferred sequence variant comprises or consists of an amino acid sequence as set forth in any one of SEQ ID NO: 13-15, 46-48, 106, 124, 162, 194-195. In an instance not encompassed by the wording of the claims, a preferred sequence variant belongs to the Family « LQWQPPLSL » (SEQ ID NO: 256). In an instance not encompassed by the wording of the claims, a preferred sequence variant comprises or consists of an amino acid sequence as set forth in any one of SEQ ID NO: 38-40, 74-76, 82, 96-100, 120-122, 141-143, 186-188, 222-224, 230. In an instance not encompassed by the wording of the claims, a preferred sequence variant belongs to the Family « NIVKPLPPV » (SEQ ID NO: 257). In an instance not encompassed by the wording of the claims, a preferred sequence variant comprises or consists of an amino acid sequence as set forth in any one of SEQ ID NO: 12, 34-36, 70-72, 119, 139, 161, 182-184, 218-220. In an instance not encompassed by the wording of the claims, a preferred sequence variant belongs to the Family « NLFYWYEGL » (SEQ ID NO: 258). In an instance not encompassed by the wording of the claims, a preferred sequence variant comprises or consists of an amino acid sequence as set forth in any one of SEQ ID NO: 21-23, 54-56, 110, 129, 168-170, 201-203. In an instance not encompassed by the wording of the claims, a preferred sequence variant belongs to the Family « QSSWAETTY » (SEQ ID NO: 259). In an instance not encompassed by the wording of the claims, a preferred sequence variant comprises or consists of an amino acid sequence as set forth in any one of SEQ ID NO: 92, 240. In an instance not encompassed by the wording of the claims, a preferred sequence variant belongs to the Family « RNIGSETWK » (SEQ ID NO: 260). In an instance not encompassed by the wording of the claims, a preferred sequence variant comprises or consists of an amino acid sequence as set forth in any one of SEQ ID NO: 101, 102. In an instance not encompassed by the wording of the claims, a preferred sequence variant belongs to the Family « VCLAIGCLY » (SEQ ID NO: 261). In an instance not encompassed by the wording of the claims, a preferred sequence variant comprises or consists of an amino acid sequence as set forth in any one of SEQ ID NO: 210. In an instance not encompassed by the wording of the claims, a preferred sequence variant belongs to the Family « VENETYTLK » (SEQ ID NO: 262). In an instance not encompassed by the wording of the claims, a preferred sequence variant comprises or consists of an amino acid sequence as set forth in any one of SEQ ID NO: 30, 63, 103-104, 116, 136, 177, 211. In an instance not encompassed by the wording of the claims, a preferred sequence variant belongs to the Family « WLPFGFILI » (SEQ ID NO: 263). In an instance not encompassed by the wording of the claims, a preferred sequence variant comprises or consists of an amino acid sequence as set forth in any one of SEQ ID NO: 31, 64, 178, 212, 267 - 274, 279, 335 and 336. In an instance not encompassed by the wording of the claims, a preferred sequence variant belongs to the Family « WQYLLCSWK» (SEQ ID NO: 264). In an instance not encompassed by the wording of the claims, a preferred sequence variant comprises or consists of an amino acid sequence as set forth in any one of SEQ ID NO: 41, 77, 189, 225. In an instance not encompassed by the wording of the claims, a preferred sequence variant belongs to the Family « WSDKQCWEG » (SEQ ID NO: 265). In an instance not encompassed by the wording of the claims, a preferred sequence variant comprises or consists of an amino acid sequence as set forth in any one of SEQ ID NO: 20, 53, 86-88, 128, 150-152, 167, 200, 234-236. In an instance not encompassed by the wording of the claims, a preferred sequence variant belongs to the Family « WLPFGFILIL» (SEQ ID NO: 276). In an instance not encompassed by the wording of the claims as such, a preferred sequence variant comprises or consists of an amino acid sequence as set forth in any one of SEQ ID NOs: 279, 335 and 336. In an instance not encompassed by the wording of the claims, a preferred sequence variant belongs to the Family « FLISTTFGCT» (SEQ ID NO: 331). In an instance not encompassed by the wording of the claims, a preferred sequence variant comprises or consists of an amino acid sequence as set forth in any one of SEQ ID NOs: 337 - 339. In an instance not encompassed by the wording of the claims, a preferred sequence variant belongs to the Family « YLYLQWQPPL » (SEQ ID NO: 332). In an instance not encompassed by the wording of the claims, a preferred sequence variant comprises or consists of an amino acid sequence as set forth in SEQ ID NO: 340. In an instance not encompassed by the wording of the claims, a preferred sequence variant belongs to the Family « GVLLDTNYNL » (SEQ ID NO: 333). In an instance not encompassed by the wording of the claims, a preferred sequence variant comprises or consists of an amino acid sequence as set forth in SEQ ID NOs 341 or 342. In an instance not encompassed by the wording of the claims, a preferred sequence variant belongs to the Family « FQLQNIVKPL » (SEQ ID NO: 334). In an instance not encompassed by the wording of the claims, a preferred sequence variant comprises or consists of an amino acid sequence as set forth in SEQ ID NOs 343 or 344.

In an instance not encompassed by the wording of the claims as such, the (poly)peptide for use as disclosed herein is selected from the group consisting of peptides or polypeptides comprising or consisting of an amino acid sequence according to any one of SEQ ID NOs: 12, 19, 21, 22, 23, 27, 28, 29, 31, 34, 35, 36, 52, 54, 55, 56, 60, 61, 62, 64, 69, 70, 71, 72, 110, 114, 115, 119, 129, 133, 134, 135, 139, 161, 166, 168, 169, 170, 174, 175, 176, 178, 182, 183, 184, 199, 201, 202, 203, 207, 208, 209, 212, 217, 218, 219, 220, 267, 268, 269 and 279.

In an instance not encompassed by the wording of the claims as such, the (poly)peptide for use as disclosed herein is selected from the group consisting of peptides or polypeptides comprising, or consisting of, any one of the amino acid sequences SEQ ID NO: 31, 64, 178, 192, 212, 267 and 279. In an instance not encompassed by the wording of the claims, the (poly)peptide comprises or consists of an amino acid sequence according to any one of SEQ ID NOs 31, 279, 64, 178, 212, 335 and 336. In an instance not encompassed by the wording of the claims as such , the (poly)peptide comprises or consists of an amino acid sequence according to any one of SEQ ID NO: 31, SEQ ID NO: 192 or SEQ ID NO: 279.

In an instance not encompassed by the wording of the claims as such, the (poly)peptide for use as defined herein is a peptide or polypeptide comprising, or consisting of, the amino acid sequence of SEQ ID NO: 279.

In an instance not encompassed by the wording of the claims , the (poly)peptide for use as disclosed herein comprises a sequence variant of the IL13RA2 epitope, wherein the sequence variant is a microbiota sequence variant.

The term "microbiota sequence variant" refers to a sequence variant of the (human) reference epitope, such as an IL13RA2 epitope, which is found in microbiota (for example, as a part of a microbiota protein, for example which is distinct from IL13RA2). In other words, the "microbiota sequence variant" is a microbiota sequence (sequence of microbiota origin), which is a sequence variant of the (human) reference epitope, such as an IL13RA2 epitope. That is, the "microbiota sequence variant" is a microbiota sequence (sequence of microbiota origin) which is similar, but contains at least one alteration, in comparison to the (human) reference epitope, such as an IL13RA2 epitope. Accordingly, the "microbiota sequence variant" is a sequence naturally occurring in microbiota (and *not* a sequence variant of a microbiota sequence).

Accordingly, the term "microbiota sequence variant" refers to a (poly)peptide sequence found in microbiota, i.e. of microbiota origin (once the sequence was identified in microbiota, it can usually also be obtained by recombinant measures well-known in the art). A "microbiota sequence variant" may refer to a complete (poly)peptide found in microbiota or, preferably, to a fragment of a (complete) microbiota (poly)peptide/protein having a length of at least 5 amino acids, preferably at least 6 amino acids, more preferably at least 7 amino acids, and even more preferably at least 8 amino acids. For example, the "microbiota sequence variant" may be a fragment of a microbiota protein/nucleic acid molecule, the fragment having a length of 9 or 10 amino acids. Preferably, the microbiota sequence variant is a fragment of a microbiota protein as described above. Preferably, the microbiota sequence variant has a length of 8 - 12 amino acids, more preferably the microbiota sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the microbiota sequence variant has a length of 13 - 24 amino acids. Peptides having such a length can bind to MHC (major histocompatibility complex) class II (MHC II), which is crucial for a CD4+ T-cell (T helper cell) response.

The term "microbiota", as used herein, refers to commensal, symbiotic and pathogenic microorganisms found in and on all multicellular organisms studied to date from plants to animals. In particular, microbiota have been found to be crucial for immunologic, hormonal and metabolic homeostasis of their host. Microbiota include bacteria, archaea, protists, fungi and viruses. Accordingly, the microbiota sequence variant is preferably selected from the group consisting of bacterial sequence variants, archaea sequence variants, protist sequence variants, fungi sequence variants and viral sequence variants. More preferably, the microbiota sequence variant is a bacterial sequence variant or an archaea sequence variant. Most preferably, the microbiota sequence variant is a bacterial sequence variant, i.e. a peptide of bacterial origin (which may exist in bacteria as a partial sequence of a larger bacterial (poly)peptide or protein or in the form of the peptide "as such").

Anatomically, microbiota reside on or within any of a number of tissues and biofluids, including the skin, conjunctiva, mammary glands, vagina, placenta, seminal fluid, uterus, ovarian follicles, lung, saliva, oral cavity (in particular oral mucosa), and the gastrointestinal tract, in particular the gut. In the context of the present invention the microbiota sequence variant is preferably a sequence variant of microbiota of the gastrointestinal tract (microorganisms residing in the gastrointestinal tract), more preferably a sequence variant of microbiota of the gut (microorganisms residing in the gut). Accordingly, it is most preferred that the microbiota sequence variant is a gut bacterial sequence variant (i.e. a sequence variant of bacteria residing in the gut).

While microbiota can be found in and on many multicellular organisms (all multicellular organisms studied to date from plants to animals), microbiota found in and on mammals are preferred. Mammals contemplated by the present invention include for example human, primates, domesticated animals such as cattle, sheep, pigs, horses, laboratory rodents and the like. Microbiota found in and on humans are most preferred. Such microbiota are referred to herein as "mammalian microbiota" or "human microbiota" (wherein the term mammalian/human refers specifically to the localization/residence of the microbiota). Preferably, the reference epitope, such as the IL13RA2 epitope, is of the same species, in/on which the microbiota (of the microbiota sequence variant) reside, e.g. human. Preferably, the microbiota sequence variant is a human microbiota sequence variant. Accordingly, it is preferred that the reference epitope, such as the IL13RA2 epitope, is a human reference epitope, such as a human IL13RA2 epitope.

The inventive idea to use a microbiota sequence variant of a (human) reference epitope, such as an IL13RA2 epitope, is based on the surprising finding that the present inventors identified such microbiota sequence variants of (human) reference epitopes, such as IL13RA2 epitopes, in the microbiome of the human gut. Without being bound to any theory, this offers a possible way to bypass the repertoire restriction of human T cells due to clonal depletion of T cells recognizing self-antigens. In particular, antigens/epitopes distinct from self-antigens, but sharing sequence similarity with the self-antigen, (i) can still be recognized due to the cross-reactivity of the T-cell receptor (see, for example, Degauque et al., Cross-Reactivity of TCR Repertoire: Current Concepts, Challenges, and Implication for Allotransplantation. Frontiers in Immunology. 2016;7:89. doi:10.3389/fimmu.2016.00089; Nelson et al., T cell receptor cross-reactivity between similar foreign and self peptides influences naive cell population size and autoimmunity. Immunity. 2015 Jan 20;42(1):95-107); and (ii) it is expected that such antigens/epitopes are recognized by T cell/TCR that have not been depleted during T cell education process. Accordingly, microbiota sequence variants of a human self antigen, such as IL13RA2, are able to elicit a strong immune response leading to clonal expansion of T cell harboring potential cross reactivity with self-antigens.

The human microbiome, which is composed of thousands of different bacterial species, is a large source of genetic diversity and potential antigenic components. The gut can be considered as the largest area of contact and exchange with microbiota. As a consequence, the gut is the largest immune organ in the body. Specialization and extrathymic T cell maturation in the human gut epithelium is known now for more than a decade. The gut contains a large panel of immune cells that could recognize our microbiota and which are tightly controlled by regulatory mechanisms.

As disclosed herein, the large repertoire of bacterial species existing in the gut provides an incredible source of antigens (microbiota sequence variants) with potential similarities with human antigens, such as IL13RA2. These microbiota sequence variants not encompassed by the wording of the claims are presented to specialized cells in a complex context, with large amount of co-signals delivered to immune cells as TLR activators. As a result, microbiota sequence variants not encompassed by the wording of the claims may elicit full functional response and drive maturation of large T memory subset or some time lead to full clonal depletion or exhaustion. Identification of bacterial components sharing similarities with human antigens, such as IL13RA2, provides a new source for selection of epitopes of antigens, which (i) overcome the problem of T cell depletion and (ii) have already "primed" the immune system in the gut, thereby providing for stronger immune responses as compared to sequence variants of antigens/epitopes of other sources and artificially mutated antigens/epitopes.

In an instance not encompassed by the wording of the claims microbiota sequence variants include the amino acid sequences according to SEQ ID NO: 31, SEQ ID NO: 192 or SEQ ID NO: 279. In an instance not encompassed by the wording of the claims as such, it is preferred that the (poly)peptide for use as disclosed herein comprises or consists of an amino acid sequence according to SEQ ID NO: 31, SEQ ID NO: 192 or SEQ ID NO: 279.

In an instance not encompassed by the wording of the claims as such, the (poly)peptide for use as disclosed herein is a peptide or polypeptide comprising, or consisting of, the amino acid sequence of SEQ ID NO: 279.

In an instance not encompassed by the wording of the claims, the (poly)peptide for use as disclosed herein does not comprise an amino acid sequence as set forth in SEQ ID NO: 282 (ALPFGFILV). Additionally or alternatively, the (poly)peptide may not comprise an amino acid sequence as set forth in SEQ ID NO: 283 (WLPFGFILV) or SEQ ID NO: 284 (ELPFGFILV).

In general, the (poly)peptide for use as disclosed herein may be of any length. In an instance not encompassed by the wording of the claims, the length of the (poly)peptide comprising the epitope of IL13RA2 or the sequence variant thereof for use as disclosed herein does not exceed 350 amino acids. For example, the maximum length of the (poly)peptide for use according to the present invention may be 300 or 250 amino acids. In an instance not encompassed by the wording of the claims, the maximum length of the IL13RA2 (poly)peptide for use as disclosed herein does not exceed 200 amino acids, e.g., not more than 190, 180, 170, 160, 150, 140, 130, 120, 110, 100, 95, 90, 85, 80, 75, 70, 65, 60, 55, 50, 45, 40, 35, 30, 29, 28, 27, 26, 25, 24, 23, 22, 21, 20, 19, 18, 17, 16, 15, 14 or 13 amino acids. In an instance not encompassed by the wording of the claims, the (poly)peptide consists of the epitope of IL13RA2 or the sequence variant thereof. In an instance not encompassed by the wording of the claims, the (poly)peptide may have a length of 8 - 12 amino acids, more preferably 8 - 10 amino acids and even more preferably 9 or 10 amino acids.

In an instance not encompassed by the wording of the claims, the (poly)peptide for use as disclosed herein may not bind to IL-13 and/or not inhibit IL-13. Without being bound to any theory, the present inventors assume that the (poly)peptide for use as disclosed herein exerts its effects by directing an immune response against IL13RA2 and/or IL13RA2 expressing cells. This approach differs considerably from the "normal" function of IL13RA2, which is binding and/or inhibiting interleukin 13 (IL-13).

### (Poly)peptide comprising a BIRC5 epitope or a sequence variant thereof

The present disclosure provides a (poly)peptide not encompassed by the wording of the claims comprising or consisting of an epitope of BIRC5 (Baculoviral IAP Repeat Containing 5) or a sequence variant thereof having at least 70% (preferably at least 75%, more preferably at least 80%, even more preferably at least 85%, and most preferably at least 90%) sequence identity for use in prevention and/or treatment of an adrenal cancer. In an instance not encompassed by the wording of the claims, the present disclosure also provides a method for ameliorating, reducing, preventing and/or treating an adrenal cancer or for reducing or preventing its recurrence in a subject, comprising administering to the subject a (poly)peptide comprising an epitope of BIRC5 or a sequence variant thereof having at least 70% (preferably at least 75%, more preferably at least 80%, even more preferably at least 85%, and most preferably at least 90%) sequence identity.

As described above, the term "epitope", as used herein, refers to a peptide, which can be recognized by the immune system. In general, an "epitope" (also known as "antigenic determinant"), is the part (or fragment) of an antigen that is recognized by the immune system, in particular by antibodies, T cell receptors, and/or B cell receptors. Thus, one antigen has at least one epitope, i.e. a single antigen has one or more epitopes. An "antigen" typically serves as a target for the receptors of an adaptive immune response, in particular as a target for antibodies, T cell receptors, and/or B cell receptors. In the context of the present invention the antigen may be BIRC 5 (Baculoviral IAP Repeat Containing 5). Other antigens, which may optionally be of interest in the context of the present invention, include IL13RA2 (as described above) and FOXM1 (Forkhead Box M1). With regard to BIRC5, human BIRC5 is preferred. The sequence of human BIRC5 is shown in the following:

MGAPTLPPAWQPFLKDHRISTFKNWPFLEGCACTPERMAEAGFIHCPTENEPDLAQCFFCFKELEGWEPDDD
PIEEHKKHSSGCAFLSVKKQFEELTLGEFLKLDRERAKNKIAKETNNKKKEFEETAKKVRRAIEQLAAMD

[SEQ ID NO: 285]

In an instance not encompassed by the wording of the claims, a preferred (poly)peptide for use as disclosed herein comprises an epitope of human BIRC5. In an instance not encompassed by the wording of the claims, a preferred (poly)peptide for use as disclosed herein may comprise an amino acid sequence according to SEQ ID NO: 285 or a fragment thereof comprising or consisting of an epitope or a sequence variant of such a fragment. As used herein, a "fragment" of an antigen comprises at least 5 consecutive amino acids of the antigen, preferably at least 6 consecutive amino acids of the antigen, more preferably at least 7 consecutive amino acids of the antigen, even more preferably at least 8 consecutive amino acids of the antigen and most preferably at least 9 consecutive amino acids of the antigen. A "sequence variant" is as defined herein, namely a sequence variant has an (amino acid) sequence which is at least 70% (preferably at least 75%, more preferably at least 80%, even more preferably at least 85%, most preferably at least 90%) identical to the reference sequence. A "functional" sequence variant means in the context of an antigen/antigen fragment/epitope, that the function of the epitope(s), e.g. comprised by the antigen (fragment), is not impaired or abolished.

In the context of the present invention, the term "epitope" is mainly used to designate T cell epitopes, which are presented on the surface of an antigen-presenting cell, where they are bound to Major Histocompatibility Complex (MHC). T cell epitopes presented by MHC class I molecules are typically, but not exclusively, peptides between 8 and 12 amino acids in length, whereas MHC class II molecules present longer peptides, generally, but not exclusively, between 12 and 25 amino acids in length. Preferably, the epitope of BIRC5 or the sequence variant thereof has a length of 8 - 12 amino acids, more preferably of 8 - 10 amino acids and most preferably of 9 or 10 amino acids.

Several epitopes of BIRC5 are known to the skilled person and can be identified by using cancer/tumor epitope databases, e.g. from van der Bruggen P, Stroobant V, Vigneron N, Van den Eynde B. Peptide database: T cell-defined tumor antigens. Cancer Immun 2013; URL: http://www.cancerimmunity.org/peptide/, wherein human tumor antigens recognized by CD4+ or CD8+ T cells are classified into four major groups on the basis of their expression pattern, or from the database "Tantigen" (TANTIGEN version 1.0, Dec 1, 2009; developed by Bioinformatics Core at Cancer Vaccine Center, Dana-Farber Cancer Institute; URL: http://cvc.dfci.harvard.edu/tadb/).

An exemplified epitope of BIRC5 not encompassed by the wording of the claims has an amino acid sequence as set forth in SEQ ID NO: 286:
LTLGEFLKL
(SEQ ID NO: 286)

In an instance not encompassed by the wording of the claims the (poly)peptide for use as disclosed herein comprises or consists of a sequence variant of a (human) BIRC5 epitope as described herein. For example the (poly)peptide for use as disclosed herein may comprise or consist of a sequence variant of an amino acid as set forth in SEQ ID NO: 286.

As described above, a "sequence variant" is similar, but contains at least one alteration, in comparison to the reference sequence, in particular a (human) reference epitope, such as a (human) BIRC5 epitope. A "sequence variant" may be a recombinant sequence variant (which does not occur in nature), for example which is designed *in vitro,* e.g. by mutating the reference sequence, in particular a (human) reference epitope, such as a (human) BIRC5 epitope. A "sequence variant" may also be a naturally occurring sequence variant, such as a naturally occurring peptide or a fragment of a naturally occurring protein (for example it may be found in a species other than human, such as a microbiota sequence variant as described below), which shares sequence identity with the reference sequence, such as a (human) BIRC5 epitope. Such a naturally occurring protein or peptide (which comprises a sequence variant of an epitope of a tumor-associated antigen, such as BIRC5 may be a homologue of the tumor-associated antigen or it may be unrelated to the tumor-associated antigen. For example, a (human) BIRC5 epitope may be an BIRC5 homologue or it may be unrelated to BIRC5. Preferably, the sequence variant has a length of at least 5 amino acids, more preferably at least 6 amino acids, even more preferably at least 7 amino acids, and most preferably at least 8 amino acids. For example, the "sequence variant" may have a length of 9 or 10 amino acids. Preferably, the sequence variant has a length of 8 - 12 amino acids, more preferably the sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the sequence variant has a length of 13 - 24 amino acids. Peptides having such a length can bind to MHC (major histocompatibility complex) class II (MHC II), which is crucial for a CD4+ T-cell (T helper cell) response.

In general, the term "sequence variant", as used herein, i.e. throughout the present application, refers to a sequence which is similar (meaning in particular at least 50% sequence identity, see below), but not (100%) identical, to a reference sequence (such as (human) BIRC5 or an epitope or fragment thereof). Accordingly, a sequence variant contains at least one alteration in comparison to a reference sequence. For example, in a sequence variant one or more of the amino acids or nucleotides of the reference sequence is deleted or substituted, or one or more amino acids or nucleotides are inserted into the sequence of the reference sequence. Therefore, the "sequence variant" is similar, but contains at least one alteration, in comparison to its reference sequence, such as an BIRC5 epitope sequence. Preferably, a sequence variant shares, in particular over the whole length of the sequence, at least 60%, preferably at least 70%, more preferably at least 75%, more preferably at least 80%, even more preferably at least 85%, and most preferably at least 90% sequence identity with a reference sequence (such as the BIRC5 epitope sequence).

A sequence variant may preserve the specific function of the reference sequence. In the context of the present invention, this function may be the functionality as an "epitope", i.e. it can be recognized by the immune system, in particular by antibodies, T cell receptors, and/or B cell receptors and, preferably, it can elicit an immune response. Accordingly, is preferred that the (poly)peptide for use according to the present invention, in particular the epitope of BIRC5 or the sequence variant thereof, is immunogenic. In other words, the (poly)peptide, in particular the epitope of BIRC5 or the sequence variant thereof, is preferably capable of eliciting an immune response.

The term "sequence variant" includes nucleotide sequence variants and amino acid sequence variants. For example, an amino acid sequence variant has an altered sequence in which one or more of the amino acids is deleted or substituted in comparison to the reference sequence, or one or more amino acids are inserted in comparison to the reference amino acid sequence. As a result of the alterations, the amino acid sequence variant has an amino acid sequence which is at least 50%, preferably at least 60%, more preferably at least 70%, more preferably at least 75%, even more preferably at least 80%, even more preferably at least 85%, and most preferably at least 90% identical to the reference sequence. For example, variant sequences which are at least 90% identical have no more than 10 alterations (i.e. any combination of deletions, insertions or substitutions) per 100 amino acids of the reference sequence.

In the context of the present invention, an amino acid sequence "sharing a sequence identity" of at least, for example, 70% to a query amino acid sequence of the present invention, is intended to mean that the sequence of the subject amino acid sequence is identical to the query sequence except that the subject amino acid sequence may include up to three amino acid alterations per each 10 amino acids of the query amino acid sequence. In other words, to obtain an amino acid sequence having a sequence of at least 70% identity to a query amino acid sequence, up to 30% (3 of 10) of the amino acid residues in the subject sequence may be inserted or substituted with another amino acid or deleted, preferably within the above definitions of variants or fragments. The same, of course, also applies similarly to nucleic acid sequences.

For (amino acid or nucleic acid) sequences without exact correspondence, a "% identity" of a first sequence (e.g., the sequence variant) may be determined with respect to a second sequence (e.g., the reference sequence). In general, the two sequences to be compared may be aligned to give a maximum correlation between the sequences. This may include inserting "gaps" in either one or both sequences, to enhance the degree of alignment. A % identity may then be determined over the whole length of each of the sequences being compared (so-called "global alignment"), that is particularly suitable for sequences of the same or similar length, or over shorter, defined lengths (so-called "local alignment"), that is more suitable for sequences of unequal length.

Methods for comparing the identity (sometimes also referred to as "similarity" or "homology") of two or more sequences are well known in the art. The percentage to which two (or more) sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

Preferably, the sequence variant not encompassed by the wording of the claims differs from the reference sequence, in particular a (human) reference epitope, such as the (human) BIRC5 epitope, (only) in primary and/or secondary anchor residues for MHC molecules. More preferably, the sequence variant differs from the reference sequence, in particular a (human) reference epitope, such as the (human) BIRC5 epitope, (only) in that it comprises amino acid substitutions (only) in primary and/or secondary anchor residues for MHC molecules. Anchor residues for the HLA subtypes are known in the art, and can be defined by large throughput analysis of structural data of existing p-HLA complexes in the Protein Data Bank. Moreover, anchor motifs for MHC subtypes can also be found in IEDB (URL: www.iedb.org; browse by allele) or in SYFPEITHI (URL: http://www.syfpeithi.de/). For example, for a 9 amino acid size HLA.A2.01 peptide, the peptide primary anchor residues, providing the main contact points, are located at residue positions P1, P2 and P9.

In a particular instance not encompassed by the wording of the claims, it is preferred that the core sequence of the sequence variant is identical with the core sequence of the reference sequence, in particular a (human) reference epitope, such as the (human) BIRC5 epitope, wherein the "core sequence" consists of all amino acids except the (at least) three most N-terminal and the (at least) three most C-terminal amino acids of the (human) reference epitope, such as the (human) BIRC5 epitope. Accordingly, it is preferred that any alterations in the sequence variant in comparison to the (human) reference epitope, such as the (human) BIRC5 epitope, are preferably located within the three N-terminal and/or within the three C-terminal amino acids of the (human) reference epitope, such as the (human) BIRC5 epitope, but not in the "core sequence" of the (human) reference epitope, such as the (human) BIRC5 epitope (amino acids in the middle of the of the (human) reference epitope sequence, e.g. in the middle of the (human) BIRC5 epitope sequence). This does not mean that all three N-terminal and/or C-terminal amino acids of a (human) reference epitope, such as a (human) BIRC5 epitope, must be altered, but only that those are the preferred amino acid positions, where an amino acid may optionally be altered. For example, in a (human) reference epitope, such as an BIRC5 epitope, of nine amino acids, the three middle amino acids may represent the core sequence and alterations may preferably only occur at any of the three N-terminal and the three C-terminal amino acid positions.

In a particular instance not encompassed by the wording of the claims, the core sequence of the (human) reference epitope, such as the (human) BIRC5 epitope, consists of all amino acids except the two most N-terminal and the two most C-terminal amino acids of the (human) reference epitope, such as the (human) BIRC5 epitope. For example, in a (human) reference epitope, such as a (human) BIRC5 epitope, of nine amino acids, the five middle amino acids may represent the core sequence and alterations may preferably only occur at any of the two N-terminal and the two C-terminal amino acid positions of the (human) reference epitope, such as the (human) BIRC5 epitope.

In a particular instance not encompassed by the wording of the claims the core sequence of the (human) reference epitope, such as the (human) BIRC5 epitope, consists of all amino acids except the most N-terminal and the most C-terminal amino acid of the (human) reference epitope, such as the (human) BIRC5 epitope. For example, in a (human) reference epitope, such as a (human) BIRC5 epitope, of nine amino acids, the seven middle amino acids may represent the core sequence and alterations may preferably only occur at the N-terminal position (P1) and the C-terminal amino acid position (P9) of the (human) reference epitope, such as the (human) BIRC5 epitope.

In a particular instance not encompassed by the wording of the claims, the core sequence of the (human) reference epitope, such as the (human) BIRC5 epitope, consists of all amino acids except the two most N-terminal amino acids and the most C-terminal amino acid of the (human) reference epitope, such as the (human) BIRC5 epitope. For example, in a (human) reference epitope, such as a (human) BIRC5 epitope, of nine amino acids, the six middle amino acids may represent the core sequence and alterations may preferably only occur at any of the two N-terminal positions (P1 and P2) and the C-terminal amino acid position (P9) of the (human) reference epitope, such as the (human) BIRC5 epitope.

In a particular instance not encompassed by the wording of the claims the sequence variant, e.g. having a length of nine amino acids, comprises at position 1 (P1; the most N-terminal amino acid position) a phenylalanine (F) or a lysine (K). In a particular instance not encompassed by the wording of the claims, it is preferred that the sequence variant, e.g. having a length of nine amino acids, comprises at position 2 (P2) a leucine (L) or a methionine (M). Moreover, it is preferred that the sequence variant, e.g. having a length of nine amino acids, comprises at position 9 (P9) a valine (V) or a leucine (L). In a particular instance not encompassed by the wording of the claims, the sequence variant, e.g. having a length of nine amino acids, comprises at position 1 (P1; the most N-terminal amino acid position) a phenylalanine (F) or a lysine (K), at position 2 (P2) a leucine (L) or a methionine (M) and/or at position 9 (P9) a valine (V) or a leucine (L).

Moreover, amino acid substitutions, in particular at positions other than the anchor position(s) for MHC molecules (e.g., P1, P2 and P9 for MHC-I subtype HLA.A2.01), are preferably conservative amino acid substitutions. Examples of conservative substitutions include substitution of one aliphatic residue for another, such as Ile, Val, Leu, or Ala for one another; or substitutions of one polar residue for another, such as between Lys and Arg; Glu and Asp; or Gln and Asn. Other such conservative substitutions, for example, substitutions of entire regions having similar hydrophobicity properties, are well known (Kyte and Doolittle, 1982, J. Mol. Biol. 157(1):105- 132). Examples of conservative amino acid substitutions are presented in Table 2 above.

Preferred examples of sequence variants not encompassed by the wording of the claims are sequence variants of a (human) BIRC5 epitope having an amino acid sequences as set forth in SEQ ID NO: 286. In a particular instance not encompassed by the wording of the claims as such, it is preferred that the (poly)peptide for use as disclosed herein comprises an amino acid sequence as set forth in any one of SEQ ID NOs 287 - 289:
YTLGEFLYI
   (SEQ ID NO: 287)
GLLGEFLQI
   (SEQ ID NO: 288)
FMLGEFLKL
   (SEQ ID NO: 289)

The amino acid sequences as set forth in SEQ ID NOs 287 - 289 refer to the sequence variants of the BIRC5 epitope of SEQ ID NO: 286.

In a particular instance not encompassed by the wording of the claims as such, the BIRC5-related (poly)peptide for use as disclosed herein is a peptide or polypeptide comprising, or consisting of, the amino acid sequence of SEQ ID NO: 289.

In a particular instance not encompassed by the wording of the claims as such, the (poly)peptide for use as disclosed herein comprises a sequence variant of the BIRC5 epitope, wherein the sequence variant is a microbiota sequence variant.

The term "microbiota sequence variant" refers to a sequence variant of the (human) reference epitope, such as an BIRC5 epitope, which is found in microbiota (for example, as a part of a microbiota protein, for example which is distinct from BIRC5). In other words, the "microbiota sequence variant" is a microbiota sequence (sequence of microbiota origin), which is a sequence variant of the (human) reference epitope, such as an BIRC5 epitope. That is, the "microbiota sequence variant" is a microbiota sequence (sequence of microbiota origin) which is similar, but contains at least one alteration, in comparison to the (human) reference epitope, such as an BIRC5 epitope. Accordingly, the "microbiota sequence variant" is a sequence naturally occurring in microbiota (and *not* a sequence variant of a microbiota sequence).

Accordingly, the term "microbiota sequence variant" refers to a (poly)peptide sequence found in microbiota, i.e. of microbiota origin (once the sequence was identified in microbiota, it can usually also be obtained by recombinant measures well-known in the art). A "microbiota sequence variant" may refer to a complete (poly)peptide found in microbiota or, preferably, to a fragment of a (complete) microbiota (poly)peptide/protein having a length of at least 5 amino acids, preferably at least 6 amino acids, more preferably at least 7 amino acids, and even more preferably at least 8 amino acids. For example, the "microbiota sequence variant" may be a fragment of a microbiota protein/nucleic acid molecule, the fragment having a length of 9 or 10 amino acids. Preferably, the microbiota sequence variant is a fragment of a microbiota protein as described above. Preferably, the microbiota sequence variant has a length of 8 - 12 amino acids, more preferably the microbiota sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the microbiota sequence variant has a length of 13 - 24 amino acids. Peptides having such a length can bind to MHC (major histocompatibility complex) class II (MHC II), which is crucial for a CD4+ T-cell (T helper cell) response.

The term "microbiota", as used herein, refers to commensal, symbiotic and pathogenic microorganisms found in and on all multicellular organisms studied to date from plants to animals. In particular, microbiota have been found to be crucial for immunologic, hormonal and metabolic homeostasis of their host. Microbiota include bacteria, archaea, protists, fungi and viruses. Accordingly, the microbiota sequence variant is preferably selected from the group consisting of bacterial sequence variants, archaea sequence variants, protist sequence variants, fungi sequence variants and viral sequence variants. More preferably, the microbiota sequence variant is a bacterial sequence variant or an archaea sequence variant. Most preferably, the microbiota sequence variant is a bacterial sequence variant, i.e. a peptide of bacterial origin (which may exist in bacteria as a partial sequence of a larger bacterial (poly)peptide or protein or in the form of the peptide "as such").

Anatomically, microbiota reside on or within any of a number of tissues and biofluids, including the skin, conjunctiva, mammary glands, vagina, placenta, seminal fluid, uterus, ovarian follicles, lung, saliva, oral cavity (in particular oral mucosa), and the gastrointestinal tract, in particular the gut. In the context of the present invention the microbiota sequence variant is preferably a sequence variant of microbiota of the gastrointestinal tract (microorganisms residing in the gastrointestinal tract), more preferably a sequence variant of microbiota of the gut (microorganisms residing in the gut). Accordingly, it is most preferred that the microbiota sequence variant is a gut bacterial sequence variant (i.e. a sequence variant of bacteria residing in the gut).

While microbiota can be found in and on many multicellular organisms (all multicellular organisms studied to date from plants to animals), microbiota found in and on mammals are preferred. Mammals contemplated by the present invention include for example human, primates, domesticated animals such as cattle, sheep, pigs, horses, laboratory rodents and the like. Microbiota found in and on humans are most preferred. Such microbiota are referred to herein as "mammalian microbiota" or "human microbiota" (wherein the term mammalian/human refers specifically to the localization/residence of the microbiota). Preferably, the reference epitope, such as the BIRC5 epitope, is of the same species, in/on which the microbiota (of the microbiota sequence variant) reside, e.g. human. Preferably, the microbiota sequence variant is a human microbiota sequence variant. Accordingly, it is preferred that the reference epitope, such as the BIRC5 epitope, is a human reference epitope, such as a human BIRC5 epitope.

The inventive idea to use a microbiota sequence variant of a (human) reference epitope, such as an BIRC5 epitope, is based on the surprising finding that the present inventors identified such microbiota sequence variants of (human) reference epitopes, such as BIRC5 epitopes, in the microbiome of the human gut. Without being bound to any theory, this offers a possible way to bypass the repertoire restriction of human T cells due to clonal depletion of T cells recognizing self-antigens. In particular, antigens/epitopes distinct from self-antigens, but sharing sequence similarity with the self-antigen, (i) can still be recognized due to the cross-reactivity of the T-cell receptor (see, for example, Degauque et al., Cross-Reactivity of TCR Repertoire: Current Concepts, Challenges, and Implication for Allotransplantation. Frontiers in Immunology. 2016;7:89. doi:10.3389/fimmu.2016.00089; Nelson et al., T cell receptor cross-reactivity between similar foreign and self peptides influences naive cell population size and autoimmunity. Immunity. 2015 Jan 20;42(1):95-107); and (ii) it is expected that such antigens/epitopes are recognized by T cell/TCR that have not been depleted during T cell education process. Accordingly, microbiota sequence variants of a human self antigen, such as BIRC5, are able to elicit a strong immune response leading to clonal expansion of T cell harboring potential cross reactivity with self-antigens.

The human microbiome, which is composed of thousands of different bacterial species, is a large source of genetic diversity and potential antigenic components. The gut can be considered as the largest area of contact and exchange with microbiota. As a consequence, the gut is the largest immune organ in the body. Specialization and extrathymic T cell maturation in the human gut epithelium is known now for more than a decade. The gut contains a large panel of immune cells that could recognize our microbiota and which are tightly controlled by regulatory mechanisms.

According to the present invention, the large repertoire of bacterial species existing in the gut provides an incredible source of antigens (microbiota sequence variants) with potential similarities with human antigens, such as BIRC5. These microbiota sequence variants are presented to specialized cells in a complex context, with large amount of co-signals delivered to immune cells as TLR activators. As a result, microbiota sequence variants may elicit full functional response and drive maturation of large T memory subset or some time lead to full clonal depletion or exhaustion. Identification of bacterial components sharing similarities with human antigens, such as BIRC5, provides a new source for selection of epitopes of antigens, which (i) overcome the problem of T cell depletion and (ii) have already "primed" the immune system in the gut, thereby providing for stronger immune responses as compared to sequence variants of antigens/epitopes of other sources and artificially mutated antigens/epitopes.

In a particular instance not encompassed by the wording of the claims as such, microbiota sequence variants include the amino acid sequences according to SEQ ID NOs: 287 - 289. In a particular instance not encompassed by the wording of the claims as such, it is preferred that the (poly)peptide for use as disclosed herein comprises or consists of an amino acid sequence according to SEQ ID NO: 287, SEQ ID NO: 288 or SEQ ID NO: 289.

In a particular instance not encompassed by the wording of the claims as such, the (poly)peptide for use as disclosed herein is a peptide or polypeptide comprising, or consisting of, the amino acid sequence of SEQ ID NO: 289.

In general, the (poly)peptide for use as disclosed herein may be of any length. In a particular instance not encompassed by the wording of the claims, the length of the (poly)peptide comprising the epitope of BIRC5 or the sequence variant thereof for use as disclosed herein does not exceed 140 amino acids. For example, the maximum length of the (poly)peptide for use as disclosed herein may be 110 or 120 amino acids. In a particular instance not encompassed by the wording of the claims, the maximum length of the BIRC5 (poly)peptide for use as disclosed herein does not exceed 100 amino acids, e.g., not more than 95, 90, 85, 80, 75, 70, 65, 60, 55, 50, 45, 40, 35, 30, 29, 28, 27, 26, 25, 24, 23, 22, 21, 20, 19, 18, 17, 16, 15, 14 or 13 amino acids. In a particular instance not encompassed by the wording of the claims, the (poly)peptide consists of the epitope of BIRC5 or the sequence variant thereof. In a particular instance not encompassed by the wording of the claims, the (poly)peptide may have a length of 8 - 12 amino acids, more preferably 8 - 10 amino acids and even more preferably 9 or 10 amino acids.

### (Poly)peptide comprising a FOXM1 epitope or a sequence variant thereof

The present disclosure provides a (poly)peptide not encompassed by the wording of the claims comprising or consisting of an epitope of FOXM1 (Forkhead box protein M1) or a sequence variant thereof having at least 70% (preferably at least 75%, more preferably at least 80%, even more preferably at least 85%, and most preferably at least 90%) sequence identity for use in prevention and/or treatment of an adrenal cancer. In an instance not encompassed by the wording of the claims, the present disclosure also provides a method for ameliorating, reducing, preventing and/or treating an adrenal cancer or for reducing or preventing its recurrence in a subject, comprising administering to the subject a (poly)peptide comprising an epitope of FOXM1 or a sequence variant thereof having at least 70% (preferably at least 75%, more preferably at least 80%, even more preferably at least 85%, and most preferably at least 90%) sequence identity.

As described above, the term "epitope", as used herein, refers to a peptide, which can be recognized by the immune system. In general, an "epitope" (also known as "antigenic determinant"), is the part (or fragment) of an antigen that is recognized by the immune system, in particular by antibodies, T cell receptors, and/or B cell receptors. Thus, one antigen has at least one epitope, i.e. a single antigen has one or more epitopes. An "antigen" typically serves as a target for the receptors of an adaptive immune response, in particular as a target for antibodies, T cell receptors, and/or B cell receptors. In the context of the present invention the antigen may be FOXM1 (Forkhead box protein M1). Other antigens, which may optionally be of interest in the context of the present invention, include IL13RA2 (as described above) and BIRC5 (as described above). With regard to FOXM1, human FOXM1 is preferred. The sequence of human FOXM1 is shown in the following:

MKTSPRRPLILKRRRLPLPVQNAPSETSEEEPKRSPAQQESNQAEASKEVAESNSCKFPAGIKIINHPTMPNTQV
VAIPNNANIHSIITALTAKGKESGSSGPNKFILISCGGAPTQPPGLRPQTQTSYDAKRTEVTLETLGPKPAARDVN
LPRPPGALCEQKRETCADGEAAGCTINNSLSNIQWLRKMSSDGLGSRSIKQEMEEKENCHLEQRQVKVEEPSR
PSASWQNSVSERPPYSYMAMIQFAINSTERKRMTLKDIYTWIEDHFPYFKHIAKPGWKNSIRHNLSLHDMFVR
ETSANGKVSFWTIHPSANRYLTLDQVFKPLDPGSPQLPEHLESQQKRPNPELRRNMTIKTELPLGARRKMKPLL
PRVSSYLVPIQFPVNQSLVLQPSVKVPLPLAASLMSSELARHSKRVRIAPKVLLAEEGIAPLSSAGPGKEEKLLFGE
GFSPLLPVQTIKEEEIQPGEEMPHLARPIKVESPPLEEWPSPAPSFKEESSHSWEDSSQSPTPRPKKSYSGLRSPT
RCVSEMLVIQHRERRERSRSRRKQHLLPPCVDEPELLFSEGPSTSRWAAELPFPADSSDPASQLSYSQEVGGPFK
TPIKETLPISSTPSKSVLPRTPESWRLTPPAKVGGLDFSPVQTSQGASDPLPDPLGLMDLSTTPLQSAPPLESPQR
LLSSEPLDLISVPFGNSSPSDIDVPKPGSPEPQVSGLAANRSLTEGLVLDTMNDSLSKILLDISFPGLDEDPLGPDN
INWSQFIPELQ

[SEQ ID NO: 290]

In an instance not encompassed by the wording of the claims, a preferred (poly)peptide for use as disclosed herein comprises an epitope of human FOXM1. In an instance not encompassed by the wording of the claims, a preferred (poly)peptide for use as disclosed herein may comprise an amino acid sequence according to SEQ ID NO: 290 or a fragment thereof comprising or consisting of an epitope or a sequence variant of such a fragment. As used herein, a "fragment" of an antigen comprises at least 5 consecutive amino acids of the antigen, preferably at least 6 consecutive amino acids of the antigen, more preferably at least 7 consecutive amino acids of the antigen, even more preferably at least 8 consecutive amino acids of the antigen and most preferably at least 9 consecutive amino acids of the antigen. A "sequence variant" is as defined herein, namely a sequence variant has an (amino acid) sequence which is at least 70% (preferably at least 75%, more preferably at least 80%, even more preferably at least 85%, most preferably at least 90%) identical to the reference sequence. A "functional" sequence variant means in the context of an antigen/antigen fragment/epitope, that the function of the epitope(s), e.g. comprised by the antigen (fragment), is not impaired or abolished.

In the context of the present invention, the term "epitope" is mainly used to designate T cell epitopes, which are presented on the surface of an antigen-presenting cell, where they are bound to Major Histocompatibility Complex (MHC). T cell epitopes presented by MHC class I molecules are typically, but not exclusively, peptides between 8 and 12 amino acids in length, whereas MHC class II molecules present longer peptides, generally, but not exclusively, between 12 and 25 amino acids in length. Preferably, the epitope of FOXM1 or the sequence variant thereof has a length of 8 - 12 amino acids, more preferably of 8 - 10 amino acids and most preferably of 9 or 10 amino acids.

Several epitopes of FOXM1 are known to the skilled person and can be identified by using cancer/tumor epitope databases, e.g. from van der Bruggen P, Stroobant V, Vigneron N, Van den Eynde B. Peptide database: T cell-defined tumor antigens. Cancer Immun 2013; URL: http://www.cancerimmunity.org/peptide/, wherein human tumor antigens recognized by CD4+ or CD8+ T cells are classified into four major groups on the basis of their expression pattern, or from the database "Tantigen" (TANTIGEN version 1.0, Dec 1, 2009; developed by Bioinformatics Core at Cancer Vaccine Center, Dana-Farber Cancer Institute; URL: http://cvc.dfci.harvard.edu/tadb/).

Exemplified epitopes of FOXM1 not encompassed by the wording of the claims having an amino acid sequence as set forth in any one of SEQ ID NOs 291 - 301 are shown in Table 4 below.

**Table 4: Preferred examples of human FOXM1 epitopes, not encompassed by the wording of the claims.**

| **SEQ ID NO.** | **FOXM1 epitope sequence** |
|---|---|
| 291 | ILLDISFPG |
| 292 | LLDISFPGL |
| 293 | LMDLSTTPL |
| 294 | RVSSYLVPI |
| 295 | SLSKILLDI |
| 296 | SQLSYSQEV |
| 297 | WAAELPFPA |
| 298 | NLSLHDMFV |
| 299 | KMKPLLPRV |
| 300 | YLVPIQFPV |
| 301 | YMAMIQFAI |

Of those exemplified FOXM1 epitopes not encompassed by the wording of the claims, an epitope of FOXM1 having an amino acid sequence as set forth in SEQ ID NO: 293 is particularly preferred.

In an instance not encompassed by the wording of the claims the (poly)peptide for use as disclosed herein comprises or consists of an epitope of FOXM1 having an amino acid sequence as set forth in any one of SEQ ID NOs 291 - 301, most preferably as set forth in SEQ ID NO: 293.

In an instance not encompassed by the wording of the claims the FOXM1 (poly)peptide for use as disclosed herein comprises or consists of a sequence variant of a (human) FOXM1 epitope as described herein. For example the (poly)peptide for use as disclosed herein may comprise or consist of a sequence variant of an amino acid as set forth in any one of SEQ ID NOs 291 - 301, most preferably as set forth in SEQ ID NO: 293.

As described above, a "sequence variant" is similar, but contains at least one alteration, in comparison to the reference sequence, in particular a (human) reference epitope, such as a (human) FOXM1 epitope. A "sequence variant" may be a recombinant sequence variant (which does not occur in nature), for example which is designed *in vitro,* e.g. by mutating the reference sequence, in particular a (human) reference epitope, such as a (human) FOXM1 epitope. A "sequence variant" may also be a naturally occurring sequence variant, such as a naturally occurring peptide or a fragment of a naturally occurring protein (for example it may be found in a species other than human, such as a microbiota sequence variant as described below), which shares sequence identity with the reference sequence, such as a (human) FOXM1 epitope. Such a naturally occurring protein or peptide (which comprises a sequence variant of an epitope of a tumor-associated antigen, such as FOXM1 may be a homologue of the tumor-associated antigen or it may be unrelated to the tumor-associated antigen. For example, a (human) FOXM1 epitope may be an FOXM1 homologue or it may be unrelated to FOXM1. Preferably, the sequence variant has a length of at least 5 amino acids, more preferably at least 6 amino acids, even more preferably at least 7 amino acids, and most preferably at least 8 amino acids. For example, the "sequence variant" may have a length of 9 or 10 amino acids. Preferably, the sequence variant has a length of 8 - 12 amino acids, more preferably the sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the sequence variant has a length of 13 - 24 amino acids. Peptides having such a length can bind to MHC (major histocompatibility complex) class II (MHC II), which is crucial for a CD4+ T-cell (T helper cell) response.

In general, the term "sequence variant", as used herein, i.e. throughout the present application, refers to a sequence which is similar (meaning in particular at least 50% sequence identity, see below), but not (100%) identical, to a reference sequence (such as (human) FOXM1 or an epitope or fragment thereof). Accordingly, a sequence variant contains at least one alteration in comparison to a reference sequence. For example, in a sequence variant one or more of the amino acids or nucleotides of the reference sequence is deleted or substituted, or one or more amino acids or nucleotides are inserted into the sequence of the reference sequence. Therefore, the "sequence variant" is similar, but contains at least one alteration, in comparison to its reference sequence, such as an FOXM1 epitope sequence. Preferably, a sequence variant shares, in particular over the whole length of the sequence, at least 60%, preferably at least 70%, more preferably at least 75%, more preferably at least 80%, even more preferably at least 85%, and most preferably at least 90% sequence identity with a reference sequence (such as the FOXM1 epitope sequence).

A sequence variant may preserve the specific function of the reference sequence. In a particular instance not encompassed by the wording of the claims, this function may be the functionality as an "epitope", i.e. it can be recognized by the immune system, in particular by antibodies, T cell receptors, and/or B cell receptors and, preferably, it can elicit an immune response. In a particular instance not encompassed by the wording of the claims it is preferred that the (poly)peptide for use as disclosed herein, in particular the epitope of FOXM1 or the sequence variant thereof, is immunogenic. In other words, the (poly)peptide, in particular the epitope of FOXM1 or the sequence variant thereof, is preferably capable of eliciting an immune response.

The term "sequence variant" includes nucleotide sequence variants and amino acid sequence variants. For example, an amino acid sequence variant has an altered sequence in which one or more of the amino acids is deleted or substituted in comparison to the reference sequence, or one or more amino acids are inserted in comparison to the reference amino acid sequence. As a result of the alterations, the amino acid sequence variant has an amino acid sequence which is at least 50%, preferably at least 60%, more preferably at least 70%, more preferably at least 75%, even more preferably at least 80%, even more preferably at least 85%, and most preferably at least 90% identical to the reference sequence. For example, variant sequences which are at least 90% identical have no more than 10 alterations (i.e. any combination of deletions, insertions or substitutions) per 100 amino acids of the reference sequence.

In the context of the present invention, an amino acid sequence "sharing a sequence identity" of at least, for example, 70% to a query amino acid sequence of the present invention, is intended to mean that the sequence of the subject amino acid sequence is identical to the query sequence except that the subject amino acid sequence may include up to three amino acid alterations per each 10 amino acids of the query amino acid sequence. In other words, to obtain an amino acid sequence having a sequence of at least 70% identity to a query amino acid sequence, up to 30% (3 of 10) of the amino acid residues in the subject sequence may be inserted or substituted with another amino acid or deleted, preferably within the above definitions of variants or fragments. The same, of course, also applies similarly to nucleic acid sequences.

For (amino acid or nucleic acid) sequences without exact correspondence, a "% identity" of a first sequence (e.g., the sequence variant) may be determined with respect to a second sequence (e.g., the reference sequence). In general, the two sequences to be compared may be aligned to give a maximum correlation between the sequences. This may include inserting "gaps" in either one or both sequences, to enhance the degree of alignment. A % identity may then be determined over the whole length of each of the sequences being compared (so-called "global alignment"), that is particularly suitable for sequences of the same or similar length, or over shorter, defined lengths (so-called "local alignment"), that is more suitable for sequences of unequal length.

Methods for comparing the identity (sometimes also referred to as "similarity" or "homology") of two or more sequences are well known in the art. The percentage to which two (or more) sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

Preferably, the sequence variant not encompassed by the wording of the claims differs from the reference sequence, in particular a (human) reference epitope, such as the (human) FOXM1 epitope, (only) in primary and/or secondary anchor residues for MHC molecules. More preferably, the sequence variant differs from the reference sequence, in particular a (human) reference epitope, such as the (human) FOXM1 epitope, (only) in that it comprises amino acid substitutions (only) in primary and/or secondary anchor residues for MHC molecules. Anchor residues for the HLA subtypes are known in the art, and can be defined by large throughput analysis of structural data of existing p-HLA complexes in the Protein Data Bank. Moreover, anchor motifs for MHC subtypes can also be found in IEDB (URL: www.iedb.org; browse by allele) or in SYFPEITHI (URL: http://www.syfpeithi.de/). For example, for a 9 amino acid size HLA.A2.01 peptide, the peptide primary anchor residues, providing the main contact points, are located at residue positions P1, P2 and P9.

In a particular instance not encompassed by the wording of the claims, it is preferred that the core sequence of the sequence variant is identical with the core sequence of the reference sequence, in particular a (human) reference epitope, such as the (human) FOXM1 epitope, wherein the "core sequence" consists of all amino acids except the (at least) three most N-terminal and the (at least) three most C-terminal amino acids of the (human) reference epitope, such as the (human) FOXM1 epitope. Accordingly, it is preferred that any alterations in the sequence variant in comparison to the (human) reference epitope, such as the (human) FOXM1 epitope, are preferably located within the three N-terminal and/or within the three C-terminal amino acids of the (human) reference epitope, such as the (human) FOXM1 epitope, but not in the "core sequence" of the (human) reference epitope, such as the (human) FOXM1 epitope (amino acids in the middle of the of the (human) reference epitope sequence, e.g. in the middle of the (human) FOXM1 epitope sequence). This does not mean that all three N-terminal and/or C-terminal amino acids of a (human) reference epitope, such as a (human) FOXM1 epitope, must be altered, but only that those are the preferred amino acid positions, where an amino acid may optionally be altered. For example, in a (human) reference epitope, such as an FOXM1 epitope, of nine amino acids, the three middle amino acids may represent the core sequence and alterations may preferably only occur at any of the three N-terminal and the three C-terminal amino acid positions.

In a particular instance not encompassed by the wording of the claims, the core sequence of the (human) reference epitope, such as the (human) FOXM1 epitope, consists of all amino acids except the two most N-terminal and the two most C-terminal amino acids of the (human) reference epitope, such as the (human) FOXM1 epitope. For example, in a (human) reference epitope, such as a (human) FOXM1 epitope, of nine amino acids, the five middle amino acids may represent the core sequence and alterations may preferably only occur at any of the two N-terminal and the two C-terminal amino acid positions of the (human) reference epitope, such as the (human) FOXM1 epitope.

In a particular instance not encompassed by the wording of the claims the core sequence of the (human) reference epitope, such as the (human) FOXM1 epitope, consists of all amino acids except the most N-terminal and the most C-terminal amino acid of the (human) reference epitope, such as the (human) FOXM1 epitope. For example, in a (human) reference epitope, such as a (human) FOXM1 epitope, of nine amino acids, the seven middle amino acids may represent the core sequence and alterations may preferably only occur at the N-terminal position (P1) and the C-terminal amino acid position (P9) of the (human) reference epitope, such as the (human) FOXM1 epitope.

In a particular instance not encompassed by the wording of the claims, the core sequence of the (human) reference epitope, such as the (human) FOXM1 epitope, consists of all amino acids except the two most N-terminal amino acids and the most C-terminal amino acid of the (human) reference epitope, such as the (human) FOXM1 epitope. For example, in a (human) reference epitope, such as a (human) FOXM1 epitope, of nine amino acids, the six middle amino acids may represent the core sequence and alterations may preferably only occur at any of the two N-terminal positions (P1 and P2) and the C-terminal amino acid position (P9) of the (human) reference epitope, such as the (human) FOXM1 epitope.

In a particular instance not encompassed by the wording of the claims the sequence variant, e.g. having a length of nine amino acids, comprises at position 1 (P1; the most N-terminal amino acid position) a phenylalanine (F) or a lysine (K). In a particular instance not encompassed by the wording of the claims that the sequence variant, e.g. having a length of nine amino acids, comprises at position 2 (P2) a leucine (L) or a methionine (M). In a particular instance not encompassed by the wording of the claims the sequence variant, e.g. having a length of nine amino acids, comprises at position 9 (P9) a valine (V) or a leucine (L). In a particular instance not encompassed by the wording of the claims, the sequence variant, e.g. having a length of nine amino acids, comprises at position 1 (P1; the most N-terminal amino acid position) a phenylalanine (F) or a lysine (K), at position 2 (P2) a leucine (L) or a methionine (M) and/or at position 9 (P9) a valine (V) or a leucine (L).

Moreover, amino acid substitutions, in particular at positions other than the anchor position(s) for MHC molecules (e.g., P1, P2 and P9 for MHC-I subtype HLA.A2.01), are preferably conservative amino acid substitutions. Examples of conservative substitutions include substitution of one aliphatic residue for another, such as Ile, Val, Leu, or Ala for one another; or substitutions of one polar residue for another, such as between Lys and Arg; Glu and Asp; or Gln and Asn. Other such conservative substitutions, for example, substitutions of entire regions having similar hydrophobicity properties, are well known (Kyte and Doolittle, 1982, J. Mol. Biol. 157(1):105- 132). Examples of conservative amino acid substitutions are presented in Table 2 above.

Preferred examples of sequence variants not encompassed by the wording of the claims are sequence variants of a (human) FOXM1 epitope having an amino acid sequences as set forth in any one of SEQ ID NOs 302 - 329.

In a particular instance not encompassed by the wording of the claims as such the (poly)peptide for use as herein disclosed comprises an amino acid sequence as set forth in any one of SEQ ID NOs 302 - 329. The amino acid sequences as set forth in any one of SEQ ID NOs 302 - 329 represent preferred examples of sequence variants of human FOXM1 epitopes.

Preferred examples of sequence variants not encompassed by the wording of the claims as such are listed in Table 5 below, which also provides information regarding the corresponding reference sequence in human FOXM1. The sequence IDs SEQ ID NO: 302 - 329 refer to the sequence variants.

**Table 5: Examples of preferred sequence variants of human FOXM1 epitopes not encompassed by the wording of the claims as such.**

| **Sequence human reference peptide** | **SEQ ID NO. human reference peptide** | **Sequence variant peptide** | **SEQ ID NO. variant peptide** |
|---|---|---|---|
| ILLDISFPG | 291 | TLLDISFAA | 302 |
| ILLDISFPG | 291 | NMLDISFYL | 303 |
| ILLDISFPG | 291 | WLLDISFPL | 304 |
| ILLDISFPG | 291 | HLLDISFPA | 305 |
| ILLDISFPG | 291 | ELLDISFPA | 306 |
| ILLDISFPG | 291 | VLLDISFEL | 307 |
| ILLDISFPG | 291 | VLLDISFKV | 308 |
| ILLDISFPG | 291 | IMLDISFLL | 309 |
| LLDISFPGL | 292 | ILDISFPLV | 310 |
| LLDISFPGL | 292 | LLDISFPSL | 311 |
| LMDLSTTPL | 293 | LMDLSTTEV | 312 |
| LMDLSTTPL | 293 | LMDLSTTNV | 313 |
| RVSSYLVPI | 294 | RLSSYLVEI | 314 |
| RVSSYLVPI | 294 | MVSSYLVEV | 315 |
| RVSSYLVPI | 294 | KVSSYLVEV | 316 |
| RVSSYLVPI | 294 | MLSSYLVPI | 317 |
| RVSSYLVPI | 294 | LLSSYLVPI | 318 |
| RVSSYLVPI | 294 | FVSSYLVPT | 319 |
| SLSKILLDI | 295 | ILSKILLFA | 320 |
| SQLSYSQEV | 296 | YQLSYSQMV | 321 |
| SQLSYSQEV | 296 | KLLSYSQEL | 322 |
| WAAELPFPA | 297 | KIAELPFPL | 323 |
| NLSLHDMFV | 298 | SLSLHDMFL | 324 |
| KMKPLLPRV | 299 | KLKPLLPWI | 325 |
| KMKPLLPRV | 299 | KLKPLLPFL | 326 |
| YLVPIQFPV | 300 | KVVPIQFPV | 327 |
| YLVPIQFPV | 300 | KIVPIQFPI | 328 |
| YMAMIQFAI | 301 | YQAMIQFLI | 329 |

In an instance not encompassed by the wording of the claims, the FOXM1 sequence variant is a sequence variant of the FOXM1 epitope (human reference epitope) "ILLDISFPG" (SEQ ID NO: 291), such as (poly)peptide comprising or consisting of an amino acid sequence as set forth in any one of SEQ ID NOs 302 - 309. In an instance not encompassed by the wording of the claims, the FOXM1 sequence variant is a sequence variant of the FOXM1 epitope (human reference epitope) "LLDISFPGL" (SEQ ID NO: 292), such as (poly)peptide comprising or consisting of an amino acid sequence as set forth in SEQ ID NO: 310 or in SEQ ID NO: 311. In an instance not encompassed by the wording of the claims, the FOXM1 sequence variant is a sequence variant of the FOXM1 epitope (human reference epitope) "LMDLSTTPL" (SEQ ID NO: 293), such as (poly)peptide comprising or consisting of an amino acid sequence as set forth in as set forth in SEQ ID NO: 312 or in SEQ ID NO: 313. In an instance not encompassed by the wording of the claims, the FOXM1 sequence variant is a sequence variant of the FOXM1 epitope (human reference epitope) "RVSSYLVPI" (SEQ ID NO: 294), such as (poly)peptide comprising or consisting of an amino acid sequence as set forth in any one of SEQ ID NOs 314 - 319. In an instance not encompassed by the wording of the claims, the FOXM1 sequence variant is a sequence variant of the FOXM1 epitope (human reference epitope) "SLSKILLDI" (SEQ ID NO: 295), such as (poly)peptide comprising or consisting of an amino acid sequence as set forth in SEQ ID NO: 320. In an instance not encompassed by the wording of the claims, the FOXM1 sequence variant is a sequence variant of the FOXM1 epitope (human reference epitope) "SQLSYSQEV" (SEQ ID NO: 296), such as (poly)peptide comprising or consisting of an amino acid sequence as set forth in SEQ ID NO: 321 or in SEQ ID NO: 322. In an instance not encompassed by the wording of the claims, the FOXM1 sequence variant is a sequence variant of the FOXM1 epitope (human reference epitope) "WAAELPFPA" (SEQ ID NO: 297), such as (poly)peptide comprising or consisting of an amino acid sequence as set forth in SEQ ID NO: 323. In some embodiments, the FOXM1 sequence variant is a sequence variant of the FOXM1 epitope (human reference epitope) "NLSLHDMFV" (SEQ ID NO: 298), such as (poly)peptide comprising or consisting of an amino acid sequence as set forth in SEQ ID NO: 324. In an instance not encompassed by the wording of the claims, the FOXM1 sequence variant is a sequence variant of the FOXM1 epitope (human reference epitope) "KMKPLLPRV" (SEQ ID NO: 299), such as (poly)peptide comprising or consisting of an amino acid sequence as set forth in SEQ ID NO: 325 or in SEQ ID NO: 326. In an instance not encompassed by the wording of the claims, the FOXM1 sequence variant is a sequence variant of the FOXM1 epitope (human reference epitope) "YLVPIQFPV" (SEQ ID NO: 300), such as (poly)peptide comprising or consisting of an amino acid sequence as set forth in SEQ ID NO: 327 or in SEQ ID NO: 328. In an instance not encompassed by the wording of the claims, the FOXM1 sequence variant is a sequence variant of the FOXM1 epitope (human reference epitope) "YMAMIQFAI" (SEQ ID NO: 301), such as (poly)peptide comprising or consisting of an amino acid sequence as set forth in SEQ ID NO: 329.

In an instance not encompassed by the wording of the claims, the FOXM1-related (poly)peptide for use as disclosed herein is a peptide or polypeptide comprising, or consisting of, the amino acid sequence of SEQ ID NO: 312.

In an instance not encompassed by the wording of the claims, the (poly)peptide for use as disclosed herein comprises a sequence variant of the FOXM1 epitope, wherein the sequence variant is a microbiota sequence variant.

The term "microbiota sequence variant" refers to a sequence variant of the (human) reference epitope, such as an FOXM1 epitope, which is found in microbiota (for example, as a part of a microbiota protein, for example which is distinct from FOXM1). In other words, the "microbiota sequence variant" is a microbiota sequence (sequence of microbiota origin), which is a sequence variant of the (human) reference epitope, such as an FOXM1 epitope. That is, the "microbiota sequence variant" is a microbiota sequence (sequence of microbiota origin) which is similar, but contains at least one alteration, in comparison to the (human) reference epitope, such as an FOXM1 epitope. Accordingly, the "microbiota sequence variant" is a sequence naturally occurring in microbiota (and *not* a sequence variant of a microbiota sequence).

Accordingly, the term "microbiota sequence variant" refers to a (poly)peptide sequence found in microbiota, i.e. of microbiota origin (once the sequence was identified in microbiota, it can usually also be obtained by recombinant measures well-known in the art). A "microbiota sequence variant" may refer to a complete (poly)peptide found in microbiota or, preferably, to a fragment of a (complete) microbiota (poly)peptide/protein having a length of at least 5 amino acids, preferably at least 6 amino acids, more preferably at least 7 amino acids, and even more preferably at least 8 amino acids. For example, the "microbiota sequence variant" may be a fragment of a microbiota protein/nucleic acid molecule, the fragment having a length of 9 or 10 amino acids. Preferably, the microbiota sequence variant is a fragment of a microbiota protein as described above. Preferably, the microbiota sequence variant has a length of 8 - 12 amino acids, more preferably the microbiota sequence variant has a length of 8 - 10 amino acids. Peptides having such a length can bind to MHC (major histocompatibility complex) class I (MHC I), which is crucial for a cytotoxic T-lymphocyte (CTL) response. It is also preferred that the microbiota sequence variant has a length of 13 - 24 amino acids. Peptides having such a length can bind to MHC (major histocompatibility complex) class II (MHC II), which is crucial for a CD4+ T-cell (T helper cell) response.

The term "microbiota", as used herein, refers to commensal, symbiotic and pathogenic microorganisms found in and on all multicellular organisms studied to date from plants to animals. In particular, microbiota have been found to be crucial for immunologic, hormonal and metabolic homeostasis of their host. Microbiota include bacteria, archaea, protists, fungi and viruses. Accordingly, the microbiota sequence variant is preferably selected from the group consisting of bacterial sequence variants, archaea sequence variants, protist sequence variants, fungi sequence variants and viral sequence variants. More preferably, the microbiota sequence variant is a bacterial sequence variant or an archaea sequence variant. Most preferably, the microbiota sequence variant is a bacterial sequence variant, i.e. a peptide of bacterial origin (which may exist in bacteria as a partial sequence of a larger bacterial (poly)peptide or protein or in the form of the peptide "as such").

Anatomically, microbiota reside on or within any of a number of tissues and biofluids, including the skin, conjunctiva, mammary glands, vagina, placenta, seminal fluid, uterus, ovarian follicles, lung, saliva, oral cavity (in particular oral mucosa), and the gastrointestinal tract, in particular the gut. In the context of the present invention the microbiota sequence variant is preferably a sequence variant of microbiota of the gastrointestinal tract (microorganisms residing in the gastrointestinal tract), more preferably a sequence variant of microbiota of the gut (microorganisms residing in the gut). Accordingly, it is most preferred that the microbiota sequence variant is a gut bacterial sequence variant (i.e. a sequence variant of bacteria residing in the gut).

While microbiota can be found in and on many multicellular organisms (all multicellular organisms studied to date from plants to animals), microbiota found in and on mammals are preferred. Mammals contemplated by the present invention include for example human, primates, domesticated animals such as cattle, sheep, pigs, horses, laboratory rodents and the like. Microbiota found in and on humans are most preferred. Such microbiota are referred to herein as "mammalian microbiota" or "human microbiota" (wherein the term mammalian/human refers specifically to the localization/residence of the microbiota). Preferably, the reference epitope, such as the FOXM1 epitope, is of the same species, in/on which the microbiota (of the microbiota sequence variant) reside, e.g. human. Preferably, the microbiota sequence variant is a human microbiota sequence variant. Accordingly, it is preferred that the reference epitope, such as the FOXM1 epitope, is a human reference epitope, such as a human FOXM1 epitope.

The inventive idea to use a microbiota sequence variant of a (human) reference epitope, such as an FOXM1 epitope, is based on the surprising finding that the present inventors identified such microbiota sequence variants of (human) reference epitopes, such as FOXM1 epitopes, in the microbiome of the human gut. Without being bound to any theory, this offers a possible way to bypass the repertoire restriction of human T cells due to clonal depletion of T cells recognizing self-antigens. In particular, antigens/epitopes distinct from self-antigens, but sharing sequence similarity with the self-antigen, (i) can still be recognized due to the cross-reactivity of the T-cell receptor (see, for example, Degauque et al., Cross-Reactivity of TCR Repertoire: Current Concepts, Challenges, and Implication for Allotransplantation. Frontiers in Immunology. 2016;7:89. doi:10.3389/fimmu.2016.00089; Nelson et al., T cell receptor cross-reactivity between similar foreign and self peptides influences naive cell population size and autoimmunity. Immunity. 2015 Jan 20;42(1):95-107); and (ii) it is expected that such antigens/epitopes are recognized by T cell/TCR that have not been depleted during T cell education process. Accordingly, microbiota sequence variants of a human self antigen, such as FOXM1, are able to elicit a strong immune response leading to clonal expansion of T cell harboring potential cross reactivity with self-antigens.

The human microbiome, which is composed of thousands of different bacterial species, is a large source of genetic diversity and potential antigenic components. The gut can be considered as the largest area of contact and exchange with microbiota. As a consequence, the gut is the largest immune organ in the body. Specialization and extrathymic T cell maturation in the human gut epithelium is known now for more than a decade. The gut contains a large panel of immune cells that could recognize our microbiota and which are tightly controlled by regulatory mechanisms.

As disclosed herein,, the large repertoire of bacterial species existing in the gut provides an incredible source of antigens (microbiota sequence variants) with potential similarities with human antigens, such as FOXM1. These microbiota sequence variants not encompassed by the wording of the claims are presented to specialized cells in a complex context, with large amount of co-signals delivered to immune cells as TLR activators. As a result, microbiota sequence variants not encompassed by the wording of the claims may elicit full functional response and drive maturation of large T memory subset or some time lead to full clonal depletion or exhaustion. Identification of bacterial components sharing similarities with human antigens, such as FOXM1, provides a new source for selection of epitopes of antigens, which (i) overcome the problem of T cell depletion and (ii) have already "primed" the immune system in the gut, thereby providing for stronger immune responses as compared to sequence variants of antigens/epitopes of other sources and artificially mutated antigens/epitopes.

In an instance not encompassed by the wording of the claims microbiota sequence variants include the amino acid sequences according to any one of SEQ ID NOs: 302 - 329. In an instance not encompassed by the wording of the claims, it is preferred that the (poly)peptide for use as disclosed herein comprises or consists of an amino acid sequence according to any one of SEQ ID NOs: 302 - 329.

In an instance not encompassed by the wording of the claims , the (poly)peptide for use as disclosed herein is a peptide or polypeptide comprising, or consisting of, the amino acid sequence of SEQ ID NO: 312.

In general, the (poly)peptide for use as disclosed herein may be of any length. In an instance not encompassed by the wording of the claims, the length of the (poly)peptide comprising the epitope of FOXM1 or the sequence variant thereof for use as disclosed herein does not exceed 750 amino acids. For example, the maximum length of the (poly)peptide for use according to the present invention may be 500 or 250 amino acids. In an instance not encompassed by the wording of the claims, the maximum length of the FOXM1 (poly)peptide for use as disclosed herein does not exceed 200 amino acids, e.g., not more than 190, 180, 170, 160, 150, 140, 130, 120, 110, 100, 95, 90, 85, 80, 75, 70, 65, 60, 55, 50, 45, 40, 35, 30, 29, 28, 27, 26, 25, 24, 23, 22, 21, 20, 19, 18, 17, 16, 15, 14 or 13 amino acids. In an instance not encompassed by the wording of the claims, the (poly)peptide consists of the epitope of FOXM1 or the sequence variant thereof. **In** an instance not encompassed by the wording of the claims the (poly)peptide may have a length of 8 - 12 amino acids, more preferably 8 - 10 amino acids and even more preferably 9 or 10 amino acids.

### Immunogenic compounds, nanoparticles, cells, cytotoxic T cells, compositions and kits comprising and nucleic acids encoding the (poly)peptide comprising an IL13RA2, BIRCS or FOXM1 epitope or a sequence variant thereof

Advantageously, the (poly)peptide for use as disclosed herein may be in the form of an immunogenic compound. Accordingly, the present disclosure also provides an immunogenic compound comprising the (poly)peptide as defined above for use in prevention and/or treatment of an adrenal cancer.

Preferably, the (poly)peptide as described above is linked to a carrier molecule, in particular a carrier protein, thereby forming an immunogenic compound for use according the present invention. Accordingly, the (poly)peptide as above defined is preferably linked to a carrier molecule, in particular a carrier protein, for example by a covalent or non-covalent bond.

Preferably, the immunogenic compound for use as disclosed herein comprises or consists of an (poly)peptide of formula (I):

PepNt- CORE-PepCt (I),

wherein:
- "PepNt" consists of a polypeptide having an amino acid length varying from 0 to 500 amino acid residues and located at the N-terminal end of the polypeptide of formula (I);
- CORE consists of a (poly)peptide as defined above; and
- "PepCt" consists of a polypeptide having an amino acid length varying from 0 to 500 amino acid residues and located at the C-terminal end of the polypeptide of formula (I).

Preferably, the (poly)peptide of formula (I) is a fusion peptide or fusion protein, in particular a recombinant fusion peptide or protein. The term "recombinant" means that it does not occur in nature.

In an instance not encompassed by the wording of the claims , the immunogenic compound comprises, or consists of, an (poly)peptide of formula (I) :

PepNt- CORE-PepCt (I),

wherein:
- "PepNt" consists of a polypeptide having an amino acid length varying from 0 to 500 amino acid residues and located at the N-terminal end of the polypeptide of formula (I);
- CORE consists of a (poly)peptide comprising, or consisting of, an amino acid sequence selected from the group consisting of SEQ ID NO: 1 to 242, 267 - 274, 279 and 335 - 344 (preferably SEQ ID NO: 31, 64, 178, 212, 267 and 279, more preferably SEQ ID NO: 31, SEQ ID NO: 192 or SEQ ID NO: 279, most preferably SEQ ID NO: 31 or SEQ ID NO: 279) and
- "PepCt" consists of a polypeptide having an amino acid length varying from 0 to 500 amino acid residues and located at the C-terminal end of the polypeptide of formula (I).

In an instance not encompassed by the wording of the claims, the immunogenic compound comprises, or consists of, an (poly)peptide of formula (I) :

PepNt- CORE-PepCt (I),

wherein:
- "PepNt" consists of a polypeptide having an amino acid length varying from 0 to 500 amino acid residues and located at the N-terminal end of the polypeptide of formula (I);
- CORE consists of a (poly)peptide comprising, or consisting of, an amino acid sequence selected from the group consisting of SEQ ID NO: 287 - 289 (preferably SEQ ID NO: 289) and "PepCt" consists of a polypeptide having an amino acid length varying from 0 to 500 amino acid residues and located at the C-terminal end of the polypeptide of formula (I).

In an instance not encompassed by the wording of the claims, the immunogenic compound comprises, or consists of, an (poly)peptide of formula (I) :

PepNt- CORE-PepCt (I),

wherein:
- "PepNt" consists of a polypeptide having an amino acid length varying from 0 to 500 amino acid residues and located at the N-terminal end of the polypeptide of formula (I);
- CORE consists of a (poly)peptide comprising, or consisting of, an amino acid sequence selected from the group consisting of SEQ ID NO: 302 - 329 (preferably SEQ ID NO: 312) and "PepCt" consists of a polypeptide having an amino acid length varying from 0 to 500 amino acid residues and located at the C-terminal end of the polypeptide of formula (I).

In an instance not encompassed by the wording of the claims, the immunogenic compound comprises or consists of an (poly)peptide of formula (la) or (Ib) :
- PepNt- CORE (la); or
- CORE-PepCt (Ib),
wherein "PepNt" and "PepCt" and CORE are as defined above.

In an instance not encompassed by the wording of the claims, the (poly)peptide or immunogenic compound as defined above comprises from 9 to 1000 amino acids; which includes 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 250, 300, 350, 400, 450, 500, 600, 700, 800, 900 and 1000 amino acids. According to said embodiment, the length of "PepNt" and "PepCt", if applicable, are defined accordingly. Thus, "PepNt" and "PepCt", as defined above, may comprise from 0 to 500 amino acid residues; which includes 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 250, 300, 350, 400, 450, and 500 amino acid residues.

The types of carrier molecules used for generating an immunogenic compound as disclosed herein, such as the ones comprising or consisting of a peptide of formula (I) linked to a carrier molecule, are well in the general knowledge of the one skilled in the art. The function of the carrier molecule may be to provide cytokine help (or T-cell help) in order to enhance the immune response against IL13RA2, BIRC5 and/or FOXM1.

Preferably, the (poly)peptide is linked to a carrier molecule, in particular to a carrier protein, preferably by covalent or non-covalent bond. The carrier molecule to which the (poly)peptide is optionally bound can be selected from a wide variety of known carriers. Examples of carrier molecules for vaccine purposes encompass proteins such as human or bovine serum albumin and keyhole limpet haemocyanin (KLH) and fatty acids. Other embodiments of carrier molecules to which an (poly)peptide (e.g. of formula (I)) may be covalently linked include bacterial toxins or toxoids, such as diphtheria, cholera, E. coli heat labile or tetanus toxoids, the N. meningitidis outer membrane protein (European patent application n° EP0372501), synthetic peptides (European patent applications n° EP0378881 and n° EP0427347), heat shock proteins (PCT application n° WO93/17712), Pertussis proteins (PCT application n° WO98/58668), protein D from H. influenzae (PCT application n° WO00/56360.) and toxin A or B from C. difficile (International patent application WO00/61761).

More preferably, the carrier protein or carrier peptide is a protein/peptide having immuno-adjuvant properties, such as providing stimulation of CD4+ Th1 cells as described herein. A preferred example thereof is a (non-IL13RA2, non-FOXM1, and/or non-BIRC5) antigen that recalls immune memory or provides a non-specific help or could be a specific helper peptide, such as tetanus helper peptide, keyhole limpet hemocyanin peptide or PADRE peptide. In a preferred embodiment, the carrier protein or carrier peptide is a protein/peptide having immuno-adjuvant properties may be a HHD-DR3 carrier peptide MAKTIAYDEEARRGLERGLN (SEQ ID NO: 266). In particular, "PepNt" and/or "PepCt" may correspond to a carrier protein or carrier peptide, such as the HHD-DR3 carrier peptide MAKTIAYDEEARRGLERGLN (SEQ ID NO: 266). For example, the immunogenic compound comprises or consists of the carrier peptide of sequence SEQ ID NO: 266 linked covalently to the N-terminus of the (poly)peptide as described herein, e.g. as set forth in SEQ ID NO: 31, SEQ ID NO: 279 or SEQ ID NO: 192. Another preferred example is h-pAg T13L (sequence: TPPAYRPPNAPIL; SEQ ID NO: 280; Bhasin M, Singh H, Raghava GP (2003) MHCBN: a comprehensive database of MHC binding and non-binding peptides. Bioinformatics 19: 665-666). Further examples of preferred carrier proteins/peptides, in particular of helper peptides, include the UCP2 peptide (for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2) and the BIRC5 peptide (for example as described in EP2119726 A1 or in Widenmeyer M, Griesemann H, Stevanović S, Feyerabend S, Klein R, Attig S, Hennenlotter J, Wernet D, Kuprash DV, Sazykin AY, Pascolo S, Stenzl A, Gouttefangeas C, Rammensee HG: Promiscuous survivin peptide induces robust CD4+ T-cell responses in the majority of vaccinated cancer patients. Int J Cancer. 2012 Jul 1;131(1):140-9. doi: 10.1002/ijc.26365. Epub 2011 Sep 14). The most preferred helper peptide is the UCP2 peptide (amino acid sequence: KSVWSKLQSIGIRQH; SEQ ID NO: 281, for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2).

Accordingly, "PepNt" and/or "PepCt" may preferably correspond to such a protein/peptide having immuno-adjuvant properties, such as providing stimulation of CD4+ Th1 cells as described herein.

In an instance not encompassed by the wording of the claims, the immunogenic compound preferably comprises or consists of such a protein/peptide having immuno-adjuvant properties, such as providing stimulation of CD4+ Th1 cells as described herein, linked covalently to the N-terminus of the (poly)peptide as defined herein, e.g. as set forth in SEQ ID NO: 279, SEQ ID NO: 289 or SEQ ID NO: 312.

In an instance not encompassed by the wording of the claims, the said (poly)peptide is covalently bound to the carrier molecule through a linker moiety.

The said restricted family of linker agents encompasses, or even consists of, the linker agents named GMBS, sulfo-GMBS, SMPB and sulfo-SMPB.

In an instance not encompassed by the wording of the claims of an immunogenic compound as defined above, the said linker agent is selected form the group consisting of GMBS (N-[γ-maleimidobutyryl-oxy]succinimide ester), Sulfo-GMBS (N-[γ-maleimidobutyryl-oxy]sulfosuccinimide ester), SMPB (succinimidyl 4-[*p*-maleimidophenyl]butyrate) and Sulfo-SMPB (sulfosuccinimidyl 4-[p-maleimidophenyl]butyrate).

Methods for conjugating two proteins with a linker agent in general, and more particularly with a linker agent selected from the group consisting of GMBS, Sulfo-GMBS, SMPB and Sulfo-SMPB, are well known by the one skilled in the art. Illustratively, such protocols are disclosed in the leaflets that are made publicly available by the Pierce Company (Illinois, USA). GMBS, Sulfo-GMBS, SMPB and Sulfo-SMPB consist of heterobifunctional linker agents that contain both a *N-*hydroxysuccinimide (NHS) ester group and a maleimide group. Conjugation using GMBS, Sulfo-GMBS, SMPB or Sulfo-SMPB is usually performed by a two-step procedure. In a first step, the amine-containing protein is reacted with a several-fold molar excess of the linker agent at pH 7-9 to form amide bonds, followed by removal of excess non-reacted linker agent, usually by desalting or dialysis. In a second step, the sulfhydryl-containing molecule (e.g. peptide of formula (I)) is added to react with the maleimide groups already attached to the first protein at pH 6.5-7.5 to form stable thioether bonds.

Using SMPB or Sulfo-SMPB as linker agents for covalently linking peptides of formula (I) to the amine-containing carrier protein, leads to a conjugate of formula (II) below: 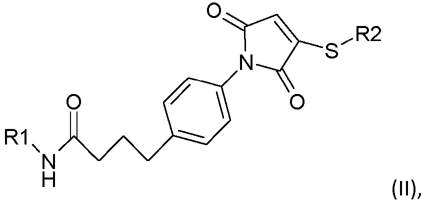 wherein:
- R1 consists of one reactive group of the amine-containing carrier protein, and wherein the NH group attached thereto derives from (i) the alpha amino group located at the N-terminal end of the amine-containing carrier protein or (ii) a lateral chain amino group from a Lysine (K) amino acid residue of the amine-containing carrier protein; and
- R2 consists of a (poly)peptide of formula (I), and wherein the sulphur (S) atom attached thereto derives from a sulfhydryl (SH) group of a cysteine residue located at the N-terminal end or at the C-terminal end of a (poly)peptide of formula (I). In some embodiments, the sulfhydryl moiety could be part of an unnatural amino acid, or any other molecule present at the end of the (poly)peptide of formula (I).

Using GMBS or Sulfo-GMBS as linker agents for covalently linking peptides of formula (I) to the amine-containing carrier protein, in particular the CRM197 carrier, protein leads to a conjugate of formula (III) below: 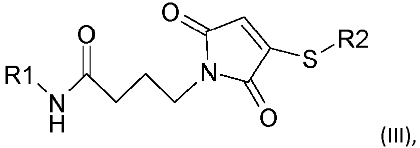 wherein:
- R1 consists of one reactive group of the amine-containing carrier protein, and wherein the NH group attached thereto derives from (i) the alpha amino group located at the N-terminal end of the amine-containing carrier proteinor (ii) a lateral chain amino group from a Lysine (K) amino acid residue of the amine-containing carrier protein; and
- R2 consists of a (poly)peptide of formula (I), and wherein the sulphur (S) atom attached thereto derives from a sulfhydryl (SH) group of a cysteine residue located at the N-terminal end or at the C-terminal end of a (poly)peptide of formula (I). In some embodiments, the sulfhydryl moiety could be part of an unnatural amino acid, or any other molecule present at the end of the (poly)peptide of formula (I).

In a further aspect, the presentdisclosure also provides a nanoparticle loaded with
- at least one (poly)peptide as defined above, or
- at least one immunogenic compound as defined above;

and, optionally, with an adjuvant
for use in prevention and/or treatment of an adrenal cancer.

Nanoparticles, in particular for use as vaccines, are known in the art and described, for example, in Shao et al., Nanoparticle-based immunotherapy for cancer, ACS Nano 2015, 9(1):16-30; Zhao et al., Nanoparticle vaccines, Vaccine 2014, 32(3):327-37; and Gregory et al., Vaccine delivery using nanoparticles, Front Cell Infect Microbiol. 2013, 3:13, doi: 10.3389/fcimb.2013.00013. eCollection 2013, Review. In particular, the nanoparticle is used for delivery of the (poly)peptide as described above (or the immunogenic compound comprising the (poly)peptide) and may optionally also act as an adjuvant. The (poly)peptide (or the immunogenic compound comprising the (poly)peptide) is typically either encapsulated within the nanoparticle or linked/bound to (decorated onto) the surface of the nanoparticle ("coating"). Compared to conventional approaches, nanoparticles can protect the payload (antigen/adjuvant) from the surrounding biological milieu, increase the half-life, minimize the systemic toxicity, promote the delivery to APCs, or even directly trigger the activation of antigen-specific T-cells. Preferably, the nanoparticle has a size (diameter) of no more than 300 nm, more preferably of no more than 200 nm and most preferably of no more than 100 nm. Such nanoparticles are adequately sheltered from phagocyte uptake, with high structural integrity in the circulation and long circulation times, capable of accumulating at target sites, and able to penetrate deep into target sites.

Preferred examples of nanoparticles include polymeric nanoparticles such as poly(ethylene glycol) (PEG) and poly (D,L-lactic-coglycolic acid) (PLGA); inorganic nanoparticles such as gold nanoparticles, iron oxide beads, iron-oxide zinc-oxide nanoparticles, carbon nanotubes and mesoporous silica nanoparticles; liposomes, such as cationic liposomes; immunostimulating complexes (ISCOM); virus-like particles (VLP); and self-assembled proteins.

Polymeric nanoparticles are nanoparticles based on/comprising polymers, such as poly(d,l-lactide-co-glycolide) (PLG), poly(d,I-lactic-coglycolic acid)(PLGA), poly(g-glutamic acid) (g-PGA), poly(ethylene glycol) (PEG), and polystyrene. Polymeric nanoparticles may entrap an antigen (e.g., the (poly)peptide or a (poly)peptide comprising the same) or bind to/conjugate to an antigen (e.g., the (poly)peptide or a (poly)peptide comprising the same). Polymeric nanoparticles may be used for delivery, e.g. to certain cells, or sustain antigen release by virtue of their slow biodegradation rate. For example, g-PGA nanoparticles may be used to encapsulate hydrophobic antigens. Polystyrene nanoparticles can conjugate to a variety of antigens as they can be surface-modified with various functional groups. Polymers, such as Poly(L-lactic acid) (PLA), PLGA, PEG, and natural polymers such as polysaccharides may also be used to synthesize hydrogel nanoparticles, which are a type of nano-sized hydrophilic three-dimensional polymer network. Nanogels have favorable properties including flexible mesh size, large surface area for multivalent conjugation, high water content, and high loading capacity for antigens. Accordingly, a preferred nanoparticle is a nanogel, such as a chitosan nanogel. Preferred polymeric nanoparticles are nanoparticles based on/comprising poly(ethylene glycol) (PEG) and poly (D,L-lactic-coglycolic acid) (PLGA).

Inorganic nanoparticles are nanoparticles based on/comprising inorganic substances, and examples of such nanoparticles include gold nanoparticles, iron oxide beads, iron-oxide zinc-oxide nanoparticles, carbon nanoparticles (e.g., carbon nanotubes) and mesoporous silica nanoparticles. Inorganic nanoparticles provide a rigid structure and controllable synthesis. For example, gold nanoparticles can be easily produced in different shapes, such as spheres, rods, cubes. Inorganic nanoparticles may be surface-modified, e.g. with carbohydrates. Carbon nanoparticles provide good biocompatibility and may be produced, for example, as nanotubes or (mesoporous) spheres. For example, multiple copies of the (poly)peptide according to the present invention (or a (poly)peptide comprising the same) may be conjugated onto carbon nanoparticles, e.g. carbon nanotubes. Mesoporous carbon nanoparticles are preferred for oral administration. Silica-based nanoparticles (SiNPs) are also preferred. SiNPs are biocompatible and show excellent properties in selective targeting and vaccine delivery. The abundant silanol groups on the surface of SiNPs may be used for further modification to introduce additional functionality, such as cell recognition, absorption of specific biomolecules, improvement of interaction with cells, and enhancement of cellular uptake. Mesoporous silica nanoparticles are particularly preferred.

Liposomes are typically formed by phospholipids, such as 1,2-dioleoyl-3-trimethylammonium propane (DOTAP). In general, cationic liposomes are preferred. Liposomes are self-assembling with a phospholipid bilayer shell and an aqueous core. Liposomes can be generated as unilameller vesicles (having a single phospholipid bilayer) or as multilameller vesicles (having several concentric phospholipid shells separated by layers of water). Accordingly, antigens can be encapsulated in the core or between different layers/shells. Preferred liposome systems are those approved for human use, such as Inflexal^{®} V and Epaxal^{®}.

Immunostimulating complexes (ISCOM) are cage like particles of about 40 nm (diameter), which are colloidal saponin containing micelles, for example made of the saponin adjuvant Quil A, cholesterol, phospholipids, and the (poly)peptide antigen (such as the (poly)peptide or a polypeptide comprising the same). These spherical particles can trap the antigen by apolar interactions. Two types of ISCOMs have been described, both of which consist of cholesterol, phospholipid (typically either phosphatidylethanolamine or phos- phatidylcholine) and saponin (such as QuilA).

Virus-like particles (VLP) are self-assembling nanoparticles formed by self-assembly of biocompatible capsid proteins. Due to the naturally-optimized nanoparticle size and repetitive structural order VLPs can induce potent immune responses. VLPs can be derived from a variety of viruses with sizes ranging from 20 nm to 800 nm, typically in the range of 20 - 150 nm. VLPs can be engineered to express additional peptides or proteins either by fusing these peptides/proteins to the particle or by expressing multiple antigens. Moreover, antigens can be chemically coupled onto the viral surface to produce bioconjugate VLPs.

Examples of self-assembled proteins include ferritin and major vault protein (MVP). Ferritin is a protein that can self-assemble into nearly-spherical 10 nm structure. Ninety-six units of MVP can self-assemble into a barrel-shaped vault nanoparticle, with a size of approximately 40 nm wide and 70 nm long. Antigens that are genetically fused with a minimal interaction domain can be packaged inside vault nanoparticles by self-assembling process when mixed with MVPs. Accordingly, the antigen (such as the (poly)peptide according to the present invention of a polypeptide comprising the same) may be fused to a self-assembling protein or to a fragment/domain thereof, such as the minimal interaction domain of MVP. Accordingly, the present invention also provides a fusion protein comprising a self-assembling protein (or a fragment/domain thereof) and the (poly)peptide according to the present invention.

In general, preferred examples of nanoparticles (NPs) include iron oxide beads, polystyrene microspheres, poly(y-glutamic acid) (y-PGA) NPs, iron oxide-zinc oxide NPs, cationized gelatin NPs, pluronic-stabilized poly(propylene sulfide) (PPS) NPs, PLGA NPs, (cationic) liposomes, (pH-responsive) polymeric micelles, PLGA, cancer cell membrane coated PLGA, lipid-calcium-phosphate (LCP) NPs, liposome-protamine-hyaluronic acid (LPH) NPs, polystyrene latex beads, magnetic beads, iron-dextran particles and quantum dot nanocrystals.

Preferably, the nanoparticle further comprises an adjuvant, for example a toll-like receptor (TLR) agonist. Thereby, the (poly)peptide (or the immunogenic compound comprising the (poly)peptide) can be delivered together with an adjuvant, for example to antigen-presenting cells (APCs), such as dendritic cells (DCs). The adjuvant may be encapsulated by the nanoparticle or bound to/conjugated to the surface of the nanoparticle, preferably similarly to the (poly)peptide.

Particularly preferred adjuvants are polyinosinic:polycytidylic acid (also referred to as "poly I:C") and/or its derivative poly-ICLC. Poly I:C is a mismatched double-stranded RNA with one strand being a polymer of inosinic acid, the other a polymer of cytidylic acid. Poly I:C is an immunostimulant known to interact with toll-like receptor 3 (TLR3). Poly I:C is structurally similar to double-stranded RNA, which is the "natural" stimulant of TLR3. Accordingly, poly I:C may be considered a synthetic analog of double-stranded RNA. Poly-ICLC is a synthetic complex of carboxymethylcellulose, polyinosinic-polycytidylic acid, and poly-L-lysine double-stranded RNA. Similar to poly I:C, also poly-ICLC is a ligand for TLR3. Poly I:C and poly-ICLC typically stimulate the release of cytotoxic cytokines. A preferred example of poly-ICLC is Hiltonol^{®}.

In an instance not encompassed by the wording of the claim as suchs, the presentdisclosure also provides a cell loaded with the (poly)peptide as described above or the immunogenic compound as described above for use in prevention and/or treatment of an adrenal cancer.

A preferred cell is an antigen presenting cell (APC), more preferably a dendritic cell (DC).

Antigen-presenting cells (APCs) are of particular interest, as their main function is to process antigens and present it on the cell surface to the T cells of the immune system, so as to initiate and modulate T-cell responses *in vivo.* In the context of the present invention, it is preferred that the APCs are loaded with the (poly)peptide(s) and/or immunogenic compound(s) as disclosed herein, which can be done by exposing APCs in vitro with said (poly)peptide(s) and/or immunogenic compound(s) (Rizzo MM, Alaniz L, Mazzolini G.Ex vivo loading of autologous dendritic cells with tumor antigens. Methods Mol Biol. 2014;1139:41-4; Rolinski J, Hus I. Breaking immunotolerance of tumors: a new perspective for dendritic cell therapy. J Immunotoxicol. 2014 Oct;11(4):311-8).

Preferred antigen-presenting cells as disclosed herein are dendritic cells (DCs). It can indeed be advantageous to combine at least one (poly)peptide or immunogenic compound according to the invention with dendritic cells, as those are the most potent antigen-presenting cells and have been reported to be frequently functionally defective in cancer patients. Dendritic cells can be easily obtained by the skilled person in the art from either healthy compatible donors (i.e. the dendritic cells are HLA-related) or from the patient himself provided that they are functional (i.e. the dendritic cells are autologous), for example by direct isolation from the peripheral blood, or by derivation from peripheral blood cells such as CD14+ monocytes or CD34+ hematopoietic precursors (Figdor CG, de Vries IJ, Lesterhuis WJ, Melief CJ. Dendritic cell immunotherapy: mapping the way. Nat Med. 2004 May;10(5):475-80). Dendritic cells can indeed be distinguished from other cells of peripheral blood by their surface markers, such as S100, p55, CD83, and/or OX62, and may thus be isolated and purified based on said markers using cell cultures techniques well-known in the art.

In an instance not encompassed by the wording of the claims, the present disclosure also provides a cytotoxic T lymphocyte (CTL) specific for the (poly)peptide as described above, in particular an activated cytotoxic T lymphocyte (CTL) specific for the (poly)peptide as described above, for use in prevention and/or treatment of an adrenal cancer..

The present disclosure further provides a method for producing cytotoxic T lymphocytes (CTL) specific for the (poly)peptide as described above, in particular activated cytotoxic T lymphocytes (CTL) specific for the (poly)peptide as described above, the method comprising contacting *in vitro* a CTL with an antigen-loaded human class I or II MHC molecule expressed on the surface of an antigen-presenting cell or an artificial construct mimicking an antigen-presenting cell, wherein said antigen is the (poly)peptide as described above. Preferred antigen-presenting cells include dendritic cells. An artificial construct mimicking an antigen-presenting cell may be, for instance, a peptide-MHC multimer as disclosed herein. The step of contacting the CTL with the antigen-loaded human class I or II MHC molecule expressed on the surface of the antigen-presenting cell or the artificial construct mimicking an antigen-presenting cell may be carried out for a period of time sufficient to activate said CTL in an antigen specific manner. Preferably, the (poly)peptide as disclosed herein as described above is a preferred (poly)peptide as described above, such as the (poly)peptide comprising or consisting of an amino acid sequence as set forth in any one of SEQ ID NOs 279, 289 and 312.

The (activated) T cells that are directed against the (poly)peptides of the invention are useful in therapy. In particular, activated T cells, which are produced by the above method, selectively recognize a cell that aberrantly expresses a tumor antigen as described above.

Preferably, the (activated) cytotoxic T lymphocytes (CTL) as disclosed herein, which are specific for the (poly)peptide as disclosed herein, may have (exhibit/express) memory markers. Such memory markers are preferably memory markers of gut memory cells, such as CCR9, CXCR3, CD103,CX3CR1 and α4β7+.

The (activated) cytotoxic T lymphocytes (CTL) as disclosed herein, which are specific for the (poly)peptide as disclosed herein, are preferably more/stronger amplified after vaccination with the (poly)peptide as disclosed herein (derived from human microbiota sequences) as compared to vaccination with peptides not derived from microbiota sequences, such as the human (reference) sequence and/or a synthetic peptide (e.g., including mutations, which were, e.g., artificially introduced). In other words, vaccination of subjects with the the (poly)peptide as disclosed hereinpreferably increases the number of (activated) cytotoxic T lymphocytes (CTL) as disclosed herein, which are specific for said (poly)peptide as disclosed herein, more than vaccination with respective human peptides or synthetic peptides (not derived from microbiota), which relate to the same reference epitope.

The (activated) cytotoxic T lymphocytes (CTL) as disclosed herein, which are specific for the (poly)peptide as disclosed herein, are preferably more/stronger and/or faster amplified after vaccination in subjects having said peptide in the gut (expressed by the subject's microbiota), e.g., the peptide can be found in a stool sample of the subject, as compared to subjects not having said peptide in the gut (not expressed by the subject's microbiota), e.g. subjects where said peptide is not detectable in stool samples. In particular, subjects having said peptide in the gut (expressed by the subject's microbiota), may respond faster (faster T cell expansion) and/or have T cells from the desired type Tc1.

In an instance not encompassed by the wording of the claims, the present disclosure also provides a nucleic acid for use in prevention and/or treatment of an adrenal cancer, the nucleic acid encoding an (poly)peptide for use as disclosed herein or an immunogenic compound for use as disclosed herein, wherein the immunogenic compound may be a (poly)peptide of formula (I) as described above.

Nucleic acids preferably comprise single stranded, double stranded or partially double stranded nucleic acids, preferably selected from genomic DNA, cDNA, RNA, siRNA, antisense DNA, antisense RNA, ribozyme, complimentary RNA/DNA sequences with or without expression elements, a mini-gene, gene fragments, regulatory elements, promoters, and combinations thereof. Further preferred examples of nucleic acid (molecules) and/or polynucleotides include, e.g., a recombinant polynucleotide, a vector, an oligonucleotide, an RNA molecule such as an rRNA, an mRNA or a tRNA, or a DNA molecule as described above. It is thus preferred that the nucleic acid (molecule) is a DNA molecule or an RNA molecule; preferably selected from genomic DNA; cDNA; rRNA; mRNA; antisense DNA; antisense RNA; complimentary RNA and/or DNA sequences; RNA and/or DNA sequences with or without expression elements, regulatory elements, and/or promoters; a vector; and combinations thereof.

In an instance not encompassed by the wording of the claims as such , the nucleic acid comprises a polynucleotide encoding a peptide as set forth in SEQID NO: 279. In an instance not encompassed by the wording of the claims as such , the nucleic acid comprises a polynucleotide encoding a peptide as set forth in SEQ ID NO: 289. In an instance not encompassed by the wording of the claims, the nucleic acid comprises a polynucleotide encoding a peptide as set forth in SEQ ID NO: 312.

The nucleic acid molecule may be a vector. The term "vector", as used in the context of the present invention, refers to a nucleic acid molecule, preferably to an artificial nucleic acid molecule, i.e. a nucleic acid molecule which does not occur in nature. A vector in the context of the present invention is suitable for incorporating or harboring a desired nucleic acid sequence. Such vectors may be storage vectors, expression vectors, cloning vectors, transfer vectors etc. A storage vector is a vector which allows the convenient storage of a nucleic acid molecule. Thus, the vector may comprise a sequence corresponding, e.g., to a desired (poly)peptide according to the present invention. An expression vector may be used for production of expression products such as RNA, e.g. mRNA, or peptides, polypeptides or proteins. For example, an expression vector may comprise sequences needed for transcription of a sequence stretch of the vector, such as a promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. For example, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector. Preferably, a vector in the context of the present application is an expression vector. A preferred vector is a vector for expression in bacterial cells. More preferably, the vector is useful for expression in so-called "live bacterial vaccine vectors", wherein live bacterial cells (such as bacteria or bacterial spores, e.g., endospores, exospores or microbial cysts) can serve as vaccines. Preferred examples thereof are described in da Silva et al., Live bacterial vaccine vectors: an overview; Braz J Microbiol. 2015 Mar 4;45(4):1117-29.

Nucleic acids encoding (poly)peptides as disclosed herein may be in the form of naked nucleic acids, or nucleic acids cloned into plasmids or viral vectors (Tregoning and Kinnear, Using Plasmids as DNA Vaccines for Infectious Diseases. Microbiol Spectr. 2014 Dec;2(6). doi: 10.1128/microbiolspec.PLAS-0028-2014), the latter being particularly preferred. Examples of suitable viral vectors as disclosed herein include, without limitation, retrovirus, adenovirus, adeno-associated virus (AAV), herpes virus and poxvirus vectors. It is within the skill of the person in the art to clone a nucleic acid into a plasmid or viral vector, using standard recombinant techniques in the art.

Preferably, the nucleic acid is a DNA molecule or an RNA molecule; preferably selected from genomic DNA; cDNA; siRNA; rRNA; mRNA; antisense DNA; antisense RNA; ribozyme; complimentary RNA and/or DNA sequences; RNA and/or DNA sequences with or without expression elements, regulatory elements, and/or promoters; a vector; and combinations thereof.

In an instance not encompassed by the wording of the claims, the present disclosure also provides a host cell for use in prevention and/or treatment of an adrenal cancer, the host cell comprising the nucleic acid for use as disclosed herein, wherein the nucleic acid is preferably a vector. Preferably, the host cell is a bacterial cell. Such a host cell may be preferably used for production of the (poly)peptide as disclosed herein or the immunogenic compound as disclosed herein. Moreover, such a host cell may also be an active component in a vaccine.

Preferably, the host cell is a bacterial cell, more preferably a gut bacterial cell. Such a bacterial host cell may serve as "live bacterial vaccine vector", wherein live bacterial cells (such as bacteria or bacterial spores, e.g., endospores, exospores or microbial cysts) can serve as vaccines. Preferred examples thereof are described in da Silva et al., Live bacterial vaccine vectors: an overview; Braz J Microbiol. 2015 Mar 4;45(4):1117-29.

Bacterial cells (such as bacteria or bacterial spores, e.g., endospores, exospores or microbial cysts), in particular (entire) gut bacterial species, can be advantageous, as they have the potential to trigger a greater immune response than the (poly)peptides or nucleic acids they contain.

Alternatively, bacterial cells, in particular gut bacteria, according to the invention may be in the form of probiotics, i.e. of live gut bacterium, which can thus be used as food additive due to the health benefits it can provide. Those can be for example lyophilized in granules, pills or capsules, or directly mixed with dairy products for consumption.

In an instance not encompassed by the wording of the claims, the present disclosure provides a pharmaceutical composition comprising
- the (poly)peptide as described above;
- the immunogenic compound as described above;
- the nanoparticle as described above;
- the cell as defined as described above;
- the nucleic acid as described above;
- the cytotoxic T cell (CTL) as described above; or
- the host cell as described above
for use in prevention and/or treatment of an adrenal cancer.

Preferably, the pharmaceutical composition further comprises and, optionally, one or more pharmaceutically acceptable excipients or carriers. Preferably, the pharmaceutical composition is an immunogenic composition.

The pharmaceutical composition for use as disclosed herein may be in any form suitable for the purposes of the intended use. For example, said composition may be in a form suitable for parenteral, enteral or topical administration, such as a liquid suspension, a solid dosage form (granules, pills, capsules or tablets), or a paste or gel. It is within the skill of the person in the art to select the appropriate form of the composition for the intended purpose.

Indeed, it can be particularly advantageous to use (poly)peptides, or nucleic acids encoding the same, because of their ease of manufacturing at a low cost and relative safety with no potential for reassortment, infection or recombination.

(Poly)peptides for use as disclosed herein may be administered in the form of immunogenic compounds for use as disclosed herein, cells loaded therewith for use as disclosed herein , nanoparticles for use as disclosed herein, nucleic acids for use as disclosed herein, host cells for use as disclosed herein and/or pharmaceutical compositions for use as described herein.

According to one embodiment, they may be administered in the form of a micro-organism such as a gut bacterial species. Entire gut bacterial species can also be advantageous as they have the potential to trigger a greater immune response than the (poly)peptides or nucleic acids they contain. Alternatively, gut bacteria according to the invention may be in the form of probiotics, i.e. of live gut bacterium, which can thus be used as food additive thanks to the health benefits it can provide. Those can be for example lyophilized in granules, pills or capsules, or directly mixed with dairy products for consumption.

In the context of the present invention co-administration of several (poly)peptides for use according to the invention is particularly preferred, so as to enhance the immune response.

The invention is set out in the appended set of claims and an object pertains to a pharmaceutical composition comprising
(i) a peptide consisting of an amino acid sequence as set forth in SEQ ID NO: 279; and
(ii) a peptide consisting of an amino acid sequence as set forth in SEQ ID NO: 289;

and, optionally, one or more pharmaceutically acceptable excipients
for use in prevention and/or treatment of an adrenal cancer.

According to a preferred embodiment, the composition of the invention comprises at least 3 (poly)peptides (which may be in the form of immunogenic compounds) as defined above, such as at least 4 (poly)peptides, or at least 5 (poly)peptides, or at least 6 (poly)peptides, or at least 7 (poly)peptides, or at least 8 (poly)peptides, or at least 9 (poly)peptides, or at least 10 (poly)peptides, or at least 11 (poly)peptides, or at least 12 (poly)peptides, or at least 13 (poly)peptides, or at least 14 (poly)peptides, or at least 15 (poly)peptides, or at least 20 (poly)peptides, or at least 25 (poly)peptides, or at least 50 (poly)peptides, or at least 100 (poly)peptides, or at least 500 (poly)peptides, or at least 1000 (poly)peptides, or at least 1500 (poly)peptides.

In particular, (at least) three (poly)peptides of the group consisting of:
- an IL13RA2 (poly)peptide, wherein it is a peptide consisting of an amino acid sequence as set forth in SEQ ID NO: 279;,
- a BIRC5 (poly)peptide, wherein it is a peptide consisting of an amino acid sequence as set forth in SEQ ID NO: 289, and
- a FOXM1 (poly)peptide for use as described above,
may be combined.

In an instance not encompassed by the wording of the claims, the pharmaceutical composition may comprise:
(i) a (poly)peptide comprising an epitope of IL13RA2 or a sequence variant thereof having at least 70% sequence identity, as described above;
(ii) an immunogenic compound as described above comprising the (poly)peptide as defined in (i) (i.e., the (poly)peptide comprising an epitope of IL13RA2 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

In an instance not encompassed by the wording of the claims, with regard to IL13RA2 (poly)peptides, the pharmaceutical composition may comprise:
(i) a (poly)peptide as described above comprising an amino acid sequence as set forth in SEQ ID NO: 279;
(ii) an immunogenic compound as described above comprising a (poly)peptide comprising an amino acid sequence as set forth in SEQ ID NO: 279;
(iii) a nanoparticle as described above loaded with a (poly)peptide comprising an amino acid sequence as set forth in SEQ ID NO: 279 or with an immunogenic compound comprising a (poly)peptide comprising an amino acid sequence as set forth in SEQ ID NO: 279;
(iv) a nucleic acid as described above comprising a polynucleotide encoding a (poly)peptide comprising an amino acid sequence as set forth in SEQ ID NO: 279 or an immunogenic compound comprising a (poly)peptide comprising an amino acid sequence as set forth in SEQ ID NO: 279; or
(v) a cytotoxic T cell as described above specific for a (poly)peptide comprising an amino acid sequence as set forth in SEQ ID NO: 279.

According to the invention and as set forth in the claims, , with regard to IL13RA2 (poly)peptides, the pharmaceutical composition may compriseJ:
(i) a (poly)peptide as described above consisting of an amino acid sequence as set forth in SEQ ID NO: 279;
(ii) an immunogenic compound as described above comprising a (poly)peptide consisting of amino acid sequence as set forth in SEQ ID NO: 279; or
(iii) a nanoparticle as described above loaded with a (poly)peptide consisting of an amino acid sequence as set forth in SEQ ID NO: 279.

In an instance not encompassed by the wording of the claims, in addition to IL13RA2 (poly)peptides as defined above), the pharmaceutical composition further comprise:
(i) a (poly)peptide comprising an epitope of BIRC5 or a sequence variant thereof having at least 70% sequence identity, as described above;
(ii) an immunogenic compound as described above comprising the (poly)peptide as defined in (i) (i.e., the (poly)peptide comprising an epitope of BIRC5 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

According to the invention, in addition to the to IL13RA2 (poly)peptides as defined above the pharmaceutical composition may comprise :
(i) a BIRC5 (poly)peptide as described above consisting of an amino acid sequence as set forth in SEQ ID NO: 289;
(ii) an immunogenic compound as described above comprising a (poly)peptide consisting of an amino acid sequence as set forth in SEQ ID NO: 289; or
(iii) a nanoparticle as described above loaded with a (poly)peptide comprising an amino acid sequence as set forth in 289 or with an immunogenic compound comprising a (poly)peptide comprising an amino acid sequence as set forth in SEQ ID NO: 289;.

Moreover, in addition to the to IL13RA2 (poly)peptides and the BIRC5 (poly)peptides as defined above, the pharmaceutical composition may further comprise:
(i) a (poly)peptide comprising an epitope of FOXM1 or a sequence variant thereof having at least 70% sequence identity, as described above;
(ii) an immunogenic compound as described above comprising the (poly)peptide as defined in (i) (i.e., the (poly)peptide comprising an epitope of FOXM1 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

For example, the pharmaceutical composition may comprise in addition to the to IL13RA2 (poly)peptides and the BIRC5 (poly)peptides as defined above :
(i) a (poly)peptide as described above comprising an amino acid sequence as set forth in SEQ ID NO: 312;
(ii) an immunogenic compound as described above comprising a (poly)peptide comprising an amino acid sequence as set forth in SEQ ID NO: 312;
(iii) a nanoparticle as described above loaded with a (poly)peptide comprising an amino acid sequence as set forth in 312 or with an immunogenic compound comprising a (poly)peptide comprising an amino acid sequence as set forth in SEQ ID NO: 312;
(iv) a nucleic acid as described above comprising a polynucleotide encoding a (poly)peptide comprising an amino acid sequence as set forth in SEQ ID NO: 312 or an immunogenic compound comprising a (poly)peptide comprising an amino acid sequence as set forth in SEQ ID NO: 312; or
(v) a cytotoxic T cell as described above specific for a (poly)peptide comprising an amino acid sequence as set forth in SEQ ID NO: 312.

In an instance not encompassed by the wording of the claims,, the pharmaceutical composition may comprise (i) a (poly)peptide comprising an epitope of IL13RA2 or a sequence variant thereof having at least 70% sequence identity, as described above and (ii) a (poly)peptide comprising an epitope of BIRC5 or a sequence variant thereof having at least 70% sequence identity, as described above. In an instance not encompassed by the wording of the claims,, the pharmaceutical composition may comprise (i) a (poly)peptide comprising an epitope of IL13RA2 or a sequence variant thereof having at least 70% sequence identity, as described above and (ii) a (poly)peptide comprising an epitope of FOXM1 or a sequence variant thereof having at least 70% sequence identity, as described above. In an instance not encompassed by the wording of the claims,, the pharmaceutical composition may comprise (i) a (poly)peptide comprising an epitope of IL13RA2 or a sequence variant thereof having at least 70% sequence identity, as described above; (ii) a (poly)peptide comprising an epitope of BIRC5 or a sequence variant thereof having at least 70% sequence identity, as described above; and (iii) a (poly)peptide comprising an epitope of FOXM1 or a sequence variant thereof having at least 70% sequence identity, as described above.

Preferred examples of (poly)peptides comprising an epitope of IL13RA2, BIRC5 and FOXM1, respectively, or a sequence variant thereof having at least 70% sequence identity, as described above, include the (poly)peptide as described above comprising an amino acid sequence as set forth in SEQ ID NO: 279 (IL13RA2 epitope variant), SEQ ID NO: 289 (BIRC5 epitope variant) and SEQ ID NO: 312 (FOXM1 epitope variant), respectively. In an instance not encompassed by the wording of the claims,, the pharmaceutical composition may comprise (i) a (poly)peptide as described above comprising an amino acid sequence as set forth in SEQ ID NO: 279 and (ii) a (poly)peptide as described above comprising an amino acid sequence as set forth in SEQ ID NO: 289. In an instance not encompassed by the wording of the claims,, the pharmaceutical composition may comprise (i) a (poly)peptide as described above comprising an amino acid sequence as set forth in SEQ ID NO: 279 and (ii) a (poly)peptide as described above comprising an amino acid sequence as set forth in SEQ ID NO: 312. In an instance not encompassed by the wording of the claims, the pharmaceutical composition may comprise (i) a (poly)peptide as described above comprising an amino acid sequence as set forth in SEQ ID NO: 279; (ii) a (poly)peptide as described above comprising an amino acid sequence as set forth in SEQ ID NO: 289; and (iii) a (poly)peptide as described above comprising an amino acid sequence as set forth in SEQ ID NO: 312. Preferably, the pharmaceutical composition comprise (i) a (poly)peptide as described above consisting of an amino acid sequence as set forth in SEQ ID NO: 279; (ii) a (poly)peptide as described above consisting of an amino acid sequence as set forth in SEQ ID NO: 289; and (iii) a (poly)peptide as described above consisting of an amino acid sequence as set forth in SEQ ID NO: 312.

In an instance not encompassed by the wording of the claims,, the pharmaceutical composition may comprise:
(i) a (poly)peptide comprising an epitope of BIRC5 or a sequence variant thereof having at least 70% sequence identity, as described above;
(ii) an immunogenic compound as described above comprising the (poly)peptide as defined in (i) (i.e., the (poly)peptide comprising an epitope of BIRC5 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

For example, with regard to BIRC5 (poly)peptides, the pharmaceutical composition may comprise:
(i) a (poly)peptide as described above comprising an amino acid sequence as set forth in SEQ ID NO: 289;
(ii) an immunogenic compound as described above comprising a (poly)peptide comprising an amino acid sequence as set forth in SEQ ID NO: 289;
(iii) a nanoparticle as described above loaded with a (poly)peptide comprising an amino acid sequence as set forth in 289 or with an immunogenic compound comprising a (poly)peptide comprising an amino acid sequence as set forth in SEQ ID NO: 289;
(iv) a nucleic acid as described above comprising a polynucleotide encoding a (poly)peptide comprising an amino acid sequence as set forth in SEQ ID NO: 289 or an immunogenic compound comprising a (poly)peptide comprising an amino acid sequence as set forth in SEQ ID NO: 289; or
(v) a cytotoxic T cell as described above specific for a (poly)peptide comprising an amino acid sequence as set forth in SEQ ID NO: 289.

In an instance not encompassed by the wording of the claims, in addition to the (poly)peptide comprising an epitope of BIRC5 or a sequence variant thereof having at least 70% sequence identity (or the respective immunogenic compound, nanoparticle or nucleic acid), the pharmaceutical composition may further comprise:
(i) a (poly)peptide comprising an epitope of IL13RA2 or a sequence variant thereof having at least 70% sequence identity, as described above;
(ii) an immunogenic compound as described above comprising the (poly)peptide as defined in (i) (i.e., the (poly)peptide comprising an epitope of IL13RA2 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

In an instance not encompassed by the wording of the claims,, the pharmaceutical composition may comprise (in addition to the (poly)peptide comprising an epitope of BIRC5 or a sequence variant thereof having at least 70% sequence identity (or the respective immunogenic compound, nanoparticle or nucleic acid)):
(i) a (poly)peptide as described above comprising an amino acid sequence as set forth in SEQ ID NO: 279;
(ii) an immunogenic compound as described above comprising a (poly)peptide comprising an amino acid sequence as set forth in SEQ ID NO: 279;
(iii) a nanoparticle as described above loaded with a (poly)peptide comprising an amino acid sequence as set forth in SEQ ID NO: 279 or with an immunogenic compound comprising a (poly)peptide comprising an amino acid sequence as set forth in SEQ ID NO: 279;
(iv) a nucleic acid as described above comprising a polynucleotide encoding a (poly)peptide comprising an amino acid sequence as set forth in SEQ ID NO: 279 or an immunogenic compound comprising a (poly)peptide comprising an amino acid sequence as set forth in SEQ ID NO: 279;
(v) a cytotoxic T cell as described above specific for a (poly)peptide comprising an amino acid sequence as set forth in SEQ ID NO: 279.

In an instance not encompassed by the wording of the claims,, in addition to the (poly)peptide comprising an epitope of BIRC5 or a sequence variant thereof having at least 70% sequence identity (or the respective immunogenic compound, nanoparticle or nucleic acid), the pharmaceutical composition may further comprise:
(i) a (poly)peptide comprising an epitope of FOXM1 or a sequence variant thereof having at least 70% sequence identity, as described above;
(ii) an immunogenic compound as described above comprising the (poly)peptide as defined in (i) (i.e., the (poly)peptide comprising an epitope of FOXM1 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

In an instance not encompassed by the wording of the claims,, the pharmaceutical composition may comprise (in addition to the (poly)peptide comprising an epitope of BIRC5 or a sequence variant thereof having at least 70% sequence identity (or the respective immunogenic compound, nanoparticle or nucleic acid)):
(i) a (poly)peptide as described above comprising an amino acid sequence as set forth in SEQ ID NO: 312;
(ii) an immunogenic compound as described above comprising a (poly)peptide comprising an amino acid sequence as set forth in SEQ ID NO: 312;
(iii) a nanoparticle as described above loaded with a (poly)peptide comprising an amino acid sequence as set forth in 312 or with an immunogenic compound comprising a (poly)peptide comprising an amino acid sequence as set forth in SEQ ID NO: 312;
(iv) a nucleic acid as described above comprising a polynucleotide encoding a (poly)peptide comprising an amino acid sequence as set forth in SEQ ID NO: 312 or an immunogenic compound comprising a (poly)peptide comprising an amino acid sequence as set forth in SEQ ID NO: 312;
(v) a cytotoxic T cell as described above specific for a (poly)peptide comprising an amino acid sequence as set forth in SEQ ID NO: 312.

In an instance not encompassed by the wording of the claims,, the pharmaceutical composition may comprise (i) a (poly)peptide comprising an epitope of BIRC5 or a sequence variant thereof having at least 70% sequence identity, as described above and (ii) a (poly)peptide comprising an epitope of IL13RA2 or a sequence variant thereof having at least 70% sequence identity, as described above. In an instance not encompassed by the wording of the claims,, the pharmaceutical composition may comprise (i) a (poly)peptide comprising an epitope of BIRC5 or a sequence variant thereof having at least 70% sequence identity, as described above and (ii) a (poly)peptide comprising an epitope of FOXM1 or a sequence variant thereof having at least 70% sequence identity, as described above. In an instance not encompassed by the wording of the claims,, the pharmaceutical composition may comprise (i) a (poly)peptide comprising an epitope of IL13RA2 or a sequence variant thereof having at least 70% sequence identity, as described above; (ii) a (poly)peptide comprising an epitope of BIRC5 or a sequence variant thereof having at least 70% sequence identity, as described above; and (iii) a (poly)peptide comprising an epitope of FOXM1 or a sequence variant thereof having at least 70% sequence identity, as described above.

Preferred examples of (poly)peptides comprising an epitope of IL13RA2, BIRC5 and FOXM1, respectively, or a sequence variant thereof having at least 70% sequence identity, as described above, include the (poly)peptide as described above comprising an amino acid sequence as set forth in SEQ ID NO: 279 (IL13RA2 epitope variant), SEQ ID NO: 289 (BIRC5 epitope variant) and SEQ ID NO: 312 (FOXM1 epitope variant), respectively. In an instance not encompassed by the wording of the claims, the pharmaceutical composition may comprise (i) a (poly)peptide as described above comprising an amino acid sequence as set forth in SEQ ID NO: 289 and (ii) a (poly)peptide as described above comprising an amino acid sequence as set forth in SEQ ID NO: 279. In an instance not encompassed by the wording of the claims, the pharmaceutical composition may comprise (i) a (poly)peptide as described above comprising an amino acid sequence as set forth in SEQ ID NO: 289 and (ii) a (poly)peptide as described above comprising an amino acid sequence as set forth in SEQ ID NO: 312. In an instance not encompassed by the wording of the claims, the pharmaceutical composition may comprise (i) a (poly)peptide as described above comprising an amino acid sequence as set forth in SEQ ID NO: 279; (ii) a (poly)peptide as described above comprising an amino acid sequence as set forth in SEQ ID NO: 289; and (iii) a (poly)peptide as described above comprising an amino acid sequence as set forth in SEQ ID NO: 312. Preferably, the pharmaceutical composition may comprise (i) a (poly)peptide as described above consisting of an amino acid sequence as set forth in SEQ ID NO: 279; (ii) a (poly)peptide as described above consisting of an amino acid sequence as set forth in SEQ ID NO: 289; and (iii) a (poly)peptide as described above consisting of an amino acid sequence as set forth in SEQ ID NO: 312.

In an instance not encompassed by the wording of the claims, the pharmaceutical composition may comprise:
(i) a (poly)peptide comprising an epitope of FOXM1 or a sequence variant thereof having at least 70% sequence identity, as described above;
(ii) an immunogenic compound as described above comprising the (poly)peptide as defined in (i) (i.e., the (poly)peptide comprising an epitope of FOXM1 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

In an instance not encompassed by the wording of the claims, with regard to FOXM1 (poly)peptides, the pharmaceutical composition may comprise:
(i) a (poly)peptide as described above comprising an amino acid sequence as set forth in SEQ ID NO: 312;
(ii) an immunogenic compound as described above comprising a (poly)peptide comprising an amino acid sequence as set forth in SEQ ID NO: 312;
(iii) a nanoparticle as described above loaded with a (poly)peptide comprising an amino acid sequence as set forth in 312 or with an immunogenic compound comprising a (poly)peptide comprising an amino acid sequence as set forth in SEQ ID NO: 312;
(iv) a nucleic acid as described above comprising a polynucleotide encoding a (poly)peptide comprising an amino acid sequence as set forth in SEQ ID NO: 312 or an immunogenic compound comprising a (poly)peptide comprising an amino acid sequence as set forth in SEQ ID NO: 312;
(v) a cytotoxic T cell as described above specific for a (poly)peptide comprising an amino acid sequence as set forth in SEQ ID NO: 312.

In an instance not encompassed by the wording of the claims in addition to the (poly)peptide comprising an epitope of FOXM1 or a sequence variant thereof having at least 70% sequence identity (or the respective immunogenic compound, nanoparticle or nucleic acid), the pharmaceutical composition may further comprise:
(i) a (poly)peptide comprising an epitope of IL13RA2 or a sequence variant thereof having at least 70% sequence identity, as described above;
(ii) an immunogenic compound as described above comprising the (poly)peptide as defined in (i) (i.e., the (poly)peptide comprising an epitope of IL13RA2 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii); or
(v) a cytotoxic T cell as described above specific for the (poly)peptide as defined in (i).

In an instance not encompassed by the wording of the claims, the pharmaceutical composition may comprise (in addition to the (poly)peptide comprising an epitope of FOXM1 or a sequence variant thereof having at least 70% sequence identity (or the respective immunogenic compound, nanoparticle or nucleic acid)):
(i) a (poly)peptide as described above comprising an amino acid sequence as set forth in SEQ ID NO: 279;
(ii) an immunogenic compound as described above comprising a (poly)peptide comprising an amino acid sequence as set forth in SEQ ID NO: 279;
(iii) a nanoparticle as described above loaded with a (poly)peptide comprising an amino acid sequence as set forth in SEQ ID NO: 279 or with an immunogenic compound comprising a (poly)peptide comprising an amino acid sequence as set forth in SEQ ID NO: 279;
(iv) a nucleic acid as described above comprising a polynucleotide encoding a (poly)peptide comprising an amino acid sequence as set forth in SEQ ID NO: 279 or an immunogenic compound comprising a (poly)peptide comprising an amino acid sequence as set forth in SEQ ID NO: 279;
(v) a cytotoxic T cell as described above specific for a (poly)peptide comprising an amino acid sequence as set forth in SEQ ID NO: 279.

In an instance not encompassed by the wording of the claims, in addition to the (poly)peptide comprising an epitope of FOXM1 or a sequence variant thereof having at least 70% sequence identity (or the respective immunogenic compound, nanoparticle or nucleic acid), the pharmaceutical composition may further comprise:
(i) a (poly)peptide comprising an epitope of BIRC5 or a sequence variant thereof having at least 70% sequence identity, as described above;
(ii) an immunogenic compound as described above comprising the (poly)peptide as defined in (i) (i.e., the (poly)peptide comprising an epitope of BIRC5 or a sequence variant thereof having at least 70% sequence identity, as described above);
(iii) a nanoparticle as described above loaded with the (poly)peptide as defined in (i) or the immunogenic compound as defined in (ii);
(iv) a nucleic acid as described above comprising a polynucleotide encoding the (poly)peptide as defined in (i) or the immunogenic compound as defined in (ii);
(v) a cytotoxic T cell as described above specific for the (poly)peptide as defined in (i).

In an instance not encompassed by the wording of the claims, the pharmaceutical composition may comprise (in addition to the (poly)peptide comprising an epitope of FOXM1 or a sequence variant thereof having at least 70% sequence identity (or the respective immunogenic compound, nanoparticle or nucleic acid)):
(i) a (poly)peptide as described above comprising an amino acid sequence as set forth in SEQ ID NO: 289;
(ii) an immunogenic compound as described above comprising a (poly)peptide comprising an amino acid sequence as set forth in SEQ ID NO: 289;
(iii) a nanoparticle as described above loaded with a (poly)peptide comprising an amino acid sequence as set forth in 289 or with an immunogenic compound comprising a (poly)peptide comprising an amino acid sequence as set forth in SEQ ID NO: 289;
(iv) a nucleic acid as described above comprising a polynucleotide encoding a (poly)peptide comprising an amino acid sequence as set forth in SEQ ID NO: 289 or an immunogenic compound comprising a (poly)peptide comprising an amino acid sequence as set forth in SEQ ID NO: 289; or
(v) a cytotoxic T cell as described above specific for a (poly)peptide comprising an amino acid sequence as set forth in SEQ ID NO: 289.

In an instance not encompassed by the wording of the claims, the pharmaceutical composition may comprise (i) a (poly)peptide comprising an epitope of FOXM1 or a sequence variant thereof having at least 70% sequence identity, as described above and (ii) a (poly)peptide comprising an epitope of IL13RA2 or a sequence variant thereof having at least 70% sequence identity, as described above. In an instance not encompassed by the wording of the claims, the pharmaceutical composition may comprise (i) a (poly)peptide comprising an epitope of FOXM1 or a sequence variant thereof having at least 70% sequence identity, as described above and (ii) a (poly)peptide comprising an epitope of BIRC5 or a sequence variant thereof having at least 70% sequence identity, as described above. In an instance not encompassed by the wording of the claims, the pharmaceutical composition may comprise (i) a (poly)peptide comprising an epitope of IL13RA2 or a sequence variant thereof having at least 70% sequence identity, as described above; (ii) a (poly)peptide comprising an epitope of BIRC5 or a sequence variant thereof having at least 70% sequence identity, as described above; and (iii) a (poly)peptide comprising an epitope of FOXM1 or a sequence variant thereof having at least 70% sequence identity, as described above.

Preferred examples of (poly)peptides comprising an epitope of IL13RA2, BIRC5 and FOXM1, respectively, or a sequence variant thereof having at least 70% sequence identity, as described above, include the (poly)peptide as described above comprising an amino acid sequence as set forth in SEQ ID NO: 279 (IL13RA2 epitope variant), SEQ ID NO: 289 (BIRC5 epitope variant) and SEQ ID NO: 312 (FOXM1 epitope variant), respectively. In an instance not encompassed by the wording of the claims, the pharmaceutical composition may comprise (i) a (poly)peptide as described above comprising an amino acid sequence as set forth in SEQ ID NO: 312 and (ii) a (poly)peptide as described above comprising an amino acid sequence as set forth in SEQ ID NO: 279. In an instance not encompassed by the wording of the claims, the pharmaceutical composition may comprise (i) a (poly)peptide as described above comprising an amino acid sequence as set forth in SEQ ID NO: 312 and (ii) a (poly)peptide as described above comprising an amino acid sequence as set forth in SEQ ID NO: 289. In an instance not encompassed by the wording of the claims, the pharmaceutical composition may comprise (i) a (poly)peptide as described above comprising an amino acid sequence as set forth in SEQ ID NO: 279; (ii) a (poly)peptide as described above comprising an amino acid sequence as set forth in SEQ ID NO: 289; and (iii) a (poly)peptide as described above comprising an amino acid sequence as set forth in SEQ ID NO: 312.

In an instance not encompassed by the wording of the claims, the pharmaceutical composition preferably comprises
(i) a (poly)peptide comprising an amino acid sequence as set forth in SEQ ID NO: 279, a (poly)peptide comprising an amino acid sequence as set forth in SEQ ID NO: 289, and a (poly)peptide comprising an amino acid sequence as set forth in SEQ ID NO: 312;
(ii) an immunogenic compound comprising a (poly)peptide comprising an amino acid sequence as set forth in SEQ ID NO: 279, an immunogenic compound comprising a (poly)peptide comprising an amino acid sequence as set forth in SEQ ID NO: 289, and an immunogenic compound comprising a (poly)peptide comprising an amino acid sequence as set forth in SEQ ID NO: 312;
(iii) a nanoparticle loaded with a (poly)peptide comprising an amino acid sequence as set forth in SEQ ID NO: 279, a nanoparticle loaded with a (poly)peptide comprising an amino acid sequence as set forth in SEQ ID NO: 289, and a nanoparticle loaded with a (poly)peptide comprising an amino acid sequence as set forth in SEQ ID NO: 312;
(iv) a nucleic acid comprising a polynucleotide encoding a (poly)peptide having an amino acid sequence as set forth in SEQ ID NO: 279, a nucleic acid comprising a polynucleotide encoding an (poly)peptide having an amino acid sequence as set forth in SEQ ID NO: 289, and a nucleic acid comprising a polynucleotide encoding an (poly)peptide having an amino acid sequence as set forth in SEQ ID NO: 312; or
(v) a cytotoxic T cell as described above specific for a (poly)peptide having an amino acid sequence as set forth in SEQ ID NO: 279, a cytotoxic T cell as described above specific for a (poly)peptide having an amino acid sequence as set forth in SEQ ID NO: 289, and a cytotoxic T cell as described above specific for a (poly)peptide having an amino acid sequence as set forth in SEQ ID NO: 312.

Preferably, the pharmaceutical composition comprises
(i) a (poly)peptide consisting of an amino acid sequence as set forth in SEQ ID NO: 279, a (poly)peptide consisting of an amino acid sequence as set forth in SEQ ID NO: 289, and a (poly)peptide consisting of an amino acid sequence as set forth in SEQ ID NO: 312;
(ii) an immunogenic compound comprising a (poly)peptide consisting of an amino acid sequence as set forth in SEQ ID NO: 279, an immunogenic compound comprising a (poly)peptide consisting of an amino acid sequence as set forth in SEQ ID NO: 289, and an immunogenic compound comprising a (poly)peptide comprising an amino acid sequence as set forth in SEQ ID NO: 312;
(iii) a nanoparticle loaded with a (poly)peptide comprising an amino acid sequence as set forth in SEQ ID NO: 279, a nanoparticle loaded with a (poly)peptide consisting of an amino acid sequence as set forth in SEQ ID NO: 289, and a nanoparticle loaded with a (poly)peptide consisting of an amino acid sequence as set forth in SEQ ID NO: 312;

The composition according to the invention can further comprise other active agents, for example such, which can enhance the effects of the (poly)peptide or immunogenic compound. Alternatively, the composition may not comprise any other active agents (i.e., other than the (poly)peptide according to the present invention, the immunogenic compound according to the present invention, the nanoparticle according to the present invention, the cell according to the present invention, the nucleic acid according to the present invention, or the host cell according to the present invention).

According to a preferred embodiment, the pharmaceutical composition for use according to the present invention further comprises at least one immunostimulatory agent, in particular so as to potentiate the immune response mediated by the (poly)peptide. Preferred immunostimulatory agents according to the invention include, without limitation, immune adjuvants, antigen-presenting cells, and combinations thereof. Accordingly, it is preferred that the immunostimulatory agent is an immuno-adjuvant (immune adjuvant) or an antigen-presenting cell (APC).

Some immune adjuvants are indeed capable of favoring and prolonging the duration of interaction between an antigen and the immune system, while others are capable of recruiting and activating cells of the natural immunity so as to induce an adaptive response. The adjuvants belonging to the former category include, without limitation, mineral compounds such as alum, aluminum hydroxide, aluminum phosphate, calcium phosphate hydroxide; and oil-based emulsions such as paraffin oil, starch oil, Freund's complete/incomplete adjuvant (FCA/FIA), saponins (e.g. from the plants Quillaja, Soybean, Polygala senega). The adjuvants of belonging to the latter category include, without limitation, immunostimulatory complexes (ISCOMs) such as cytokines (e.g. GM-CSF; Interleukins such as IL-1, IL-2, IL6, IL8, or IL12; Tumor necrosis factors (TNFs) such as TNFα or TNFβ ; Interferons IFNS such as IFNα, IFNβ, IFNγ or IFNδ, etc); ligands of toll-like receptors (TLRs) such as imiquimod, resiquimod or MPL; exosomes such as exosomes derived from dendritic cells (DCs); bacterial products such as heat-shock proteins (HSPs such as gp96, hsp90, hsp70, calreticulin, hsp110, hsp170), pathogen-associated molecular patterns (PAMPs), trehalose dimicolate (TDM), muramyldipeptide (MDP), polysaccharide (PLS) such as polysaccharide-K.

More preferably, the immune adjuvant is a protein/peptide having immuno-adjuvant properties, such as providing stimulation of CD4+ Th1 cells, as described herein. A preferred example thereof is an antigen distinct from IL13RA2, BIRC5 and/or FOXM1 that recalls immune memory or provides a non-specific help or could be a specific helper peptide, such as tetanus helper peptide, keyhole limpet hemocyanin peptide or PADRE peptide. In particular, the immune adjuvant may be the HHD-DR3 peptide of sequence MAKTIAYDEEARRGLERGLN (SEQ ID NO: 266). This peptide represents another example of a helper peptide (having immuno-adjuvant properties), which is preferred in the context of the present invention. Another preferred example is h-pAg T13L (sequence: TPPAYRPPNAPIL; SEQ ID NO: 280; Bhasin M, Singh H, Raghava GP (2003) MHCBN: a comprehensive database of MHC binding and non-binding peptides. Bioinformatics 19: 665-666). Further examples of preferred immune adjuvants, in particular of helper peptides, include the UCP2 peptide (for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2) and the BIRC5 peptide (for example as described in EP2119726 A1 or in Widenmeyer M, Griesemann **H,** Stevanović S, Feyerabend S, Klein R, Attig S, Hennenlotter J, Wernet D, Kuprash DV, Sazykin AY, Pascolo S, Stenzl A, Gouttefangeas C, Rammensee HG: Promiscuous survivin peptide induces robust CD4+ T-cell responses in the majority of vaccinated cancer patients. Int J Cancer. 2012 Jul 1;131(1):140-9. doi: 10.1002/ijc.26365. Epub 2011 Sep 14). The most preferred helper peptide is the UCP2 peptide (amino acid sequence: KSVWSKLQSIGIRQH; SEQ ID NO: 281, for example as described in WO 2013/135553 A1 or in Dosset M, Godet Y, Vauchy C, Beziaud L, Lone YC, Sedlik C, Liard C, Levionnois E, Clerc B, Sandoval F, Daguindau E, Wain-Hobson S, Tartour E, Langlade-Demoyen P, Borg C, Adotévi O: Universal cancer peptide-based therapeutic vaccine breaks tolerance against telomerase and eradicates established tumor. Clin Cancer Res. 2012 Nov 15;18(22):6284-95. doi: 10.1158/1078-0432.CCR-12-0896. Epub 2012 Oct 2).

Particularly preferred adjuvants are polyinosinic:polycytidylic acid (also referred to as "poly I:C") and/or its derivative poly-ICLC. Poly I:C is a mismatched double-stranded RNA with one strand being a polymer of inosinic acid, the other a polymer of cytidylic acid. Poly I:C is an immunostimulant known to interact with toll-like receptor 3 (TLR3). Poly I:C is structurally similar to double-stranded RNA, which is the "natural" stimulant of TLR3. Accordingly, poly I:C may be considered a synthetic analog of double-stranded RNA. Poly-ICLC is a synthetic complex of carboxymethylcellulose, polyinosinic-polycytidylic acid, and poly-L-lysine double-stranded RNA. Similar to poly I:C, also poly-ICLC is a ligand for TLR3. Poly I:C and poly-ICLC typically stimulate the release of cytotoxic cytokines. A preferred example of poly-ICLC is Hiltonol^{®}.

Most preferably, the adjuvant is Montanide, such as Montanide ISA 51 VG and/or Montanide ISA 720 VG. Those adjuvants are rendering stable water-in-oil emulsions when mixed with water based antigenic media. Montanide ISA 51 VG is based on a blend of mannide monooleate surfactant and mineral oil, whereas Montanide ISA 720 VG uses a non-mineral oil (Aucouturier J, Dupuis L, Deville S, Ascarateil S, Ganne V. Montanide ISA 720 and 51: a new generation of water in oil emulsions as adjuvants for human vaccines. Expert Rev Vaccines. 2002 Jun;1(1):111-8; Ascarateil S, Puget A, Koziol M-E. Safety data of Montanide ISA 51 VG and Montanide ISA 720 VG, two adjuvants dedicated to human therapeutic vaccines. Journal for Immunotherapy of Cancer. 2015;3(Suppl 2):P428. doi:10.1186/2051-1426-3-S2-P428).

Antigen-presenting cells (APCs) are also of particular interest, as their main function is to process antigens and present it on the cell surface to the T cells of the immune system, so as to initiate and modulate T-cell responses in vivo. In the present composition, it is preferred that the APCs are loaded with the (poly)peptide(s) and/or immunogenic compound(s) according to the invention, which can be done by exposing APCs in vitro with said (poly)peptide(s) and/or immunogenic compound(s) (Rizzo et al., Ex vivo loading of autologous dendritic cells with tumor antigens. Methods Mol Biol. 2014;1139:41-4; Rolinski and Hus, Breaking immunotolerance of tumors: a new perspective for dendritic cell therapy. J Immunotoxicol. 2014 Oct;11(4):311-8).

Preferred antigen-presenting cells according to the invention are dendritic cells (DCs). It can indeed be advantageous to combine at least one (poly)peptide or immunogenic compound according to the invention with dendritic cells, as those are the most potent antigen-presenting cells and have been reported to be frequently functionally defective in cancer patients. Dendritic cells can be easily obtained by the skilled person in the art from either healthy compatible donors (i.e. the dendritic cells are HLA-related) or from the patient himself provided that they are functional (i.e. the dendritic cells are autologous), for example by direct isolation from the peripheral blood, or by derivation from peripheral blood cells such as CD14+ monocytes or CD34+ hematopoietic precursors (Emens et al., 2008). Dendritic cells can indeed be distinguished from other cells of peripheral blood by their surface markers, such as S100, p55, CD83, and/or OX62, and may thus be isolated and purified based on said markers using cell cultures techniques well-known in the art.

According to a preferred embodiment, the pharmaceutical composition may further comprise at least one anti-cancer therapeutic agent. Said therapeutic agent is thus preferably capable of preventing and/or treating the same type of cancer than the one for which the antigenic peptide according to the invention is used. Preferably, the anti-cancer therapeutic agent is selected from antibodies, tumor cell lysates, chemotherapeutic agents, radiotherapeutic agents, immune checkpoint modulators and combinations thereof.

Antibodies are particularly advantageous in cancer therapy as they can either bind to specific antigens on cancer cell surfaces, thereby directing the therapy to the tumor (i.e. these are referred as tumor-targeting antibodies), or block immune checkpoints that are dysregulated in cancer (i.e. these are referred herein as immunomodulatory antibodies). The purpose of the later type of antibodies is to inhibit cancer immune resistance, which can notably be observed against T cells that are specific for tumor antigens. Indeed, as well-known in the art, under normal physiological conditions, immune checkpoints are crucial for the maintenance of self-tolerance (i.e. prevention of autoimmunity) and protect tissues from damage when the immune system is responding to pathogenic infection. However, in cancer, immune-checkpoints expression can be dysregulated as an important mechanism of immune resistance. Said resistance has notably been observed in melanoma, ovarian, lung, glioblastoma, breast, and pancreatic cancers with regard to the PD-L1 checkpoint (Konishi et al., B7-H1 expression on non-small cell lung cancer cells and its relationship with tumor-infiltrating lymphocytes and their PD-1 expression. Clin Cancer Res. 2004 Aug 1;10(15):5094-100; Ghebeh et al., The B7-H1 (PD-L1) T lymphocyte-inhibitory molecule is expressed in breast cancer patients with infiltrating ductal carcinoma: correlation with important high-risk prognostic factors. Neoplasia. 2006 Mar;8(3):190-8; Hino et al., Tumor cell expression of programmed cell death-1 ligand 1 is a prognostic factor for malignant melanoma. Cancer. 2010 Apr 1;116(7):1757-66). Other examples of immune checkpoints include, without limitation, PD-L2, PD-1, CD80, CD86, CTLA-4, B7H3, B7H4, PVR, TIGIT, GAL9, LAG-3, GITR, CD137, TIM3, VISTA, VISTA-R (Pico de Coaña et al., Checkpoint blockade for cancer therapy: revitalizing a suppressed immune system. Trends Mol Med. 2015 Aug;21(8):482-91; Pardoll DM. The blockade of immune checkpoints in cancer immunotherapy. Nat Rev Cancer. 2012 Mar 22;12(4):252-64).

Antibodies are usually employed for the above purposes either in the form of naked monoclonal antibodies (i.e. non-conjugated), or conjugated to another molecule which can be toxic to cells or radioactive.

Examples of immunomodulatory antibodies include, without limitation, ipilimumab which blocks the CTLA4-dependent immune checkpoint, nivolumab and prembrolizubmab which both block the PDCD1-dependent immune checkpoint, as well as MPDL3280A, MEDI4736, MEDI0680, and MSB0010718C which all block the PD-L1-dependent immune checkpoint (Sharma and Allison, The future of immune checkpoint therapy. Science. 2015 Apr 3;348(6230):56-61).

Other antibodies for cancer immunotherapy have been described in Buqué et al., Trial Watch: Immunomodulatory monoclonal antibodies for oncological indications. Oncoimmunology. 2015 Mar 2;4(4):e1008814. eCollection 2015 Apr; Redman et al., Mechanisms of action of therapeutic antibodies for cancer. Mol Immunol. 2015 Oct;67(2 Pt A):28-45; Simpson and Caballero, Monoclonal antibodies for the therapy of cancer MC Proc. 2014; 8(Suppl 4): O6 as well as on the antibody society website (list of therapeutic monoclonal antibodies approved or in review in the European Union or United States available on the weblink http://www.antibodysociety.org/news/approved_mabs.php).

Tumor cell lysates may also be combined with the antigenic peptide(s) according to the invention. Tumor cells are indeed capable of priming the immune response, by presenting endogenous peptides-MHC complexes, as well as via dendritic cells (DCs) of the host which can process and present the antigen delivered by said lysates. The range of antigens against which an immune response can be induced is thereby increased. Tumor cell lysates can be easily obtained by treating tumor cells with a heat shock and/or a chemical treatment, and can be autologous (i.e. isolated from the patient), or allogeneic (i.e. isolated from another subject).

Standard chemotherapeutic drugs and radiotherapeutic agents need not be further described herein as they have been extensively described in the literature, notably by Baskar et al. (Baskar et al., Cancer and radiation therapy: current advances and future directions. Int J Med Sci. 2012;9(3):193-9), Paci et al., (Paci et al., Review of therapeutic drug monitoring of anticancer drugs part 1--cytotoxics. Eur J Cancer. 2014 Aug;50(12):2010-9) and Widmer et al. (Widmer et al., Review of therapeutic drug monitoring of anticancer drugs part two--targeted therapies. Eur J Cancer. 2014 Aug;50(12):2020-36). A list of such drugs and agents is also available on the cancer.gov website (http://www.cancer.gov/about-cancer/treatment/drugs). Non-limiting examples of chemotherapeutic drugs include etoposide, doxorubicin and cisplatin.

Preferably, the immune checkpoint modulator for combination with the antigenic peptide as defined herein is an activator or an inhibitor of one or more immune checkpoint point molecule(s) selected from CD27, CD28, CD40, CD122, CD137, OX40, GITR, ICOS, A2AR, B7-H3, B7-H4, BTLA, CD40, CTLA-4, IDO, KIR, LAG3, PD-1, TIM-3, VISTA, CEACAM1, GARP, PS, CSF1R, CD94/NKG2A, TDO, GITR, TNFR and/or FasR/DcR3; or an activator or an inhibitor of one or more ligands thereof.

More preferably, the immune checkpoint modulator is an activator of a (co-)stimulatory checkpoint molecule or an inhibitor of an inhibitory checkpoint molecule or a combination thereof. Accordingly, the immune checkpoint modulator is more preferably (i) an activator of CD27, CD28, CD40, CD122, CD137, OX40, GITR and/or ICOS or (ii) an inhibitor of A2AR, B7-H3, B7-H4, BTLA, CD40, CTLA-4, IDO, KIR, LAG3, PD-1, PDL-1, PD-L2, TIM-3, VISTA, CEACAM1, GARP, PS, CSF1R, CD94/NKG2A, TDO, TNFR and/or FasR/DcR3.

Even more preferably, the immune checkpoint modulator is an inhibitor of an inhibitory checkpoint molecule (but preferably no inhibitor of a stimulatory checkpoint molecule). Accordingly, the immune checkpoint modulator is even more preferably an inhibitor of A2AR, B7-H3, B7-H4, BTLA, CTLA-4, IDO, KIR, LAG3, PD-1, PDL-1, PD-L2, TIM-3, VISTA, CEACAM1, GARP, PS, CSF1R, CD94/NKG2A, TDO, TNFR and/or DcR3 or of a ligand thereof.

It is also preferred that the immune checkpoint modulator is an activator of a stimulatory or costimulatory checkpoint molecule (but preferably no activator of an inhibitory checkpoint molecule). Accordingly, the immune checkpoint modulator is more preferably an activator of CD27, CD28, CD40, CD122, CD137, OX40, GITR and/or ICOS or of a ligand thereof.

It is even more preferred that the immune checkpoint modulator is a modulator of the CD40 pathway, of the IDO pathway, of the LAG3 pathway, of the CTLA-4 pathway and/or of the PD-1 pathway. In particular, the immune checkpoint modulator is preferably a modulator of CD40, LAG3, CTLA-4, PD-L1, PD-L2, PD-1 and/or IDO, more preferably the immune checkpoint modulator is an inhibitor of CTLA-4, PD-L1, PD-L2, PD-1, LAG3, and/or IDO or an activator of CD40, even more preferably the immune checkpoint modulator is an inhibitor of CTLA-4, PD-L1, PD-1, LAG3 and/or IDO, even more preferably the immune checkpoint modulator is an inhibitor of LAG3, CTLA-4 and/or PD-1, and most preferably the immune checkpoint modulator is an inhibitor of CTLA-4 and/or PD-1.

Accordingly, the checkpoint modulator for combination with the antigenic peptide may be selected from known modulators of the CTLA-4 pathway or the PD-1 pathway. Preferably, the checkpoint modulator for combination with the antigenic peptide as defined herein may be selected from known modulators of the CTLA-4 pathway or the PD-1 pathway. Particularly preferably, the immune checkpoint modulator is a PD-1 inhibitor. Preferred inhibitors of the CTLA-4 pathway and of the PD-1 pathway include the monoclonal antibodies Yervoy^{®} (Ipilimumab; Bristol Myers Squibb) and Tremelimumab (Pfizer/Medimmune) as well as Opdivo^{®} (Nivolumab; Bristol Myers Squibb), Keytruda^{®} (Pembrolizumab, also known as Lambrolizumab or MK-3475; Merck), Imfinzi^{®} (Durvalumab, also known as MEDI4736; MedImmune/AstraZeneca), Tecentriq^{®} (Atezolizumab, also known as MPDL3280A; Roche/Genentech), Pidilizumab (CT-011; CureTech), MEDI0680 (AMP-514; AstraZeneca), Bavencio^{®} (Avelumab; Merck KGaA/Pfizer, also known as MSB-0010718C), MIH1 (Affymetrix), LY3300054 (Eli Lilly) and Spartalizumab (also known as PDR001; Novartis). More preferred checkpoint inhibitors include the CTLA-4 inhibitors Yervoy^{®} (Ipilimumab; Bristol Myers Squibb) and Tremelimumab (Pfizer/Medimmune) as well as the PD-1 inhibitors Opdivo^{®} (Nivolumab; Bristol Myers Squibb), Keytruda^{®} (Pembrolizumab; Merck), Pidilizumab (CT-011; CureTech), MEDI0680 (AMP-514; AstraZeneca), AMP-224 (a PD-L2 Fc fusion protein; MedImmune).

It is also preferred that the immune checkpoint modulator for combination with the (poly)peptide for use as defined herein is selected from the group consisting of Pembrolizumab, Ipilimumab, Nivolumab, Atezolizumab, Durvalumab, Tremelimumab, Avelumab, Spartalizumab, LAG525 (an anti-LAG-3 monoclonal antibody), Epacadostat (also known as INCB24360; an IDO inhibitor), Varlilumab (an anti-CD27 monoclonal antibody), Urelumab (an anti-CD137 monoclonal antibody), AMP-224 and CM-24 (an anti-CEACAM1 monoclonal antibody). In some embodiments, the immune checkpoint modulator for combination with the (poly)peptide for use as defined herein is Pembrolizumab.

Preferably, the anti-cancer agent may be cabozantinib. Cabozantinib is a small molecule tyrosine kinase inhibitor that initially showed activity in medullary thyroid cancer and was recently approved by the Food and Drug Administration for the treatment of metastatic renal cell carcinoma after progression on first line therapy.

Furthermore, it is preferred that the anticancer agent may be mitotane. Mitotane is a steroidogenesis inhibitor and cytostatic antineoplastic medication, which is a derivative of the early insecticide DDT and an isomer of p,p'-DDD, and is also known as 1,1-(dichlorodiphenyl)-2,2-dichloroethane (o,p'-DDD).

The anti-cancer therapeutic agent can also be administered in combination with the composition of the invention, either simultaneously, separately, or sequentially. Should the composition and the therapeutic agent be administered in a separate or sequential manner, those may be administered in distinct pharmaceutical forms.

Thus, in another aspect, the invention relates to a composition of the invention and at least one anti-cancer therapeutic agent as described above, as a combined preparation for a simultaneous, separate, or sequential administration. In other terms, the invention proposes a combined use of the composition the invention and least one anti-cancer therapeutic agent as described above, for a simultaneous, separate, or sequential administration.

In particular, the pharmaceutical composition for use according to the present invention as described above may be used as a vaccine for immunotherapy. Moreover,
- the immunogenic compound for use as disclosed herein,
- the (poly)peptide for use as disclosed herein,
- the nanoparticle for use as disclosed herein,
- the cell for use as disclosed herein,
- the nucleic acid for use as disclosed herein,
- the host cell for use as disclosed herein,
- the cytotoxic T cell (CTL) for use as disclosed herein, or
- the pharmaceutical composition for use according to the present invention and the embodiments thereof as described above may be used as vaccine, in particular for immunotherapy for preventing and/or treating an adrenal cancer.

As used in the context of the present invention, the term "vaccine" refers to a biological preparation that provides innate and/or adaptive immunity, typically to a particular disease, preferably an adrenal cancer. Thus, a vaccine supports in particular an innate and/or an adaptive immune response of the immune system of a subject to be treated. For example, the (poly)peptide according to the present invention typically leads to or supports an adaptive immune response in the patient to be treated.

In the context of the present invention, the vaccine (composition) can induce a specific immune response against an antigen/protein, and is thus preferably used to prevent or treat an adrenal cancer.

In an instance not encompassed by the wording of the claims, the present invention also provides a kit of parts comprising at least one of
- the (poly)peptide as described above;
- the immunogenic compound as described above;
- the nanoparticle as described above;
- the cell as described above;
- the nucleic acid as described above;
- the host cell as described above;
- the cytotoxic T cell (CTL) as described above; and/or
- the pharmaceutical composition as described above
for use in prevention and/or treatment of an adrenal cancer.

In particular, the kit-of-parts as disclosed herein may comprise more than one of the above described components. For example, the kit-of-parts as disclosed herein may comprise at least two different immunogenic compounds, at least two different (poly)peptides, at least two different nanoparticles, at least two different cells, at least two different nucleic acids, at least two different host cells, at least two different CTLs, and/or at least two different pharmaceutical compositions.

Preferably, such different components comprised by the kit-of-parts as described above differ in the (poly)peptides according to the present invention, for example one component relating to a first (poly)peptide, such as a (poly)peptide (poly)peptide comprising an epitope of IL13RA2 or a sequence variant thereof having at least 70% sequence identity as described above (also referred to herein as "IL13RA2 (poly)peptide"), and one component relating to a second (poly)peptide (distinct from the first (poly)peptide), such as a (poly)peptide comprising an epitope of BIRC5 or a sequence variant thereof having at least 70% sequence identity as described above (also referred to herein as "BIRC5 (poly)peptide"). In some embodiments, the first component may relate to a (poly)peptide (poly)peptide comprising an epitope of IL13RA2 or a sequence variant thereof having at least 70% sequence identity as described above, and the other component may relate to a (poly)peptide comprising an epitope of FOXM1 or a sequence variant thereof having at least 70% sequence identity as described above (also referred to herein as "FOXM1 (poly)peptide"). In some embodiments, the first component may relate to a (poly)peptide (poly)peptide comprising an epitope of BIRC5 or a sequence variant thereof having at least 70% sequence identity as described above, and the other component may relate to a (poly)peptide comprising an epitope of FOXM1 or a sequence variant thereof having at least 70% sequence identity as described above. Preferably, the kit comprises three distinct components, with the first component relating to a (poly)peptide (poly)peptide comprising an epitope of IL13RA2 or a sequence variant thereof having at least 70% sequence identity as described above; the second component relating to a (poly)peptide comprising an epitope of BIRC5 or a sequence variant thereof having at least 70% sequence identity as described above; and the third component relating to a (poly)peptide comprising an epitope of FOXM1 or a sequence variant thereof having at least 70% sequence identity as described above.

For example, the kit may comprise
- a (poly)peptide as described above comprising an amino acid sequence as set forth in SEQ ID NO: 279, and
- a (poly)peptide as described above comprising an amino acid sequence as set forth in SEQ ID NO: 289.

For example, the kit may comprise
- an immunogenic compound as described above comprising a (poly)peptide comprising an amino acid sequence as set forth in SEQ ID NO: 279, and
- an immunogenic compound as described above comprising a (poly)peptide comprising an amino acid sequence as set forth in SEQ ID NO: 289.

For example, the kit may comprise
- a nanoparticle as described above comprising a (poly)peptide comprising an amino acid sequence as set forth in SEQ ID NO: 279, and
- a nanoparticle as described above comprising a (poly)peptide comprising an amino acid sequence as set forth in SEQ ID NO: 289.

For example, the kit may comprise
- a nucleic acid as described above encoding a (poly)peptide comprising an amino acid sequence as set forth in SEQ ID NO: 279, and
- a nucleic acid as described above encoding a (poly)peptide comprising an amino acid sequence as set forth in SEQ ID NO: 289.

For example, the kit may comprise
- a cytotoxic T cell (CTL) as described above specific for a (poly)peptide comprising an amino acid sequence as set forth in SEQ ID NO: 279, and
- a cytotoxic T cell (CTL) as described above specific for a (poly)peptide comprising an amino acid sequence as set forth in SEQ ID NO: 289.

For example, the kit may comprise
- a (poly)peptide as described above comprising an amino acid sequence as set forth in SEQ ID NO: 279, and
- a (poly)peptide as described above comprising an amino acid sequence as set forth in SEQ ID NO: 312.

For example, the kit may comprise
- an immunogenic compound as described above comprising a (poly)peptide comprising an amino acid sequence as set forth in SEQ ID NO: 279, and
- an immunogenic compound as described above comprising a (poly)peptide comprising an amino acid sequence as set forth in SEQ ID NO: 312.

For example, the kit may comprise
- a nanoparticle as described above comprising a (poly)peptide comprising an amino acid sequence as set forth in SEQ ID NO: 279, and
- a nanoparticle as described above comprising a (poly)peptide comprising an amino acid sequence as set forth in SEQ ID NO: 312.

For example, the kit may comprise
- a nucleic acid as described above encoding a (poly)peptide comprising an amino acid sequence as set forth in SEQ ID NO: 279, and
- a nucleic acid as described above encoding a (poly)peptide comprising an amino acid sequence as set forth in SEQ ID NO: 312.

For example, the kit may comprise
- a cytotoxic T cell (CTL) as described above specific for a (poly)peptide comprising an amino acid sequence as set forth in SEQ ID NO: 279, and
- a cytotoxic T cell (CTL) as described above specific for a (poly)peptide comprising an amino acid sequence as set forth in SEQ ID NO: 312.

For example, the kit may comprise
- a (poly)peptide as described above comprising an amino acid sequence as set forth in SEQ ID NO: 289, and
- a (poly)peptide as described above comprising an amino acid sequence as set forth in SEQ ID NO: 312.

For example, the kit may comprise
- an immunogenic compound as described above comprising a (poly)peptide comprising an amino acid sequence as set forth in SEQ ID NO: 289, and
- an immunogenic compound as described above comprising a (poly)peptide comprising an amino acid sequence as set forth in SEQ ID NO: 312.

For example, the kit may comprise
- a nanoparticle as described above comprising a (poly)peptide comprising an amino acid sequence as set forth in SEQ ID NO: 289, and
- a nanoparticle as described above comprising a (poly)peptide comprising an amino acid sequence as set forth in SEQ ID NO: 312.

For example, the kit may comprise
- a nucleic acid as described above encoding a (poly)peptide comprising an amino acid sequence as set forth in SEQ ID NO: 289, and
- a nucleic acid as described above encoding a (poly)peptide comprising an amino acid sequence as set forth in SEQ ID NO: 312.

For example, the kit may comprise
- a cytotoxic T cell (CTL) as described above specific for a (poly)peptide comprising an amino acid sequence as set forth in SEQ ID NO: 289, and
- a cytotoxic T cell (CTL) as described above specific for a (poly)peptide comprising an amino acid sequence as set forth in SEQ ID NO: 312.

Preferably, the kit may comprise
- a (poly)peptide as described above comprising an amino acid sequence as set forth in SEQ ID NO: 279,
- a (poly)peptide as described above comprising an amino acid sequence as set forth in SEQ ID NO: 289, and
- a (poly)peptide as described above comprising an amino acid sequence as set forth in SEQ ID NO: 312.

For example, the kit may comprise
- an immunogenic compound as described above comprising a (poly)peptide comprising an amino acid sequence as set forth in SEQ ID NO: 279,
- an immunogenic compound as described above comprising a (poly)peptide comprising an amino acid sequence as set forth in SEQ ID NO: 289, and
- an immunogenic compound as described above comprising a (poly)peptide comprising an amino acid sequence as set forth in SEQ ID NO: 312.

For example, the kit may comprise
- a nanoparticle as described above comprising a (poly)peptide comprising an amino acid sequence as set forth in SEQ ID NO: 279,
- a nanoparticle as described above comprising a (poly)peptide comprising an amino acid sequence as set forth in SEQ ID NO: 289, and
- a nanoparticle as described above comprising a (poly)peptide comprising an amino acid sequence as set forth in SEQ ID NO: 312.

For example, the kit may comprise
- a nucleic acid as described above encoding a (poly)peptide comprising an amino acid sequence as set forth in SEQ ID NO: 279,
- a nucleic acid as described above encoding a (poly)peptide comprising an amino acid sequence as set forth in SEQ ID NO: 289, and
- a nucleic acid as described above encoding a (poly)peptide comprising an amino acid sequence as set forth in SEQ ID NO: 312.

For example, the kit may comprise
- a cytotoxic T cell (CTL) as described above specific for a (poly)peptide comprising an amino acid sequence as set forth in SEQ ID NO: 279,
- a cytotoxic T cell (CTL) as described above specific for a (poly)peptide comprising an amino acid sequence as set forth in SEQ ID NO: 289, and
- a cytotoxic T cell (CTL) as described above specific for a (poly)peptide comprising an amino acid sequence as set forth in SEQ ID NO: 312.

The various components of the kit-of-parts may be packaged in one or more containers. The above components may be provided in a lyophilized or dry form or dissolved in a suitable buffer. The kit may also comprise additional reagents including, for instance, preservatives, growth media, and/or buffers for storage and/or reconstitution of the above-referenced components, washing solutions, and the like.

In addition, the kit-of-parts as disclosed herein may optionally contain instructions of use. Accordingly, it is preferred that the kit further comprises a package insert or instruction leaflet with directions to prevent and/or to treat an adrenal cancer by using the (poly)peptide for use as disclosed herein, the immunogenic compound for use as disclosed herein, the nanoparticle for use as disclosed herein, the cell for use as disclosed herein, the nucleic acid for use as disclosed herein, the host cell for use as disclosed herein, or the pharmaceutical composition for use according to the present invention.

In an instance not encompassed by the wording of the claims, the present disclosure also provides a vaccination kit for treating, preventing and/or stabilizing adrenal cancer, comprising the pharmaceutical composition as described herein or the vaccine as described herein and instructions for use of said pharmaceutical composition or of said vaccine in the prevention and/or treatment of adrenal cancer.

It is also preferred that, in addition to any of components as described above, the kit comprises an anti-cancer agent as described herein.

### Combination therapy

The administration of the (poly)peptide according to the present invention, the immunogenic compound according to the present invention, the nanoparticle according to the present invention, the cell according to the present invention, the nucleic acid according to the present invention, the host cell according to the present invention, and the pharmaceutical composition according to the present invention, in particular in the methods and uses according to the invention, can be carried out alone or in combination with a co-agent useful for treating and/or preventing an adrenal cancer, such as an anti-cancer agent.

The co-agent can be administered in association with the (poly)peptide comprising the epitope of IL13RA2, BIRC5 or FOXM1, or the sequence variant thereof, for use according to the present invention, the immunogenic compound according to the present invention, the nanoparticle according to the present invention, the cell according to the present invention, the nucleic acid according to the present invention, the host cell according to the present invention, or the pharmaceutical composition according to the present invention, either at about the same time or consecutively as described herein and in the same or distinct pharmaceutical forms.

The co-agent is thus preferably capable of preventing and/or treating the same type of cancer as the one for which the (poly)peptide as described above is used. Particularly preferred anti-cancer therapeutic agents according to the invention include, without limitation, antibodies, tumor cell lysates, chemotherapeutic agents, radiotherapeutic agents, immune checkpoint modulators and combinations thereof.

Antibodies are particularly advantageous in cancer therapy as they can either bind to specific antigens on cancer cell surfaces, thereby directing the therapy to the tumor (i.e. these are referred as tumor-targeting antibodies), or block immune checkpoints that are dysregulated in cancer (i.e. these are referred herein as immunomodulatory antibodies). The purpose of the later type of antibodies is to inhibit cancer immune resistance, which can notably be observed against T cells that are specific for tumour antigens. Indeed, as well-known in the art, under normal physiological conditions, immune checkpoints are crucial for the maintenance of self-tolerance (i.e. prevention of autoimmunity) and protect tissues from damage when the immune system is responding to pathogenic infection. However, in cancer, immune-checkpoints expression can be dysregulated as an important mechanism of immune resistance. Said resistance has notably been observed in melanoma, ovarian, lung, glioblastoma, breast, and pancreatic cancers with regard to the PD-L1 checkpoint (Konishi et al., B7-H1 expression on non-small cell lung cancer cells and its relationship with tumor-infiltrating lymphocytes and their PD-1 expression. Clin Cancer Res. 2004 Aug 1;10(15):5094-100; Ghebeh et al., The B7-H1 (PD-L1) T lymphocyte-inhibitory molecule is expressed in breast cancer patients with infiltrating ductal carcinoma: correlation with important high-risk prognostic factors. Neoplasia. 2006 Mar;8(3):190-8; Hino et al., Tumor cell expression of programmed cell death-1 ligand 1 is a prognostic factor for malignant melanoma. Cancer. 2010 Apr 1;116(7):1757-66). Other examples of immune checkpoints include, without limitation, PD-L2, PD-1, CD80, CD86, CTLA4, B7H3, B7H4, PVR, TIGIT, GAL9, LAG-3, GITR, CD137, TIM3, VISTA, VISTA-R (Pico de Coaña et al., Checkpoint blockade for cancer therapy: revitalizing a suppressed immune system. Trends Mol Med. 2015 Aug;21(8):482-91; Pardoll DM1. The blockade of immune checkpoints in cancer immunotherapy. Nat Rev Cancer. 2012 Mar 22;12(4):252-64).

Antibodies are usually employed for the above purposes either in the form of naked monoclonal antibodies (i.e. non-conjugated), or conjugated to another molecule which can be toxic to cells or radioactive.

Examples of well-known immunomodulatory antibodies include, without limitation, ipilimumab which blocks the CTLA4-dependent immune checkpoint, nivolumab and prembrolizubmab which both block the PDCD1-dependent immune checkpoint, as well as MPDL3280A, MEDI4736, MEDI0680, and MSB0010718C which all block the PD-L1-dependent immune checkpoint (Sharma and Allison, The future of immune checkpoint therapy. Science. 2015 Apr 3;348(6230):56-61).

Other antibodies for cancer immunotherapy have been described in Buqué et al. (Buqué et al., Trial Watch: Immunomodulatory monoclonal antibodies for oncological indications. Oncoimmunology. 2015 Mar 2;4(4):e1008814. eCollection 2015 Apr), Redman et al. (Redman et al., Mechanisms of action of therapeutic antibodies for cancer. Mol Immunol. 2015 Oct;67(2 Pt A):28-45), and in Simpson and Caballero, Monoclonal antibodies for the therapy of cancer MC Proc. 2014; 8(Suppl 4): O6 as well as on the antibody society website (list of therapeutic monoclonal antibodies approved or in review in the European Union or United States available on the weblink http://www.antibodysociety.org/news/approved_mabs.php).

Tumor cell lysates may also be combined with the antigenic peptide(s) according to the invention. Tumor cells are indeed capable of priming the immune response, by presenting endogenous peptides-MHC complexes, as well as via dendritic cells (DCs) of the host which can process and present the antigen delivered by said lysates. The range of antigens against which an immune response can be induced is thereby increased. Tumor cell lysates can be easily obtained by treating tumor cells with a heat shock and/or a chemical treatment, and can be autologous (i.e. isolated from the patient), or allogeneic (i.e. isolated from another subject).

Standard chemotherapeutic drugs and radiotherapeutic agents need not be further described herein as they have been extensively described in the literature, notably by Baskar et al. (Baskar et al., Cancer and radiation therapy: current advances and future directions. Int J Med Sci. 2012;9(3):193-9), Paci et al. (Paci et al., Review of therapeutic drug monitoring of anticancer drugs part 1--cytotoxics. Eur J Cancer. 2014 Aug;50(12):2010-9) and Widmer et al. (Widmer et al., Review of therapeutic drug monitoring of anticancer drugs part two--targeted therapies. Eur J Cancer. 2014 Aug;50(12):2020-36). A list of such drugs and agents is also available on the cancer.gov website (http://www.cancer.gov/about-cancer/treatment/drugs). Non-limiting examples of chemotherapeutic drugs include etoposide, doxorubicin and cisplatin.

Preferably, the immune checkpoint modulator for combination with the antigenic peptide as defined herein is an activator or an inhibitor of one or more immune checkpoint point molecule(s) selected from CD27, CD28, CD40, CD122, CD137, OX40, GITR, ICOS, A2AR, B7-H3, B7-H4, BTLA, CD40, CTLA-4, IDO, KIR, LAG3, PD-1, TIM-3, VISTA, CEACAM1, GARP, PS, CSF1R, CD94/NKG2A, TDO, GITR, TNFR and/or FasR/DcR3; or an activator or an inhibitor of one or more ligands thereof.

More preferably, the immune checkpoint modulator is an activator of a (co-)stimulatory checkpoint molecule or an inhibitor of an inhibitory checkpoint molecule or a combination thereof. Accordingly, the immune checkpoint modulator is more preferably (i) an activator of CD27, CD28, CD40, CD122, CD137, OX40, GITR and/or ICOS or (ii) an inhibitor of A2AR, B7-H3, B7-H4, BTLA, CD40, CTLA-4, IDO, KIR, LAG3, PD-1, PDL-1, PD-L2, TIM-3, VISTA, CEACAM1, GARP, PS, CSF1R, CD94/NKG2A, TDO, TNFR and/or FasR/DcR3.

Even more preferably, the immune checkpoint modulator is an inhibitor of an inhibitory checkpoint molecule (but preferably no inhibitor of a stimulatory checkpoint molecule). Accordingly, the immune checkpoint modulator is even more preferably an inhibitor of A2AR, B7-H3, B7-H4, BTLA, CTLA-4, IDO, KIR, LAG3, PD-1, PDL-1, PD-L2, TIM-3, VISTA, CEACAM1, GARP, PS, CSF1R, CD94/NKG2A, TDO, TNFR and/or DcR3 or of a ligand thereof.

It is also preferred that the immune checkpoint modulator is an activator of a stimulatory or costimulatory checkpoint molecule (but preferably no activator of an inhibitory checkpoint molecule). Accordingly, the immune checkpoint modulator is more preferably an activator of CD27, CD28, CD40, CD122, CD137, OX40, GITR and/or ICOS or of a ligand thereof.

It is even more preferred that the immune checkpoint modulator is a modulator of the CD40 pathway, of the IDO pathway, of the LAG3 pathway, of the CTLA-4 pathway and/or of the PD-1 pathway. In particular, the immune checkpoint modulator is preferably a modulator of CD40, LAG3, CTLA-4, PD-L1, PD-L2, PD-1 and/or IDO, more preferably the immune checkpoint modulator is an inhibitor of CTLA-4, PD-L1, PD-L2, PD-1, LAG3, and/or IDO or an activator of CD40, even more preferably the immune checkpoint modulator is an inhibitor of CTLA-4, PD-L1, PD-1, LAG3 and/or IDO, even more preferably the immune checkpoint modulator is an inhibitor of LAG3, CTLA-4 and/or PD-1, and most preferably the immune checkpoint modulator is an inhibitor of CTLA-4 and/or PD-1.

Accordingly, the checkpoint modulator for combination with the antigenic peptide may be selected from known modulators of the CTLA-4 pathway or the PD-1 pathway. Preferably, the checkpoint modulator for combination with the antigenic peptide as defined herein may be selected from known modulators of the CTLA-4 pathway or the PD-1 pathway. Particularly preferably, the immune checkpoint modulator is a PD-1 inhibitor. Preferred inhibitors of the CTLA-4 pathway and of the PD-1 pathway include the monoclonal antibodies Yervoy^{®} (Ipilimumab; Bristol Myers Squibb) and Tremelimumab (Pfizer/Medimmune) as well as Opdivo^{®} (Nivolumab; Bristol Myers Squibb), Keytruda^{®} (Pembrolizumab; also known as Lambrolizumab or MK-3475; Merck), Imfinzi^{®} (Durvalumab also known as MEDI4736; MedImmune/AstraZeneca), Tecentriq^{®} (Atezolizumab also known as MPDL3280A; Roche/Genentech), Pidilizumab (CT-011; CureTech), MEDI0680 (AMP-514; AstraZeneca), Bavencio^{®} (Avelumab; Merck KGaA/Pfizer also known as MSB-0010718C), MIH1 (Affymetrix), LY3300054 (Eli Lilly) and and Spartalizumab (also known as PDR001; Novartis). More preferred checkpoint inhibitors include the CTLA-4 inhibitors Yervoy^{®} (Ipilimumab; Bristol Myers Squibb) and Tremelimumab (Pfizer/Medimmune) as well as the PD-1 inhibitors Opdivo^{®} (Nivolumab; Bristol Myers Squibb), Keytruda^{®} (Pembrolizumab; Merck), Pidilizumab (CT-011; CureTech), MEDI0680 (AMP-514; AstraZeneca), AMP-224 (a PD-L2 Fc fusion protein; MedImmune).

It is also preferred that the immune checkpoint modulator for combination with the antigenic peptide as defined herein is selected from the group consisting of Pembrolizumab, Ipilimumab, Nivolumab, Atezolizumab, MEDI4736, Tremelimumab, Avelumab, Spartalizumab, LAG525 (an anti-LAG3 monoclonal antibody), Epacadostat (formely INCB24360; an IDO inhibitor), Varlilumab (an anti-CD27 monoclonal antibody), Urelumab (an anti-CD137 monoclonal antibody), AMP-224 and CM-24 (an anti-CEACAM1 monoclonal antibody). In some embodiments, the immune checkpoint modulator for combination with the (poly)peptide for use as defined herein is Pembrolizumab.

Preferably, the anti-cancer agent may be cabozantinib. Cabozantinib is a small molecule tyrosine kinase inhibitor that initially showed activity in medullary thyroid cancer and was recently approved by the Food and Drug Administration for the treatment of metastatic renal cell carcinoma after progression on first line therapy.

Furthermore, it is preferred that the anticancer agent may be mitotane. Mitotane is a steroidogenesis inhibitor and cytostatic antineoplastic medication, which is a derivative of the early insecticide DDT and an isomer of p,p'-DDD, and is also known as 1,1-(dichlorodiphenyl)-2,2-dichloroethane (o,p'-DDD).

The anti-cancer therapeutic agent can also be administered in association with the antigenic peptide according to the present invention, the immunogenic compound according to the present invention, the nanoparticle according to the present invention, the cell according to the present invention, the nucleic acid according to the present invention, the host cell according to the present invention, or the pharmaceutical composition according to the present invention, either at about the same time or consecutively as described herein and in the same or distinct pharmaceutical forms. Thus, the invention proposes a combined use of the composition the invention and least one anti-cancer therapeutic agent as described above, for a simultaneous, separate, or sequential administration as described herein.

Furthermore, the present invention also relates to a combination of at least two distinct (poly)peptides as defined in the claims for use in the prevention and/or treatment of an adrenal cancer. Furthermore, the present invention also relates to a combination of at least two distinct immunogenic compounds as defined in the claims for use in the prevention and/or treatment of an adrenal cancer. Furthermore, the present invention also relates to a combination of at least two distinct nanoparticles as defined in the claims for use in the prevention and/or treatment of an adrenal cancer. Furthermore, the present disclosure also relates to a combination of at least two distinct nucleic acids as disclosed herein , e.g. for use in the prevention and/or treatment of an adrenal cancer.

Thus, at least two antigenic peptides as defined in the claims are administered in combination, for example in the same pharmaceutical composition. For example, at least 3 antigenic peptides, at least 4 antigenic peptides, at least 5 antigenic peptides, at least 6 antigenic peptides, at least 7 antigenic peptides, at least 8 antigenic peptides, at least 9 antigenic peptides, at least 10 antigenic peptides, at least 11 antigenic peptides, at least 12 antigenic peptides, at least 13 antigenic peptides, at least 14 antigenic peptides, at least 15 antigenic peptides, at least 20 antigenic peptides, at least 25 antigenic peptides, at least 50 antigenic peptides, at least 100 antigenic peptides, at least 500 antigenic peptides, at least 1000 antigenic peptides, or at least 1500 antigenic peptides are administered in combination, for example in the same pharmaceutical composition.

In an instance not encompassed by the wording of the claims two distinct antigenic peptides according to the present invention (e.g., relating to distinct reference antigens, such as IL13RA2, BIRC5 and/or FOXM1) are combined. For example,
(i) at least two distinct (poly)peptides as described above comprising an epitope of IL13RA2, BIRC5 or FOXM1 or a sequence variant thereof having at least 70% sequence identity as described above;
(ii) at least two distinct immunogenic compounds as described above;
(iii) at least two distinct nanoparticles as described above; or
(iv) at least two distinct nucleic acids as described above; or
(v) at least two distinct cytotoxic T cells as described above
may be combined.

In an instance not encompassed by the wording of the claims, the present disclosure provides a combination, e.g. for use in the prevention and/or treatment of an adrenal cancer, comprising (i) a (poly)peptide, which comprises or consists of an epitope of IL13RA2 or a sequence variant thereof having at least 70% sequence identity as described above, and (ii) a (poly)peptide, which comprises or consists of an epitope of BIRC5 or a sequence variant thereof having at least 70% sequence identity as described above.According to the invention, the IL13RA2 (poly)peptide consists of a microbiota sequence variant of the IL13RA2 epitope (human reference peptide) of SEQ ID NO: 263, which is the (poly)peptide consisting of an amino acid sequence as set forth in SEQ ID NO: 279, and the BIRC5 (poly)peptide consists of a microbiota sequence variant of the BIRC5 epitope (human reference peptide) of SEQ ID NO: 286, which is the (poly)peptide consisting of an amino acid sequence as set forth in SEQ ID NO: 289. Even more preferably, the combination, e.g. for use in the prevention and/or treatment of an adrenal cancer, comprises a (poly)peptide comprising or consisting of SEQ ID NO: 279 and a (poly)peptide comprising or consisting of SEQ ID NO: 289.

In an instance not encompassed by the wording of the claims, the present disclosure provides a combination, e.g. for use in the prevention and/or treatment of an adrenal cancer, comprising (i) a (poly)peptide, which comprises or consists of an epitope of IL13RA2 or a sequence variant thereof having at least 70% sequence identity as described above, and (ii) a (poly)peptide, which comprises or consists of an epitope of FOXM1 or a sequence variant thereof having at least 70% sequence identity as described above. Preferably, the IL13RA2 (poly)peptide comprises or consists of a microbiota sequence variant of the IL13RA2 epitope (human reference peptide) of SEQ ID NO: 263, such as a (poly)peptide comprising or consisting of an amino acid sequence as set forth in SEQ ID NO: 279, and the FOXM1 (poly)peptide comprises or consists of a microbiota sequence variant of the FOXM1 epitope (human reference peptide) of SEQ ID NO: 293, such as a (poly)peptide comprising or consisting of an amino acid sequence as set forth in SEQ ID NO: 312. Even more preferably, the combination, e.g. for use in the prevention and/or treatment of an adrenal cancer, comprises a (poly)peptide comprising or consisting of SEQ ID NO: 279 and a (poly)peptide comprising or consisting of SEQ ID NO: 312.

In an instance not encompassed by the wording of the claims, the present disclosure provides a combination, e.g. for use in the prevention and/or treatment of an adrenal cancer, comprising (i) a (poly)peptide, which comprises or consists of an epitope of BIRC5 or a sequence variant thereof having at least 70% sequence identity as described above, and (ii) a (poly)peptide, which comprises or consists of an epitope of FOXM1 or a sequence variant thereof having at least 70% sequence identity as described above. Preferably, the BIRC5 (poly)peptide comprises or consists of a microbiota sequence variant of the BIRC5 epitope (human reference peptide) of SEQ ID NO: 286, such as a (poly)peptide comprising or consisting of an amino acid sequence as set forth in SEQ ID NO: 289, and the FOXM1 (poly)peptide comprises or consists of a microbiota sequence variant of the FOXM1 epitope (human reference peptide) of SEQ ID NO: 293, such as a (poly)peptide comprising or consisting of an amino acid sequence as set forth in SEQ ID NO: 312. Even more preferably, the combination, e.g. for use in the prevention and/or treatment of an adrenal cancer, comprises a (poly)peptide comprising or consisting of SEQ ID NO: 289 and a (poly)peptide comprising or consisting of SEQ ID NO: 312.

More preferably, the combination according to the present invention (e.g. for use in the prevention and/or treatment of a cancer) comprises at least three distinct components as described above, in particular at least three distinct (poly)peptides as described above. The above description regarding the combination of two distinct components applies accordingly for three distinct components.

In an instance not encompassed by the wording of the claims, the present disclosure provides a combination, e.g. for use in the prevention and/or treatment of an adrenal cancer, comprising (i) a (poly)peptide, which comprises or consists of an epitope of IL13RA2 or a sequence variant thereof having at least 70% sequence identity as described above, (ii) a (poly)peptide, which comprises or consists of an epitope of BIRC5 or a sequence variant thereof having at least 70% sequence identity as described above, and (iii) a (poly)peptide, which comprises or consists of an epitope of FOXM1 or a sequence variant thereof having at least 70% sequence identity as described above. Preferably, the combination. for use in the prevention and/or treatment of an adrenal cancer, comprises a (poly)peptide consisting of SEQ ID NO: 279, a (poly)peptide consisting of SEQ ID NO: 289, and a (poly)peptide consisting of SEQ ID NO: 312.

It is understood that the combination, e.g. for use in the prevention and/or treatment of a cancer, may also contain - instead of the above-described preferred combinations of antigenic peptides - a respective combination of immunogenic compounds of the invention, a respective combination of nanoparticles of the invention or a respective combination of nucleic acids of the invention.

In an instance not encompassed by the wording of the claims, the present invention provides a combination of
(i) a (poly)peptide, which comprises or consists of an epitope of IL13RA2 or a sequence variant thereof having at least 70% sequence identity as described above, and
(ii) a (poly)peptide, which comprises or consists of an epitope of BIRC5 or a sequence variant thereof having at least 70% sequence identity as described above,
for use in prevention and/or treatment of an adrenal cancer.

Preferably, the combination not encompassed by the wording of the claims for use according as disclosed herein comprises:
(i) a (poly)peptide as described above comprising an amino acid sequence as set forth in SEQ ID NO: 279, and
(ii) a (poly)peptide as described above comprising an amino acid sequence as set forth in SEQ ID NO: 289.

According to the invention, the combination for use as indicated in the claims herein may comprise:
(i) a (poly)peptide as described above consisting of an amino acid sequence as set forth in SEQ ID NO: 279, and
(ii) a (poly)peptide as described above consisting of an amino acid sequence as set forth in SEQ ID NO: 289.

Such a combination may further comprise a (poly)peptide, which comprises or consists of an epitope of FOXM1 or a sequence variant thereof having at least 70% sequence identity as described above, such as a (poly)peptide as described above comprising or consisting of an amino acid sequence as set forth in SEQ ID NO: 312.

For example, the present disclosure provides a combination of
(i) a (poly)peptide, which comprises or consists of an epitope of IL13RA2 or a sequence variant thereof having at least 70% sequence identity as described above, and
(ii) a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a (poly)peptide as described above comprising or consisting of an amino acid sequence as set forth in SEQ ID NO: 279, and
(ii) a (poly)peptide as described above comprising or consisting of an amino acid sequence as set forth in SEQ ID NO: 312.

Such a combination not encompassed by the wording of the claims may further comprise a (poly)peptide, which comprises or consists of an epitope of BIRC5 or a sequence variant thereof having at least 70% sequence identity as described above, such as a (poly)peptide as described above comprising or consisting of an amino acid sequence as set forth in SEQ ID NO: 289.

For example, the present disclosure provides a combination of
(i) a (poly)peptide, which comprises or consists of an epitope of BIRC5 or a sequence variant thereof having at least 70% sequence identity as described above, and
(ii) a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a (poly)peptide as described above comprising or consisting of an amino acid sequence as set forth in SEQ ID NO: 289, and
(ii) a (poly)peptide as described above comprising or consisting of an amino acid sequence as set forth in SEQ ID NO: 312.

Such a combination not encompassed by the wording of the claims may further comprise a (poly)peptide, which comprises or consists of an epitope of IL13RA2 or a sequence variant thereof having at least 70% sequence identity as described above, such as a (poly)peptide as described above comprising or consisting of an amino acid sequence as set forth in SEQ ID NO: 279.

For example, the present disclosure provides a combination of
(i) an immunogenic compound as described above comprising a (poly)peptide, which comprises or consists of an epitope of IL13RA2 or a sequence variant thereof having at least 70% sequence identity as described above, and
(ii) an immunogenic compound as described above comprising a (poly)peptide, which comprises or consists of an epitope of BIRC5 or a sequence variant thereof having at least 70% sequence identity as described above,
for use in prevention and/or treatment of an adrenal cancer.

Preferably, the combination not encompassed by the wording of the claims for use as disclosed herein comprises
(i) an immunogenic compound as described above comprising a (poly)peptide as described above comprising of an amino acid sequence as set forth in SEQ ID NO: 279, and
(ii) an immunogenic compound as described above comprising a (poly)peptide as described above comprising of an amino acid sequence as set forth in SEQ ID NO: 289.

According to the invention, the combination for use as indicated in the claims herein may comprise:
(i) an immunogenic compound as described above comprising a (poly)peptide consisting of an amino acid sequence as set forth in SEQ ID NO: 279, and
(ii) an immunogenic compound as described above comprising a (poly)peptide consisting of an amino acid sequence as set forth in SEQ ID NO: 289.

Such a combination may further comprise an immunogenic compound as described above comprising a (poly)peptide, which comprises or consists of an epitope of FOXM1 or a sequence variant thereof having at least 70% sequence identity as described above, such as an immunogenic compound as described above comprising a (poly)peptide as described above comprising or consisting of an amino acid sequence as set forth in SEQ ID NO: 312.

For example, the present disclosure provides a combination of
(i) an immunogenic compound as described above comprising a (poly)peptide, which comprises or consists of an epitope of IL13RA2 or a sequence variant thereof having at least 70% sequence identity as described above, and
(ii) an immunogenic compound as described above comprising a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) an immunogenic compound as described above comprising a (poly)peptide as described above comprising or consisting of an amino acid sequence as set forth in SEQ ID NO: 279, and
(ii) an immunogenic compound as described above comprising a (poly)peptide as described above comprising or consisting of an amino acid sequence as set forth in SEQ ID NO: 312.

Such a combination not encompassed by the wording of the claims may further comprise an immunogenic compound as described above comprising a (poly)peptide, which comprises or consists of an epitope of BIRC5 or a sequence variant thereof having at least 70% sequence identity as described above, such as an immunogenic compound as described above comprising a (poly)peptide as described above comprising or consisting of an amino acid sequence as set forth in SEQ ID NO: 289.

For example, the present disclosure provides a combination of
(i) an immunogenic compound as described above comprising a (poly)peptide, which comprises or consists of an epitope of BIRC5 or a sequence variant thereof having at least 70% sequence identity as described above, and
(ii) an immunogenic compound as described above comprising a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) an immunogenic compound as described above comprising a (poly)peptide as described above comprising or consisting of an amino acid sequence as set forth in SEQ ID NO: 289, and
(ii) an immunogenic compound as described above comprising a (poly)peptide as described above comprising or consisting of an amino acid sequence as set forth in SEQ ID NO: 312.

Such a combination not encompassed by the wording of the claims may further comprise an immunogenic compound as described above comprising a (poly)peptide, which comprises or consists of an epitope of IL13RA2 or a sequence variant thereof having at least 70% sequence identity as described above, such as an immunogenic compound as described above comprising a (poly)peptide as described above comprising or consisting of an amino acid sequence as set forth in SEQ ID NO: 279.

For example, the present disclosure provides a combination of
(i) a nanoparticle as described above comprising a (poly)peptide, which comprises or consists of an epitope of IL13RA2 or a sequence variant thereof having at least 70% sequence identity as described above, and
(ii) a nanoparticle as described above comprising a (poly)peptide, which comprises or consists of an epitope of BIRC5 or a sequence variant thereof having at least 70% sequence identity as described above,
for use in prevention and/or treatment of an adrenal cancer.

Preferably, the combination not encompassed by the wording of the claims for use as disclosed herein comprises
(i) a nanoparticle as described above comprising a (poly)peptide as described above comprising an amino acid sequence as set forth in SEQ ID NO: 279, and
(ii) a nanoparticle as described above comprising a (poly)peptide as described above comprising an amino acid sequence as set forth in SEQ ID NO: 289.

According to the invention, the combination for use as indicated in the claims may comprise:
(i) a nanoparticle as described above comprising a (poly)peptide as described above consisting of an amino acid sequence as set forth in SEQ ID NO: 279, and
(ii) a nanoparticle as described above comprising a (poly)peptide as described above consisting of an amino acid sequence as set forth in SEQ ID NO: 289.

Such a combination may further comprise a nanoparticle as described above comprising a (poly)peptide, which comprises or consists of an epitope of FOXM1 or a sequence variant thereof having at least 70% sequence identity as described above, such as a nanoparticle as described above comprising a (poly)peptide as described above comprising or consisting of an amino acid sequence as set forth in SEQ ID NO: 312.

For example, the present :disclosure provides a combination of
(i) a nanoparticle as described above comprising a (poly)peptide, which comprises or consists of an epitope of IL13RA2 or a sequence variant thereof having at least 70% sequence identity as described above, and
(ii) a nanoparticle as described above comprising a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as herein disclosed comprises
(i) a nanoparticle as described above comprising a (poly)peptide as described above comprising or consisting of an amino acid sequence as set forth in SEQ ID NO: 279, and
(ii) a nanoparticle as described above comprising a (poly)peptide as described above comprising or consisting of an amino acid sequence as set forth in SEQ ID NO: 312.

Such a combination not encompassed by the wording of the claims may further comprise a nanoparticle as described above comprising a (poly)peptide, which comprises or consists of an epitope of BIRC5 or a sequence variant thereof having at least 70% sequence identity as described above, such as a nanoparticle as described above comprising a (poly)peptide as described above comprising or consisting of an amino acid sequence as set forth in SEQ ID NO: 289.

For example, the present disclosure provides a combination of
(i) a nanoparticle as described above comprising a (poly)peptide, which comprises or consists of an epitope of BIRC5 or a sequence variant thereof having at least 70% sequence identity as described above, and
(ii) a nanoparticle as described above comprising a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein invention comprises
(i) a nanoparticle as described above comprising a (poly)peptide as described above comprising or consisting of an amino acid sequence as set forth in SEQ ID NO: 289, and
(ii) a nanoparticle as described above comprising a (poly)peptide as described above comprising or consisting of an amino acid sequence as set forth in SEQ ID NO: 312.

Such a combination not encompassed by the wording of the claims may further comprise a nanoparticle as described above comprising a (poly)peptide, which comprises or consists of an epitope of IL13RA2 or a sequence variant thereof having at least 70% sequence identity as described above, such as a nanoparticle as described above comprising a (poly)peptide as described above comprising or consisting of an amino acid sequence as set forth in SEQ ID NO: 279.

For example, the present disclosure provides a combination of
(i) a nucleic acid as described above encoding a (poly)peptide, which comprises or consists of an epitope of IL13RA2 or a sequence variant thereof having at least 70% sequence identity as described above, and
(ii) a nucleic acid as described above encoding a (poly)peptide, which comprises or consists of an epitope of BIRC5 or a sequence variant thereof having at least 70% sequence identity as described above,
for use in prevention and/or treatment of an adrenal cancer.

Preferably, the combination not encompassed by the wording of the claims for use as disclosed herein comprises
(i) a nucleic acid as described above encoding a (poly)peptide as described above comprising or consisting of an amino acid sequence as set forth in SEQ ID NO: 279, and
(ii) a nucleic acid as described above encoding a (poly)peptide as described above comprising or consisting of an amino acid sequence as set forth in SEQ ID NO: 289.

Such a combination not encompassed by the wording of the claims may further comprise a nucleic acid as described above encoding a (poly)peptide, which comprises or consists of an epitope of FOXM1 or a sequence variant thereof having at least 70% sequence identity as described above, such as a nucleic acid as described above encoding a (poly)peptide as described above comprising or consisting of an amino acid sequence as set forth in SEQ ID NO: 312.

For example, the present disclosure provides a combination of
(i) a nucleic acid as described above encoding a (poly)peptide, which comprises or consists of an epitope of IL13RA2 or a sequence variant thereof having at least 70% sequence identity as described above, and
(ii) a nucleic acid as described above encoding a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a nucleic acid as described above encoding a (poly)peptide as described above comprising or consisting of an amino acid sequence as set forth in SEQ ID NO: 279, and
(ii) a nucleic acid as described above encoding a (poly)peptide as described above comprising or consisting of an amino acid sequence as set forth in SEQ ID NO: 312.

Such a combination not encompassed by the wording of the claims may further comprise a nucleic acid as described above encoding a (poly)peptide, which comprises or consists of an epitope of BIRC5 or a sequence variant thereof having at least 70% sequence identity as described above, such as a nucleic acid as described above encoding a (poly)peptide as described above comprising or consisting of an amino acid sequence as set forth in SEQ ID NO: 289.

For example, the present disclosure provides a combination of
(i) a nucleic acid as described above encoding a (poly)peptide, which comprises or consists of an epitope of BIRC5 or a sequence variant thereof having at least 70% sequence identity as described above, and
(ii) a nucleic acid as described above encoding a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a nucleic acid as described above encoding a (poly)peptide as described above comprising or consisting of an amino acid sequence as set forth in SEQ ID NO: 289, and
(ii) a nucleic acid as described above encoding a (poly)peptide as described above comprising or consisting of an amino acid sequence as set forth in SEQ ID NO: 312.

Such a combination not encompassed by the wording of the claims may further comprise a nucleic acid as described above encoding a (poly)peptide, which comprises or consists of an epitope of IL13RA2 or a sequence variant thereof having at least 70% sequence identity as described above, such as a nucleic acid as described above encoding a (poly)peptide as described above comprising or consisting of an amino acid sequence as set forth in SEQ ID NO: 279.

For example, the present disclosure provides a combination of
(i) a cytotoxic T cell (CTL) as described above specific for a (poly)peptide, which comprises or consists of an epitope of IL13RA2 or a sequence variant thereof having at least 70% sequence identity as described above, and
(ii) a cytotoxic T cell (CTL) as described above specific for a (poly)peptide, which comprises or consists of an epitope of BIRC5 or a sequence variant thereof having at least 70% sequence identity as described above,
for use in prevention and/or treatment of an adrenal cancer.

Preferably, the combination not encompassed by the wording of the claims for use as disclosed herein comprises
(i) a cytotoxic T cell (CTL) as described above specific for a (poly)peptide as described above comprising or consisting of an amino acid sequence as set forth in SEQ ID NO: 279, and
(ii) a cytotoxic T cell (CTL) as described above specific for a (poly)peptide as described above comprising or consisting of an amino acid sequence as set forth in SEQ ID NO: 289.

Such a combination not encompassed by the wording of the claims may further comprise a cytotoxic T cell (CTL) as described above specific for a (poly)peptide, which comprises or consists of an epitope of FOXM1 or a sequence variant thereof having at least 70% sequence identity as described above, such as a cytotoxic T cell (CTL) as described above specific for a (poly)peptide as described above comprising or consisting of an amino acid sequence as set forth in SEQ ID NO: 312.

For example, the present disclosure provides a combination of
(i) a cytotoxic T cell (CTL) as described above specific for a (poly)peptide, which comprises or consists of an epitope of IL13RA2 or a sequence variant thereof having at least 70% sequence identity as described above, and
(ii) a cytotoxic T cell (CTL) as described above specific for a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a cytotoxic T cell (CTL) as described above specific for a (poly)peptide as described above comprising or consisting of an amino acid sequence as set forth in SEQ ID NO: 279, and
(ii) a cytotoxic T cell (CTL) as described above specific for a (poly)peptide as described above comprising or consisting of an amino acid sequence as set forth in SEQ ID NO: 312.

Such a combination not encompassed by the wording of the claims may further comprise a cytotoxic T cell (CTL) as described above specific for a (poly)peptide, which comprises or consists of an epitope of BIRC5 or a sequence variant thereof having at least 70% sequence identity as described above, such as a cytotoxic T cell (CTL) as described above specific for a (poly)peptide as described above comprising or consisting of an amino acid sequence as set forth in SEQ ID NO: 289.

For example, the present disclosure provides a combination of
(i) a cytotoxic T cell (CTL) as described above specific for a (poly)peptide, which comprises or consists of an epitope of BIRC5 or a sequence variant thereof having at least 70% sequence identity as described above, and
(ii) a cytotoxic T cell (CTL) as described above specific for a (poly)peptide, which comprises or consists of an epitope of FOXM1 or a sequence variant thereof having at least 70% sequence identity as described above,
for use in prevention and/or treatment of an adrenal cancer.

Preferably, the combination not encompassed by the wording of the claims for use as disclosed herein comprises
(i) a cytotoxic T cell (CTL) as described above specific for a (poly)peptide as described above comprising or consisting of an amino acid sequence as set forth in SEQ ID NO: 289, and
(ii) a cytotoxic T cell (CTL) as described above specific for a (poly)peptide as described above comprising or consisting of an amino acid sequence as set forth in SEQ ID NO: 312.

Such a combination not encompassed by the wording of the claims may further comprise a cytotoxic T cell (CTL) as described above specific for a (poly)peptide, which comprises or consists of an epitope of IL13RA2 or a sequence variant thereof having at least 70% sequence identity as described above, such as a cytotoxic T cell (CTL) as described above specific for a (poly)peptide as described above comprising or consisting of an amino acid sequence as set forth in SEQ ID NO: 279.

In general, the distinct components administered in a combination therapy as described herein may be administered simultaneously, in particular at about the same time, or sequentially/consecutively.

Preferably, the (active) components, which are to be combined, are administered at about the same time, in particular simultaneously. More preferably, the (active) components which are administered at about the same time, in particular simultaneously, are preferably administered in the same form (i.e., in the same type of formulation, e.g., as nanoparticles, as pharmaceutical compositions, etc.).

"At about the same time", as used herein, means in particular simultaneous administration or that directly after administration of (i) the first component, (ii) the second component is administered or directly after administration of (ii) the second component (i) the first component is administered. The skilled person understands that "directly after" includes the time necessary to prepare the second administration - in particular the time necessary for exposing and disinfecting the location for the second administration as well as appropriate preparation of the "administration device" (e.g., syringe, pump, etc.). Simultaneous administration also includes if the periods of administration of (i) the first component and of (ii) the second component overlap or if, for example, one component is administered over a longer period of time, such as 30 min, 1 h, 2 h or even more, e.g. by infusion, and the other component is administered at some time during such a long period. Administration of (i) the first component and of (ii) the second component at about the same time is in particular preferred if different routes of administration and/or different administration sites are used.

It is also preferred that the (active) components, which are to be combined, are administered consecutively. In more general, it is preferred that the first component and the second component are administered consecutively, wherein the (active) components are preferably administered in the same form (i.e., in the same type of formulation, e.g., as nanoparticles, as pharmaceutical compositions, etc.).

This means that (i) the first component is administered before or after (ii) the second component. In consecutive administration, the time between administration of the first component and administration of the second component is preferably no more than one week, more preferably no more than 3 days, even more preferably no more than 2 days and most preferably no more than 24 h. It is particularly preferred that (i) the first component and (ii) the second component are administered at the same day with the time between administration of the first component and administration of the second component being preferably no more than 6 hours, more preferably no more than 3 hours, even more preferably no more than 2 hours and most preferably no more than 1 h.

Preferably, the (active) components, which are to be combined, are administered via the same or distinct routes of administration.

Preferably, (i) the first component and (ii) the second component are administered via the same route of administration. In more general, it is preferred that the first component and the second component are administered via the same route of administration, wherein the (active) components are preferably administered in the same form (i.e., in the same type of formulation, e.g., as nanoparticles, as pharmaceutical compositions, etc.).

It is also preferred that the (active) components, which are to be combined, are administered via distinct routes of administration. In more general, it is preferred that the first component and the second component are administered via distinct routes of administration, wherein the (active) components are preferably administered in the same form (i.e., in the same type of formulation, e.g., as nanoparticles, as pharmaceutical compositions, etc.).

Preferably, the (active) components, which are to be combined, are comprised in the same or distinct compositions.

Preferably, the (active) components are comprised in the same composition. In more general, it is preferred that the first component and the second component are comprised in the same composition, wherein the (active) components are preferably administered in the same form (i.e., in the same type of formulation, e.g., as nanoparticles, etc.).

It is also preferred that the (active) components are comprised in distinct compositions. In more general, it is preferred that the first component and the second component are comprised in distinct compositions, wherein the (active) components, which are to be combined, are preferably administered in the same form (i.e., in the same type of formulation, e.g., as nanoparticles, etc.).

### Medical treatment and uses

As outlined above, the (poly)peptide, the immunogenic compound, the nanoparticle, the cell, the nucleic acid, the host cell, or the pharmaceutical composition for use according to the present invention is used for the prevention and/or treatment of an adrenal cancer. As used herein, the expression "prevention and/or treatment of an adrenal cancer" refers to ameliorating, reducing, preventing and/or treating an adrenal cancer or to reducing or preventing the recurrence of an adrenal cancer. Accordingly, the present invention also provides a method for ameliorating, reducing, preventing and/or treating an adrenal cancer or for reducing or preventing its recurrence in a subject comprising administering to the subject
- the pharmaceutical composition asdefined in the claims; and/or
- the combination as defined in the claims.

In an instance not encompassed by the wording of the claims, the present disclosure also provides a method for ameliorating, reducing, preventing and/or treating an adrenal cancer or for reducing or preventing its recurrence in a subject comprising administering to the subject
- the (poly)peptide as described herein;
- the immunogenic compound as described herein;
- the nanoparticle as described herein;
- the cell as described herein;
- the nucleic acid as described herein;
- the host cell as described herein;
- the cytotoxic T cell as described herein;

In an instance not encompassed by the wording of the claims, the (poly)peptide, the immunogenic compound, the nanoparticle, the cell, the nucleic acid, the host cell are used as medication/medicament for the prevention and or treatment of an adrenal cancer. According to the invention, the pharmaceutical composition as defined in the claims; and/or the combination as defined in the claims are used as medication/medicament for the prevention and or treatment of an adrenal cancer.

Accordingly, the term "medicament" or "medication" as used in the following refers to the (poly)peptide, the immunogenic compound , the nanoparticle, the cell, the nucleic acid, the host cell, or the pharmaceutical composition as disclosed herein for use as disclosed herein.

In particular, the medicament as defined above is used for triggering a specific immune response towards a particular antigen/protein, namely IL13RA2, BIRC5 and/or FOXM1 so as to prevent or treat an adrenal cancer in a patient in need thereof.

Methods of administration of a medicament are well-known to the skilled person in the art. For example the medicament can be directly administered into the subject, into the affected organ (i.e. local administration) or systemically (i.e. enteral or parenteral administration), or even applied *ex vivo* to cells derived from the subject or a human cell line which are subsequently administered to the subject, or even used *in vitro* to select a subpopulation of immune cells derived from the subject, which are then re-administered to the said subject. Administration may be by enteral or parenteral routes. Enteral administrations as used herein includes oral and rectal administrations, as well as administrations via gastric feeding tubes, duodenal feeding tubes or gastrostomy, while parenteral administrations includes, among others, subcutaneous, intravenous, intramuscular, intra-arterial, intradermal, intraosseous, intracerebral, and intrathecal injections. The administration method will often depend upon the (poly)peptide(s) and/or immunogenic compound(s) present in the composition, and the specific type of adrenal cancer to be treated and other active agents that may be contained in said composition. For example, the administration is preferably an intramuscular or an intradermal injection if the immunogenic compound is a nucleic acid as defined above, the oral/nasal administration being particularly preferred if said nucleic acid is cloned into a viral vector. Alternatively, the administration is preferably an intramuscular, an intradermal or an oral administration if the (poly)peptide and/or immunogenic compound is a (poly)peptide as defined above or if it is loaded in/on a nanoparticle as described herein. Yet, still alternatively, the administration is preferably an oral administration if the (poly)peptide and/or immunogenic compound is delivered in the form of a gut bacterium as defined above, notably if the gut bacterium is in the form of probiotics.

The (poly)peptides and/or immunogenic compounds as disclosed herein can further be encapsulated so as to facilitate their administration to the subject in need thereof. For example, those may be encapsulated into peptide nanocarriers (preferable if the immunogenic compound is a nucleic acid or a (poly)peptide), into virosomes (preferable if the immunogenic compound is a nucleic acid or a (poly)peptide), or into lipid-based carrier systems such as liposome-polycation-DNA complex (preferable if the immunogen is a nucleic acid or a (poly)peptide) (Trovato M, De Berardinis P. Novel antigen delivery systems. World J Virol. 2015 Aug 12;4(3):156-68; Saade F, Petrovsky N. Technologies for enhanced efficacy of DNA vaccines. Expert Rev Vaccines. 2012 Feb;11(2):189-209; Li et al., Peptide Vaccine: Progress and Challenges. Vaccines (Basel). 2014 Jul 2;2(3):515-36).

The medicament may be administered once or more than once, so as to achieve the desired effect. In a preferred embodiment, the medicament is administered repeatedly, at least twice, and preferably more than twice. This can be done over an extended period of time, such as weekly, every other week, monthly, yearly, or even several years after the first administration to ensure that the subject is properly immunized.

In an instance not encompassed by the wording of the claims, an (poly)peptide or an immunogenic compound as disclosed herein may be used for the preparation of a composition and/or of a pharmaceutical composition for preventing or treating an adrenal cancer in a subject in need thereof.

The term "adrenal cancer" (also referred to as "adrenal carcinoma"), as used herein, refers to cancers of the adrenal glands. Typically, an adrenal cancer is a cancer, which started (originated) in the adrenal gland. Accordingly, cancers, which started elsewhere but spread (metastasized) through the blood stream to the adrenal glands are usually not considered as "adrenal cancer". In general, it is understood that a "cancer" or "carcinoma" is a malignant condition. Accordingly, the term "adrenal cancer" does not include benign tumors.

The adrenal glands are small glands located on top of each kidney (suprarenal) and each adrenal gland has two parts, the cortex (outer part) and the medulla (inner part). Cancers of the cortex of the adrenal glands are also referred to as "adrenocortical" cancers, "adrenal cortical" cancers or adrenocortical carcinoma (all also referred to as "ACC"). Accordingly, the adrenal cancer to be ameliorated, reduced, prevented and/or treated (or the recurrence of which is to be reduced or prevented) may be an adrenocortical cancer (adrenocortical carcinoma). About two out of three adrenal cortical carcinomas cause symptoms by producing high levels of one of the adrenal cortex hormones, such as glucocorticoids, mineralocorticoids, and sex hormones. Examples of adrenocortical cancers include (the malignant forms of) aldosteronoma, adrenal tumors making excessive cortisol (resulting in Cushing's syndrome), virilizing adrenal tumors and feminizing adrenal tumors.

Preferably, adrenocortical carcinoma are unresectable locally advanced or metastatic (ENSAT/AJCC stage 3 = tumor has spread into nearby tissues or lymph nodes, or stage 4 = metastatic disease) adrenocortical carcinoma.

Cancers of the adrenal medulla include neuroblastomas of the adrenal medulla and pheochromocytomas. Pheochromocytomas (also referred to as "PH" or "PCC") are catecholamine-producing neuroendocrine tumors arising from chromaffin cells of the adrenal medulla. Accordingly, the primary site of pheochromocytomas is the adrenal medulla. The term "pheochromocytoma" does not include so-called "extra-adrenal pheochromocytomas", which are usually paragangliomas (not pheochromocytomas). Accordingly, the adrenal cancer to be ameliorated, reduced, prevented and/or treated may be a neuroblastoma of the adrenal medulla or a (malignant) pheochromocytoma. Paragangliomas, closely related to pheochromocytomas, are also encompassed within the present invention.

Pheochromocytomas and paragangliomas are two entities combined and referred to as malignant (defined as metastatic disease due to lack of other specific markers; metastatic defined as presence of chromaffin tissue in non-chromaffin organs) pheochromocytoma/paraganglioma (MPP) which constitutes a rare subgroup of endocrine tumors. MPP frequently spread to regional and distant lymph nodes, bones, liver, and lungs. As expected, patients with MPP have shorter overall survival (OS) durations than do patients with non-metastatic disease. Approximately 10% - 15% of pheochromocytomas and paragangliomas are malignant (Plouin PF, Amar L, Dekkers OM, Fassnacht M, Gimenez-Roqueplo AP, Lenders JW, Lussey-Lepoutre C, Steichen O; Guideline Working Group. Eur J Endocrinol. 2016 May;174(5):G1-G10. doi: 10.1530/EJE-16-0033.). The therapeutic strategy of MPP aims to control excessive catecholamine secretion and tumor burden, but no curative treatment is currently achievable. Preferably, pheochromocytomas and paragangliomas are malignant pheochromocytoma/paraganglioma (MPP).

In view of the function of the adrenal glands in hormone production, adrenal tumors are often classified as "functioning" and "non-functioning". Functioning adrenal cancers increase hormone production and produce symptoms related to the hormones that are overproduced. These may include abnormal weight gain or weight loss, hypertension, and anxiety. For example, functioning ACC of glucocorticoid producing cells may result in Cushing's syndrome, whose symptoms include rapid weight gain, rounding of the face, excessive sweating, easy bruising and excess facial or body hair growth in women. Functioning ACC of mineralocorticoid cells may result in Conn's syndrome (aldosteronism). Pheochromocytoma may result in high levels of circulating catecholamines, thereby inducing hypertension and anxiety. In particular when the adrenal cancer concerns cells producing sex hormones, women may see altered menstrual cycles and men may experience feminization, while children may experience early puberty. Non-functioning adrenal cancers fail to produce hormones and can produce pain from pressure on the abdominal organs, and sometimes a mass in the abdomen that can be felt with the fingers.

### BRIEF DESCRIPTION OF THE FIGURES

In the following a brief description of the appended figures will be given.
- Figure 1: shows the general protocol for the validation of the proof-of-concept of an antigen-based immunotherapy targeting IL13RA2.
- Figure 2:: shows a schematic view of the immunization scheme. d: day.

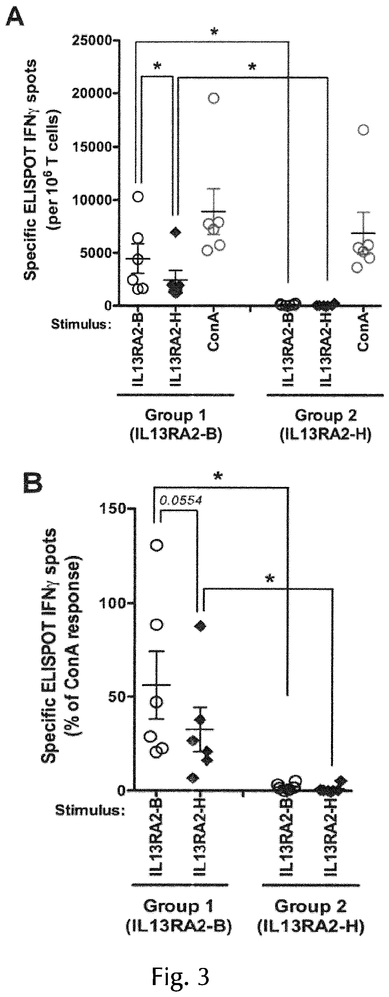

- Figure 3:: shows ELISPOT-IFNγ results for group 1 (IL13RA2-B) and group 2 (IL13RA2-A). The peptide used for vaccination (in between brackets under each group) and the stimulus used in the ELISPOT culture (X-axis) are indicated on the graphs. (A) Number of specific ELISPOT-IFNγ spots (medium condition subtracted). Each dot represents the average value for one individual/mouse from the corresponding condition quadruplicate. (B) For each individual, the level of specific ELISPOT-IFNγ response is compared to the ConA stimulation (value: 100%). Statistical analysis: paired t-test for intra-group comparison and unpaired t-test for inter-group comparison; * p<0.05.
- Figure 4:: shows the results of Example 3.
- Figure 5:: shows the results of Example 4.
- Figure 6:: shows for Example 6 ELISPOT results for HLA-A2 transgenic mice vaccinated with the peptide IL13R2A-L as indicated in the figure and cross-reactivity with the human corresponding peptide IL13RA2-H. For each group the normalized number of spot-forming cells (SFC) is shown.
- Figure 7: shows for Example 7 *in vitro* affinity for peptides IL13RA2-B and IL13RA2-L in comparison to the corresponding human peptide IL13RA2-H and to the comparative peptide 1A9V.
- Figure 8:: shows for Example 8 *in vitro* affinity for antigenic peptides IL13RA2-B and IL13RA2-L in comparison to the corresponding human IL13RA2 epitope IL13RA2-H.
- Figure 9:: shows for Example 8 *in vitro* affinity for antigenic peptides BIRC5-B1, BIRC5-B2 and BIRC5-B3 in comparison to the corresponding human BIRC5 epitope BIRC5-H.
- Figure 10:: shows for Example 8 *in vitro* affinity for (A) antigenic peptide FOXM1-B in comparison to the corresponding human FOXM1 epitope FOXM1-H and (B) antigenic peptide FOXM1-B2 in comparison to the corresponding human FOXM1 epitope FOXM1-H2.
- Figure 11:: shows for Example 9 ELISPOT results for mice vaccinated with the antigenic peptides as indicated in the figure (BIRC5-H, BIRC5-B1, BIRC5-B2, BIRC5-B3, FOXM1-H2, FOXM1-B2, IL13RA2-H, IL13RA2-B. For each group the normalized number of spot-forming cells (SFC) is shown. Each dot represents the average value for one individual/mouse.
- Figure 12:: shows for Example 10 ELISPOT results for HLA-A2 transgenic mice vaccinated with the antigenic peptide BIRC5-B1 as indicated in the figure and cross-reactivity with the human corresponding peptide BIRC5-H. For each group the normalized number of spot-forming cells (SFC) is shown.
- Figure 13:: shows for Example 11 ELISPOT results for HLA-A2 transgenic mice vaccinated with the antigenic peptide FOXM1-B2 as indicated in the figure and cross-reactivity with the human corresponding peptide FOXM1-H2. For each group the normalized number of spot-forming cells (SFC) is shown.
- Figure 14:: shows for Example 12 *in vitro* affinity for the antigenic peptides BIRC5-B1 in comparison to the corresponding human BIRC5 epitope BIRC5-H, to the comparative peptide 2M and to the positive control HIV.
- Figure 15:: shows for Example 14 the detection of IL13RA2-L, BIRC5-B1 and FOXM1-B2 peptide-specific CD8+ T cells detected in peripheral blood from HLA-A2 positive healthy donors.
- Figure 16:: shows for Example 14 the cytotoxic capacity of the of IL13RA2-L peptide-specific human T cells clones expanded *in vitro* by microbiome derived peptide stimulation. IL13RA2-L peptide-specific T cells have the ability to kill T2 cells loaded with bacterial peptide.

### EXAMPLES

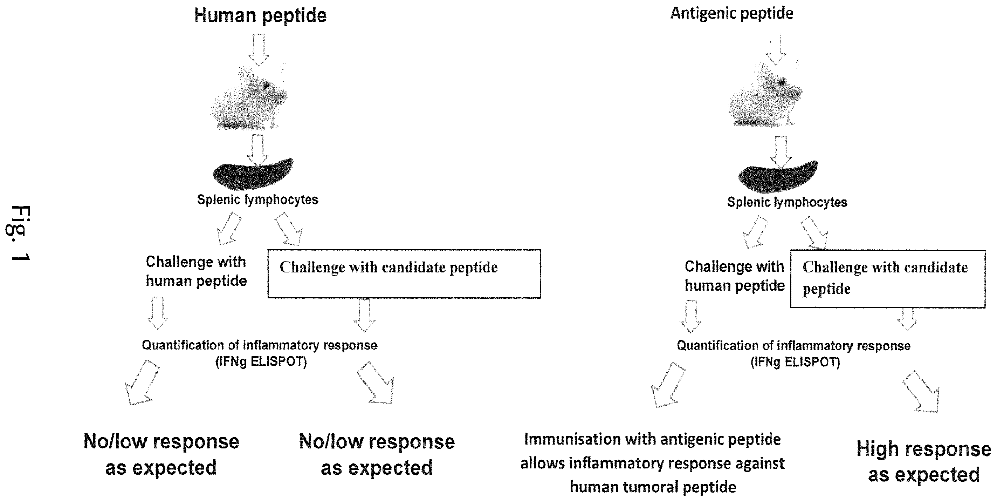

Examples 1 and 2 are both linked to the general protocol described in figure 1.

### EXAMPLE 1: Identification of a candidate (poly)peptide having superior affinity to the HLA-A*0201 allele.

This Example provides evidence that the (poly)peptide of sequence SEQ ID NO: 31 («FLPFGFILV» also referred herein as IL13RA2-B) has high affinity to the HLA-A*0201 allele, whereas the corresponding reference human peptide derived from IL13RA2 («WLPFGFILI», SEQ ID N° 263, also referred herein as IL13RA2-H) has low affinity.

### A. Materials and Methods

### A1. Measuring the affinity of the peptide to T2 cell line.

The experimental protocol is similar to the one that was validated for peptides presented by the HLA-A*0201 (Tourdot et al., A general strategy to enhance immunogenicity of low-affinity HLA-A2.1-associated peptides: implication in the identification of cryptic tumor epitopes. Eur J Immunol. 2000 Dec; 30(12):3411-21). Affinity measurement of the peptides is achieved with the human tumoral cell T2 which expresses the HLA-A*0201 molecule, but which is TAP1/2 negative and incapable of presenting endogenous peptides.

T2 cells (2.10⁵ cells per well) are incubated with decreasing concentrations of peptides from 100 µM to 0.1 µM in a AIMV medium supplemented with 100 ng/µl of human β2m at 37°C for 16 hours. Cells are then washed two times and marked with the anti-HLA-A2 antibody coupled to PE (clone BB7.2, BD Pharmagen).

The analysis is achieved by FACS (Guava Easy Cyte).

For each peptide concentration, the geometric mean of the labelling associated with the peptide of interest is substracted from background noise and reported as a percentage of the geometric mean of the HLA-A*0202 labelling obtained for the reference peptide HIV pol 589-597 at a concentration of 100µM. The relative affinity is then determined as follows: relative affinity = concentration of each peptide inducing 20% of expression of HLA - A * 0201/ concentration of the reference peptide inducing 20% of expression of HLA - A * 0201.

### A2. Solubilisation of peptides

Each peptide is solubilized by taking into account the amino acid composition. For peptides which do not include any Cystein, Methionin, or Tryptophane, the addition of DMSO is possible to up to 10% of the total volume. Other peptides are resuspended in water or PBS pH7.4.

### B. Results

For T2 Cells: Mean fluorescence intensity for variable peptidic concentrations: Regarding the couple IL13RA2 peptides (IL13RA2-H and IL13RA2-B), it appears that the human peptide does not bind to the HLA-A*0201 contrarily to the candidate peptide IL13RA2-B, which binds strongly to HLA-A*0201 : 112.03 vs 18.64 at 100 µM ; 40.77 vs 11.61 at 10 µM ; 12.18 vs 9.41 at 1 µM ; 9.9 vs 7.46 at 0.1 µM.

Also, IL13RA2-B at 4.4µM induces 20% of expression of the HLA-A*0201 (vs 100 µM for IL13RA2-H).

Similar results were obtained from a second distinct T2 cell clone.

### EXAMPLE 2: Vaccination on mice with the candidate (poly)peptide induces improved T cell responses in a ELISPOT-IFNγ assay.

### A. Materials and Methods

### A.1 Mouse model

The features of the model used in this project are shown in Table 6.

**Table 6. Model features.**

| | |
|---|---|
| Mouse Model | C57BL/6J B2m ^{tm1Unc}IAb^{-/-}Tg(HLA-DRA HLA-DRB1*0301)^{#Gjh} Tg(HLA-A/H2-D/B2M)^{1Bpe} |
| Acronym | β/A2/DR3 |
| Description | Immunocompetent, no mouse class I and class II MHC |
| Housing | SOPF conditions (ABSL3) |
| Number of mice | 24 adults (> 8 weeks of age) |

### A.2. Immunization scheme.

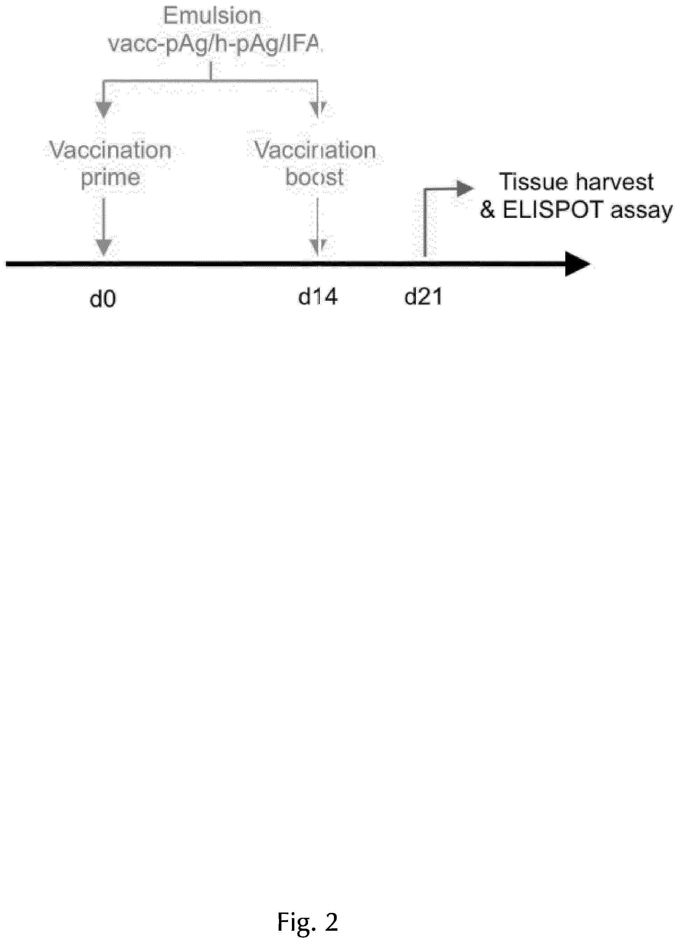

The immunization scheme is shown in figure 2. Briefly,14 β/A2/DR3 mice were assigned randomly (based on mouse sex and age) to two experimental groups, each immunized with a specific vaccination peptide (vacc-pAg) combined to a common helper peptide (h-pAg) (as outlined in Table 7 below). The vacc-pAg were compared in couples (group 1 vs. group 2). Thereby, both native and optimized versions of a single peptide were compared in each wave.

**Table 7. Experimental group composition. h-pAg: 'helper' peptide; vacc-pAg: vaccination peptide.The number of boost injections is indicated into brackets.**

| **Group** | **Peptide (vacc-pAg)** | **Helper (h-pAg)** | **Prime** | **Boost** | **Animal number** |
|---|---|---|---|---|---|
| 1 | IL13RA2-B (100µg) | HHD-DR3 (150µg) | + | +(1X) | 6 |
| 2 | IL13RA2-H (100µg) | HHD-DR3 (150µg) | + | +(1X) | 6 |

The peptides were provided as follows:
- couples of vacc-pAg: IL13RA2-H and IL13RA2-B; all produced and provided at a 4 mg/ml (4mM) concentration;
- h-pAg: HHD-DR3; provided lyophilized (50.6 mg; Eurogentec batch 1611166) and re-suspended in pure distilled water at a 10 mg / mL concentration;

The animals were immunized on day 0 (d0) with a prime injection, and on d14 with a boost injection. Each mouse was injected s.c. at tail base with 100 µL of an oil-based emulsion that contained :
- 100 µg of vacc-pAg (25 µL of 4 mg/mL stock per mouse);
- 150 µg of h-pAg (15 µL of 10 mg/mL stock per mouse);
- 10 µL of PBS to reach a total volume of 50 µL (per mouse);
- Incomplete Freund's Adjuvant (IFA) added at 1:1 (v:v) ratio (50 µL per mouse).

A separate emulsion was prepared for each vacc-pAg, as follows: IFA reagent was added to the vacc-pAg/h-pAg/PBS mixture in a 15 mL tube and mixed on vortex for repeated cycles of 1 min until forming a thick emulsion.

### A.3. Mouse analysis

Seven days after the boost injection (i.e. on d21), the animals were euthanized and the spleen was harvested. Splenocytes were prepared by mechanical disruption of the organ followed by 70 µm-filtering and Ficoll density gradient purification.

The splenocytes were immediately used in an ELISPOT-IFNγ assay (Table 8). Experimental conditions were repeated in quadruplets, using 2*10⁵ total splenocytes per well, and were cultured in presence of vacc-pAg (10 µM), Concanavalin A (ConA, 2.5 µg/mL) or medium-only to assess for their capacity to secrete IFNγ. The commercial ELISPOT-IFNγ kit (Diaclone Kit Mujrine IFNγ ELISpot) was used following the manufacturer's instructions, and the assay was performed after about 16h of incubation.

**Table 8. Setup of the ELISPOT-IFNγ assay.**

| **Group** | **Stimulus** | **Wells** | **Animal** | **Total** |
|---|---|---|---|---|
| 1 | IL13RA2-B (10µM) | 4 | 6 | 24 |
| | IL13RA2-H (10µM) | 4 | 6 | 24 |
| | ConA (2,5µg/ml) | 4 | 6 | 24 |
| | Medium | 4 | 6 | 24 |
| 2 | IL13RA2-B (10µM) | 4 | 6 | 24 |
| | IL13RA2-H (10µM) | 4 | 6 | 24 |
| | ConA (2,5µg/ml) | 4 | 6 | 24 |
| | Medium | 4 | 6 | 24 |

Spots were counted on a Grand ImmunoSpot^{®} S6 Ultimate UV Image Analyzer interfaced to the ImmunoSpot 5.4 software (CTL-Europe). Data plotting and statistical analysis were performed with the Prism-5 software (GraphPad Software Inc.).

The cell suspensions were also analyzed by flow cytometry, for T cell counts normalization. The monoclonal antibody cocktail (data not shown) was applied on the purified leucocytes in presence of Fc-block reagents targeting murine (1:10 diluted 'anti-mCD16/CD32 CF11 clone' - internal source) Fc receptors. Incubations were performed in 96-well plates, in the dark and at 4°C for 15-20 minutes. The cells were washed by centrifugation after staining to remove the excess of monoclonal antibody cocktail, and were re-suspended in PBS for data acquisition.

All data acquisitions were performed with an LSR-II Fortessa flow cytometer interfaced with the FACS-Diva software (BD Bioscience). The analysis of the data was performed using the FlowJo-9 software (TreeStar Inc.) using a gating strategy (not shown).

**Table 9. FACS panel EXP-1.**

| **Target** | **Label** | **Clone** | **Provider** | **Dilution** |
|---|---|---|---|---|
| mCD3εγ | FITC | 145-2C11 | Biolegend | 1/100 |
| mCD4 | PE | RM4-5 | Biolegend | 1/100 |
| mCD8α | APC | 53-6,7 | Biolegend | 1/100 |

### B. Results

A total of 14 β/A2/DR3 mice were used for this experiment (see Table 10). At time of sacrifice, the spleen T cell population was analysed by flow cytometry, showing that the large majority belonged to the CD4+ T cell subset.

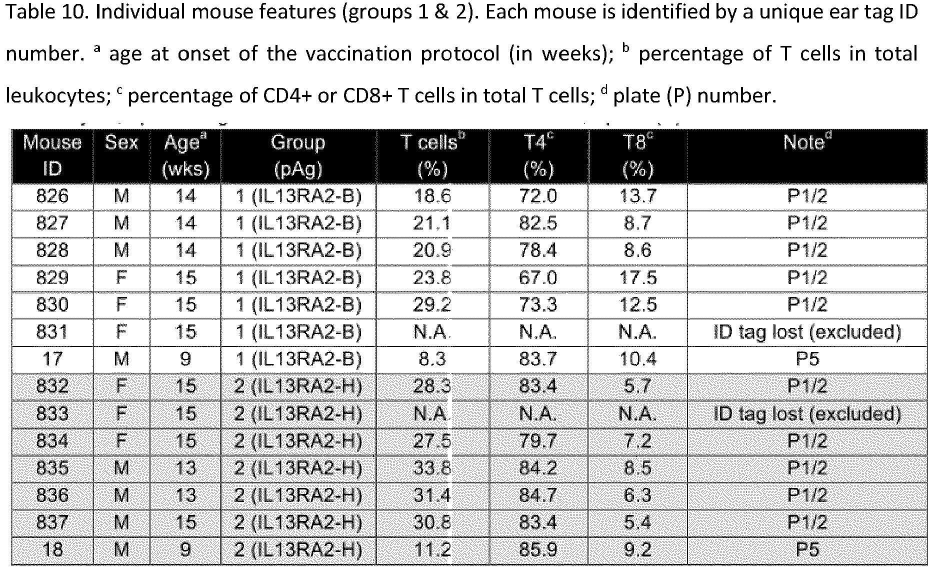

After plating and incubation with the appropriate stimuli, the IFNy-producing cells were revealed and counted. The data were then normalized as a number of specific spots (the average counts obtained in the 'medium only' condition being subtracted) per 10⁶ total T cells.

The individual average values (obtained from the quadruplicates) were next used to plot the group average values (see Figure 3A). As the functional capacity of T cells might vary from individual to individual, the data were also expressed as the percentage of the ConA response per individual (see Figure 3B).

Overall, vaccination with the IL13RA2-B pAg (candidate) peptide induced improved T cell responses in the ELISPOT-IFNγ assay, as compared to IL13RA2-H pA (reference human)-vaccinated animals (group 2). For group 1 (IL13RA2-B), ex vivo restimulation with the IL13RA2-B pAg promoted higher response than with the IL13RA2-H pAg. It was not the case for group 2 (IL13RA2-H). The percentage of ConA-induced response (mean +/- SEM) for each condition was as follows:
- Group 1 (IL13RA2-B) / IL13RA2-B pAg: 56.3% +/- 18.1
- Group 1 (IL13RA2-B) / IL13RA2-H pAg: 32.3% +/- 11.8
- Group 2 (IL13RA2-H) / IL13RA2-B pAg: 2.0% +/- 0.8
- Group 2 (IL13RA2-H) / IL13RA2-H pAg: 1.1% +/- 0.8

Accordingly, those results provide experimental evidence that antigen-based immunotherapy targeting IL13RA2 is able to improve T cell response *in vivo* and that the IL13RA2-B candidate peptide (SEQ ID NO: 31) is particularly efficient for that purpose.

### EXAMPLE 3: Candidate (poly)peptides having superior affinity to the HLA-A*0201 allele.

This Example provides further evidence that the (poly)peptide of sequence SEQ ID NO: 31 (« FLPFGFILV », also referred to herein as IL13RA2-B) has high affinity to the HLA-A*0201 allele, whereas the corresponding reference human peptide derived from IL13RA2 (« WLPFGFILI », SEQ ID N° 263, also referred to herein as IL13RA2-H) has low affinity. Moreover, this Example provides evidence that the (poly)peptide of sequence SEQ ID NO: 192 (« YLYTFLIST », also referred to herein as IL13RA2-B2) has high affinity to the HLA-A*0201 allele, whereas the corresponding reference human peptide derived from IL13RA2 (« CLYTFLIST », SEQ ID NO: 245, also referred to herein as IL13RA2-H2) has low affinity.

### A. Materials and Methods

### A1. Measuring the affinity of the peptide to T2 cell line.

The experimental protocol was similar to the one that was validated for peptides presented by the HLA-A*0201 (Tourdot et al., A general strategy to enhance immunogenicity of low-affinity HLA-A2.1-associated peptides: implication in the identification of cryptic tumor epitopes. Eur J Immunol. 2000 Dec; 30(12):3411-21). Affinity measurement of the peptides was achieved with the human tumor cell T2 which expresses the HLA-A*0201 molecule, but which is TAP1/2 negative and incapable of presenting endogenous peptides.

T2 cells (2.10⁵ cells per well) were incubated with decreasing conceontrations of peptides from 100 µM to 0.1 µM in a AIMV medium supplemented with 100 ng/µl of human β2m at 37°C for 16 hours. Cells were then washed two times and marked with the anti-HLA-A2 antibody coupled to PE (clone BB7.2, BD Pharmagen).

The analysis was achieved by FACS (Guava Easy Cyte).

For each peptide concentration, the geometric mean of the labelling associated with the peptide of interest was subtracted from background noise and reported as a percentage of the geometric mean of the HLA-A*0202 labelling obtained for the reference peptide HIV pol 589-597 at a concentration of 100µM. The relative affinity is then determined as follows: relative affinity = concentration of each peptide inducing 20% of expression of HLA - A * 0201/ concentration of the reference peptide inducing 20% of expression of HLA - A * 0201.

### A2. Solubilisation of peptides

Each peptide was solubilized by taking into account the amino acid composition. For peptides which do not include any Cystein, Methionin, or Tryptophane, the addition of DMSO is possible to up to 10% of the total volume. Other peptides are resuspended in water or PBS pH7.4.

### B. Results

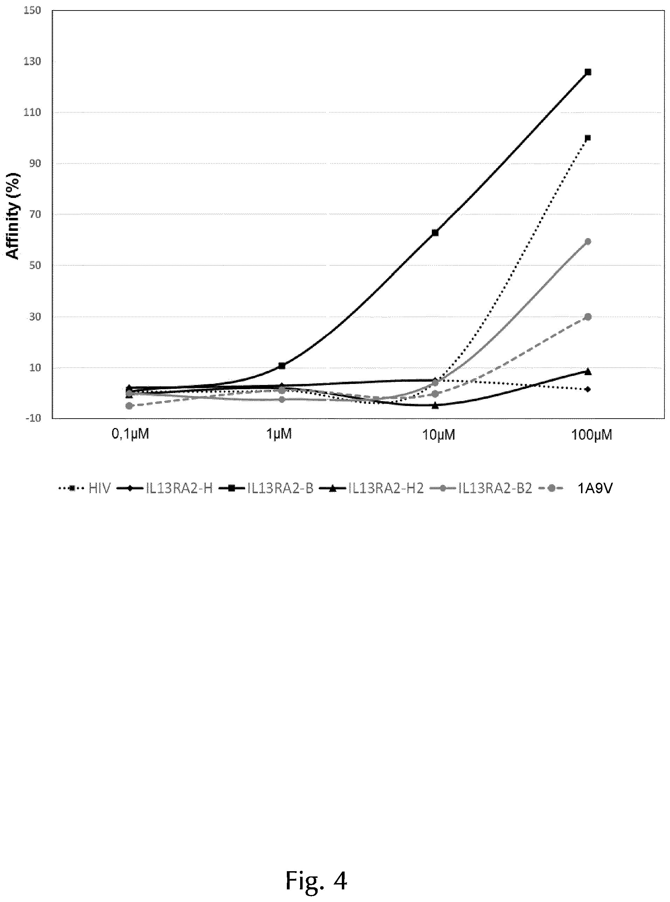

Results are shown in Figure 4. Regarding the two couples of IL13RA2 peptides ((i) IL13RA2-H and IL13RA2-B, and (ii) IL13RA2-H2 and IL13RA2-B2), the human peptides do not bind to or show much lower affinity to HLA-A*0201, whereas the candidate peptides IL13RA2-B and IL13RA2-B2, bind strongly to HLA-A*0201. Moreover, both candidate peptides bind to HLA-A*0201 with higher affinity than the peptide "1A9V" (as described by Eguchi Junichi et al., 2006, Identification of interleukin-13 receptor alpha 2 peptide analogues capable of inducing improved antiglioma CTL responses. Cancer Research 66(11): 5883-5891). Reference peptide HIV pol 589-597 ("HIV") served as positive control.

Similar results were obtained from a second distinct T2 cell clone.

### EXAMPLE 4: Candidate (poly)peptide provides in vitro cytotoxicity against cells expressing IL13RA2.

This Example provides evidence that the (poly)peptide of sequence SEQ ID NO: 31 («FLPFGFILV», also referred to herein as IL13RA2-B) provides *in vitro* cytotoxicity against U87 cells, which express IL13RA2. In contrast, the corresponding reference human peptide derived from IL13RA2 («WLPFGFILI», SEQ ID N° 263, also referred to herein as IL13RA2-H) does not provide *in vitro* cytotoxicity against U87 cells.

### Methods:

Briefly, CD8 T cells from mice immunized with IL13RA2-H or IL13RA2-B were used. These cells were obtained after sorting of splenocyte from immunized mice and were placed on top of U87 cells (expressing IL13RA2).

In more detail, CD3⁺ T cells were purified from splenocytes of HHD mice immunized with IL13RA2-H (WLPFGFILI, SEQ ID N° 263) or IL13RA2-B (FLPFGFILV). To this end, B6 β2m^{ko} HHD/DR3 mice were injected s.c. at tail base with 100 µL of an oil-based emulsion containing vaccination peptide plus helper peptide plus CFA (complete Freund's adjuvant), at day 0 and day 14. On d21, i.e. seven days after the boost injection, the animals were euthanized and the spleen was harvested. Splenocytes were prepared by mechanical disruption of the organ. CD3+ purification was performed using the mouse total T cells isolation kit from Miltenyi biotec using the recommended procedure. Efficient purification of cells and viability was validated by cytometry using appropriate marker for viability, CD8, CD4, CD3, and CD45.

U87-MG cells were seeded at 6 × 10⁵ cells/well in flat-bottomed 24-well culture plates and incubated for 24 h at 37°C in DMEM (Dulbecco's Modified Eagle Medium) containing 10% of FCS (fetal calf serum) and antibiotics. After 24 hours, culture media were removed and replaced with media containing purified T CD3+ cells. The following ratios of T cells vs. U87-MG cells were used: 1/0.5, 1/1 and 1/5.

72 hours after co-culture of U87-MG cells and CD3+ T cells, all cells from the wells were harvested and specific U87-MG cell death was evaluated after immunostaining of CD45 negative cells with DAPI and fluorescent annexin V followed by cytometry analysis.

### Results:

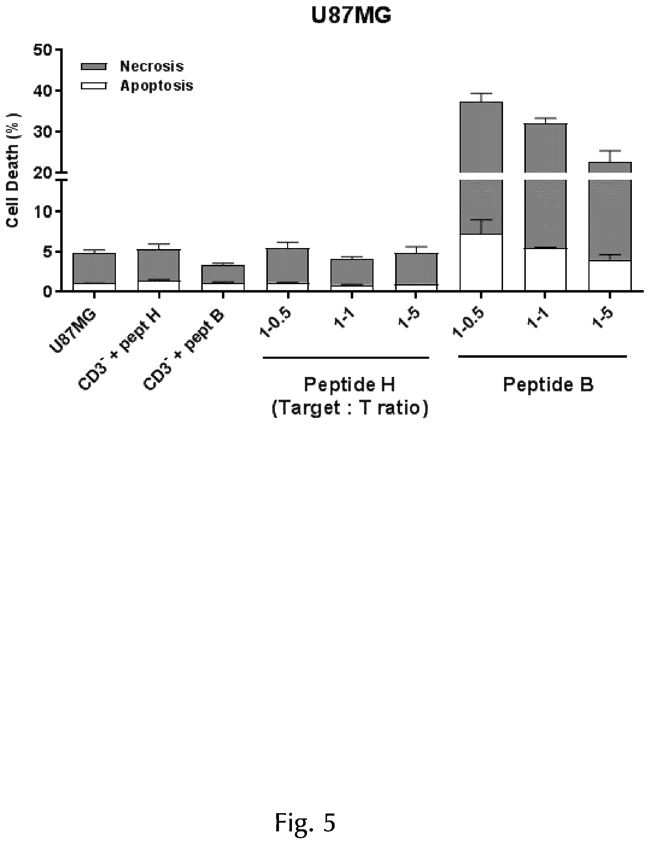

Results are shown in Fig. 5. In general, U87 cell lysis was observed after treatment with IL13RA2-B, but not with IL13RA2-H.

### EXAMPLE 5: Candidate (poly)peptide has superior affinity to the HLA-A*0201 allele.

This Example provides evidence that the (poly)peptide of sequence SEQ ID NO: 31 (« FLPFGFILV », also referred to herein as IL13RA2-B) has higher affinity to the HLA-A*0201 allele than other sequence variants of the corresponding reference human peptide derived from IL13RA2 (« WLPFGFILI », SEQ ID N° 263, also referred to herein as IL13RA2-H). In this experiment, the (poly)peptide of sequence SEQ ID NO: 31 was compared to
- the peptide "1A9V" (SEQ ID NO: 282), as described by Eguchi Junichi et al., 2006, Identification of interleukin-13 receptor alpha 2 peptide analogues capable of inducing improved antiglioma CTL responses. Cancer Research 66(11): 5883-5891, in which the tryptophan at position 1 of SEQ ID N° 263 was substituted by alanine (1A) and the isoleucine at position 9 of SEQ ID N° 263 was substituted by valine (9V);
- peptide "119A", wherein the tryptophan at position 1 of SEQ ID N° 263 was substituted by isoleucine (1I) and the isoleucine at position 9 of SEQ ID N° 263 was substituted by alanine (9A); and
- peptide "1F9M", wherein the tryptophan at position 1 of SEQ ID N° 263 was substituted by phenylalanine (1F) and the isoleucine at position 9 of SEQ ID N° 263 was substituted by methionine (9M).

### A. Materials and Methods

The experimental protocol, materials and methods correspond to those outlined in Example 3, with the only difference that the above mentioned (poly)peptides were used.

### B. Results

The following *in vitro* binding affinities were obtained:

**(Table 11)**

| **Peptide** | **In vitro binding affinity** |
|---|---|
| IL13RA2-B (SEQ ID NO: 31) | 0.49 |
| 1A9V | 3.06 |
| 1I9A | 2.22 |
| 1F9M | 2.62 |

Accordingly, the (poly)peptide (IL13RA2-B (SEQ ID NO: 31)) showed considerably higher binding affinity to HLA-A*0201 than all other peptides tested, whereas the peptide "1A9V", as described by Eguchi Junichi et al., 2006, Identification of interleukin-13 receptor alpha 2 peptide analogues capable of inducing improved antiglioma CTL responses. Cancer Research 66(11): 5883-5891, showed the lowest affinity of the peptides tested.

### EXAMPLE 6: Immunogenicity of IL13R2A-L in HLA-A2 transgenic mice and cross-reactivity with the corresponding human peptide.

### A. Materials and Methods

The (poly)peptide IL13RA2-L (SEQ ID NO: 279) and the corresponding human reference peptide IL13RA2-H (SEQ ID NO: 263) were tested in distinct groups of male and female HHD DR3 mice expressing human HLA-A2 and HLA-DR3 MHC and lacking the murine H-2 class I and class II MHCs. Groups of 5 mice (male and female) were subcutaneously injected on days 0 and 14 with 100 µg of IL13RA2-L (SEQ ID NO: 279) or IL13RA2-H (SEQ ID NO: 263), 150 µg of helper peptide (DR3) and IFA. On day 21, the mice were euthanized and splenocytes were prepared and stimulated *in vitro* with IL13RA2-L or the human corresponding peptide IL13RA2-H to assess their capacity to secrete IFN-y as assessed by ELISpot. Concanavalin A (ConA) was used as a positive control.

### B. Results

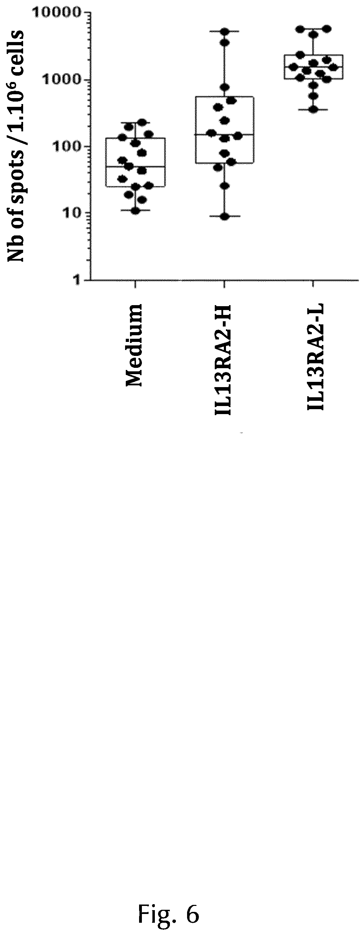

The number of spot forming cells (SFC) (normalized to the number of CD8 cells) are depicted in Figure 6. Results are shown for mice immunized with IL13RA2-L. The results show that immunisation of mice with IL13RA2-L allows to induce T-cells that are able to react strongly after challenge with either IL13RA2-L or the human corresponding peptide. Thus, IL13RA2-L is strongly immunogenic and is able to drive an effective immune response against the corresponding human peptide. As expected, the immunisation of mice with the human corresponding peptide IL13RA2-H does not induce an immune response after challenge with either IL13RA2-L or the human corresponding peptide IL13RA2-H (data not shown).

These results were confirmed in HHD DR1 mice expressing human HLA-A2 and HLA-DR1 MHC and lacking the murine H-2 class I and class II MHCs (groups of 5 mice).

### EXAMPLE 7: IL13RA2-L has superior affinity to the HLA-A*0201 allele.

This Example provides evidence that the (poly)peptide as set forth in SEQ ID NO: 279 (also referred to herein as IL13RA2-L) has a similarly high affinity to the HLA-A*0201 allele as the (poly)peptide as set forth in SEQ ID NO: 31 (FLPFGFILV, also referred to herein as IL13RA2-B) - and a higher affinity than the corresponding reference human peptide derived from IL13RA2 (IL13RA2-H, WLPFGFILI, SEQ ID NO: 263) and other sequence variants thereof. In this experiment, the (poly)peptide of sequence SEQ ID NO: 279 (IL13RA2-L) was compared to
- the comparative peptide "1A9V", as described by Eguchi Junichi et al., 2006, Identification of interleukin-13 receptor alpha 2 peptide analogues capable of inducing improved antiglioma CTL responses. Cancer Research 66(11): 5883-5891, in which the tryptophan at position 1 of SEQ ID NO: 263 was substituted by alanine (1A) and the isoleucine at position 9 of SEQ ID NO: 263 was substituted by valine (9V);
- the (poly)peptide as set forth in SEQ ID NO: 31 (IL13RA2-B);
- the corresponding reference human peptide IL13RA2-H (SEQ ID NO: 263); and
- a positive control (HIV).

### A. Materials and Methods

The experimental protocol, materials and methods correspond to those outlined in Example 2, with the only difference that the above mentioned (poly)peptides were used.

### B. Results

The following *in vitro* binding affinities were obtained:

**(Table 12)**

| **Peptide** | **SEQ ID NO** | **Concentration of peptide that induces 20% of HLA-A2 expression (µM)** | **In vitro binding affinity** |
|---|---|---|---|
| IL13RA2-H | 263 | ND | ND |
| IL13RA2-B | 31 | 2,9 | 0,3 |
| IL13RA2-L | 279 | 3,2 | 0,3 |
| 1A9V | 282 | 36,5 | 3,6 |

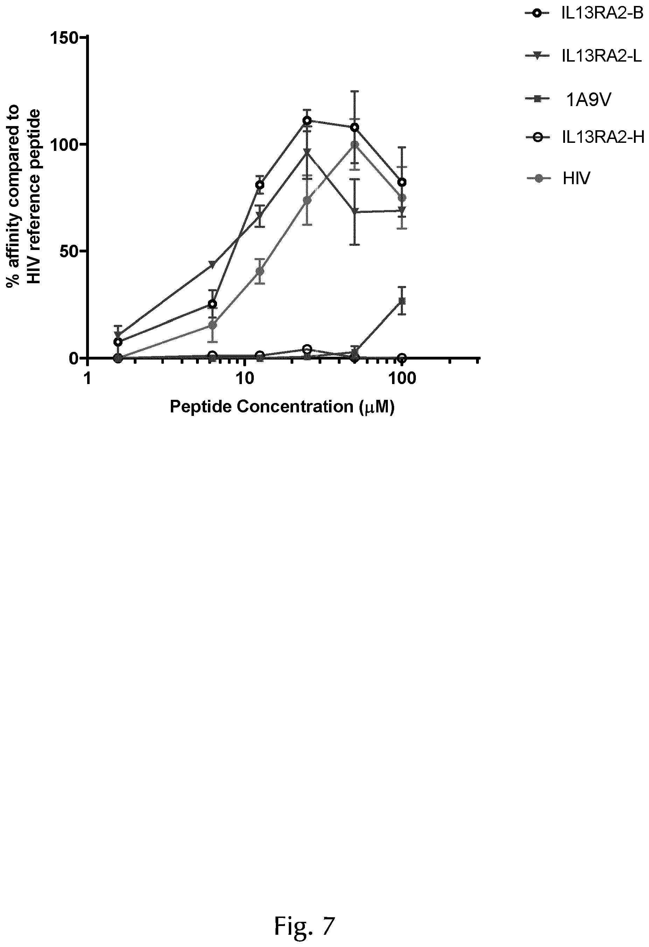

Accordingly, the (poly)peptides IL13RA2-B( SEQ ID NO: 31) and IL13RA2-L (SEQ ID NO: 279) showed considerably higher binding affinity to HLA-A*0201 than the corresponding human peptide (IL13RA2-H) and the comparative peptide "1A9V", as described by Eguchi Junichi et al., 2006, Identification of interleukin-13 receptor alpha 2 peptide analogues capable of inducing improved antiglioma CTL responses. Cancer Research 66(11): 5883-5891. In particular, the (poly)peptide IL13RA2-L (SEQ ID NO: 279) shows a strong binding affinity to HLA-A*0201, namely, 69% of maximum HIV pol 589-597 binding activity at 100 µM; 96% at 25µM and 43% at 6.25 µM. Results are also shown in Figure 7.

### EXAMPLE 8: IL13RA2, BIRC5 and FOXM1 peptides have superior affinity to the HLA-A*0201 allele.

Binding affinity of various selected sequence variants of epitopes and of the corresponding fragments of human tumor antigens (human reference peptides) to the HLA-A*0201 allele was confirmed *in vitro.* Namely, the antigenic peptides of sequence SEQ ID NO: 289 («FMLGEFLKL» also referred herein as BIRC5-B1); SEQ ID NO: 287 («YTLGEFLYI» also referred herein as BIRC5-B2); and SEQ ID NO: 288 («GLLGEFLQI» also referred herein as BIRC5-B3) were compared to the corresponding reference human peptide derived from BIRC5 («LTLGEFLKL», SEQ ID NO: 286, also referred herein as BIRC5-H). Moreover, the antigenic peptides of sequence SEQ ID NO: 314 («RLSSYLVEI» also referred herein as FOXM1-B) and sequence SEQ ID NO: 312 («LMDLSTTEV» also referred herein as FOXM1-B2) were compared to the corresponding reference human peptides derived from FOXM1 («RVSSYLVPI», SEQ ID NO: 294, also referred herein as FOXM1-H and «LMDLSTTPL», SEQ ID NO: 293, also referred herein as FOXM1-H2, respectively). Moreover, the antigenic peptides of sequence SEQ ID NO: 31 («FLPFGFILV» also referred herein as IL13RA2-B) and sequence SEQ ID NO: 279 («FLPFGFILPV» also referred herein as IL13RA2-L) were compared to the corresponding reference human peptide derived from IL13RA2 («WLPFGFILI», SEQ ID NO: 263, also referred herein as IL13RA2-H).

### A. Materials and Methods

### A1. Measuring the affinity of the peptide to T2 cell line.

The experimental protocol is similar to the one that was validated for peptides presented by the HLA-A*0201 (Tourdot et al., A general strategy to enhance immunogenicity of low-affinity HLA-A2.1-associated peptides: implication in the identification of cryptic tumor epitopes. Eur J Immunol. 2000 Dec; 30(12):3411-21). Affinity measurement of the peptides is achieved with the human tumoral cell T2 which expresses the HLA-A*0201 molecule, but which is TAP1/2 negative and incapable of presenting endogenous peptides.

T2 cells (2.10⁵ cells per well) are incubated with decreasing concentrations of peptides from 100 µM to 1.5625 µM in a AIMV medium supplemented with 100 ng/µl of human β2m at 37°C for 16 hours. Cells are then washed two times and marked with the anti-HLA-A2 antibody coupled to PE (clone BB7.2, BD Pharmagen).

The analysis is achieved by FACS (Guava Easy Cyte).

For each peptide concentration, the geometric mean of the labelling associated with the peptide of interest is substracted from background noise and reported as a percentage of the geometric mean of the HLA-A*0202 labelling obtained for the reference peptide HIV pol 589-597 at a concentration of 100µM. The relative affinity is then determined as follows: relative affinity = concentration of each peptide inducing 20% of expression of HLA - A * 0201/ concentration of the reference peptide inducing 20% of expression of HLA - A * 0201.

### A2. Solubilisation of peptides

Each peptide is solubilized by taking into account the amino acid composition. For peptides which do not include any Cystein, Methionin, or Tryptophane, the addition of DMSO is possible to up to 10% of the total volume. Other peptides are resuspended in water or PBS pH7.4.

### B. Results

The mean relative fluorescence intensity values (data are normalized to the mean fluorescence of HIV peptide, i.e. a value of 100 is equal to the best binding observed with HIV peptide) of T2 cells obtained for the various concentrations of each peptide are shown in Table 13 below:

**Table 13.**

| **Peptide** | | **Peptide concentration (µM)** | | | | | |
|---|---|---|---|---|---|---|---|
| **Name** | **SEQ ID NO.** | **100** | **50** | **25** | **6,25** | **3,125** | **1,5625** |
| BIRC5-H | 286 | 35,6 | 18,9 | 9,8 | 10,8 | 1,4 | 1,7 |
| BIRC5-B1 | 289 | 117,0 | 77,1 | 61,7 | 36,1 | 22,3 | 1,9 |
| BIRC5-B2 | 287 | 58,0 | 54,4 | 29,6 | 9,0 | 6,6 | nd |
| BIRC5-B3 | 288 | 35,0 | 29,8 | 20,9 | nd | 8,9 | 9,4 |
| FOXM 1-H | 294 | 83,8 | 30,7 | 10,5 | 0,0 | 0,0 | 0,0 |
| FOXM 1-B | 314 | 47,5 | 21,3 | 7,6 | 0,0 | 0,0 | 0,0 |
| FOXM1-H2 | 293 | 77,6 | 62,5 | 65,4 | 19,7 | 0,9 | 5,3 |
| FOXM1-B2 | 312 | 105,0 | 91,5 | 98,2 | 33,5 | 12,7 | 7,2 |
| IL13RA2-H | 263 | 26,5 | 14,2 | 11,2 | 9,6 | 3,7 | 3,0 |
| IL13RA2-B | 31 | 128,4 | 112,7 | 86,5 | 40,8 | 15,7 | 14,8 |
| IL13RA2-L | 279 | 107,7 | 85,5 | 77,3 | 30,4 | 19,8 | 13,3 |

Table 14 below summarizes for each tested peptide the concentration required to induce 20% of HLA-A2 expression and the *in vitro* binding affinity.

**Table 14. ND - not determined**

| **Peptide** | **Concentration of peptide that induces 20% of HLA-A2 expression (µM)** | **In vitro binding affinity** |
|---|---|---|
| BIRC5-H | 53,0 | 16,1 |
| BIRC5-B1 | 2,7 | 0,8 |
| BIRC5-B2 | 14,7 | 4,5 |
| BIRC5-B3 | 22,9 | 7,0 |
| FOXM1-H | 37,6 | 3,7 |
| FOXM1-B | 46,7 | 4,6 |
| FOXM1-H2 | 12,6 | 2,5 |
| FOXM1-B2 | 6,7 | 1,3 |
| IL13RA2-H | ND | ND |
| IL13RA2-B | 2,9 | 0,3 |
| IL13RA2-L | 3,2 | 0,3 |

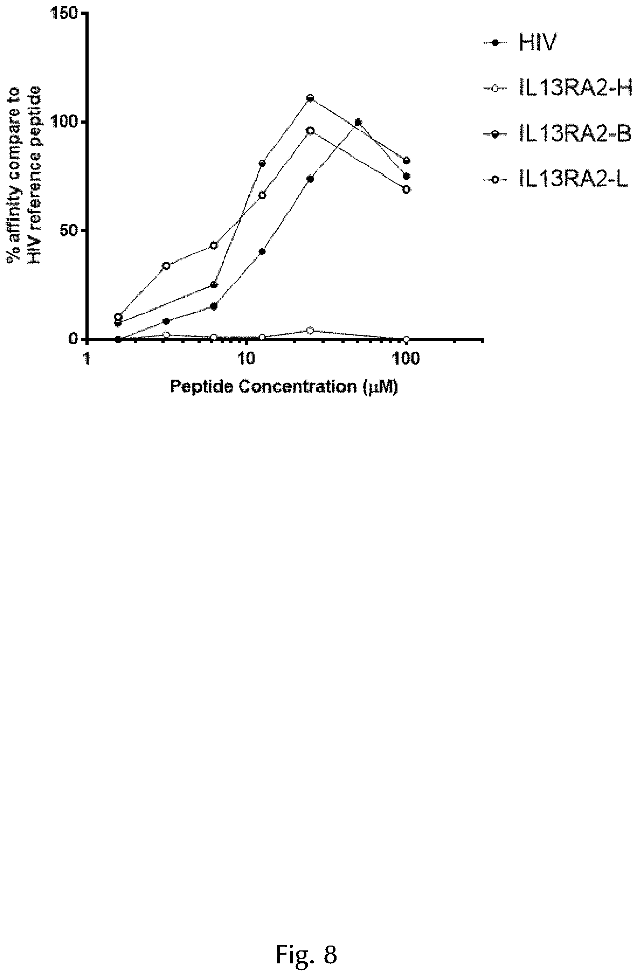

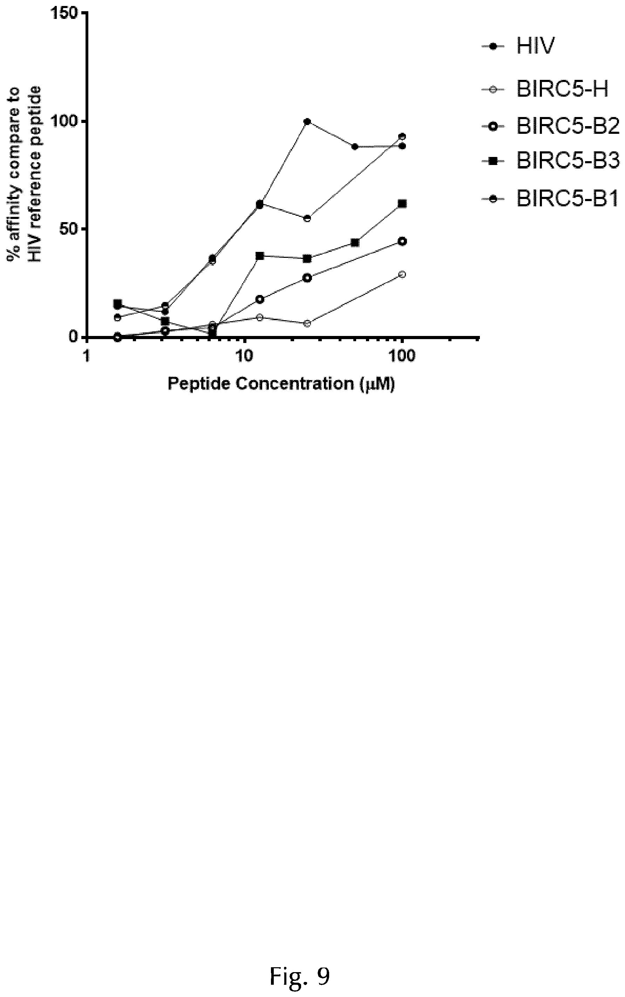

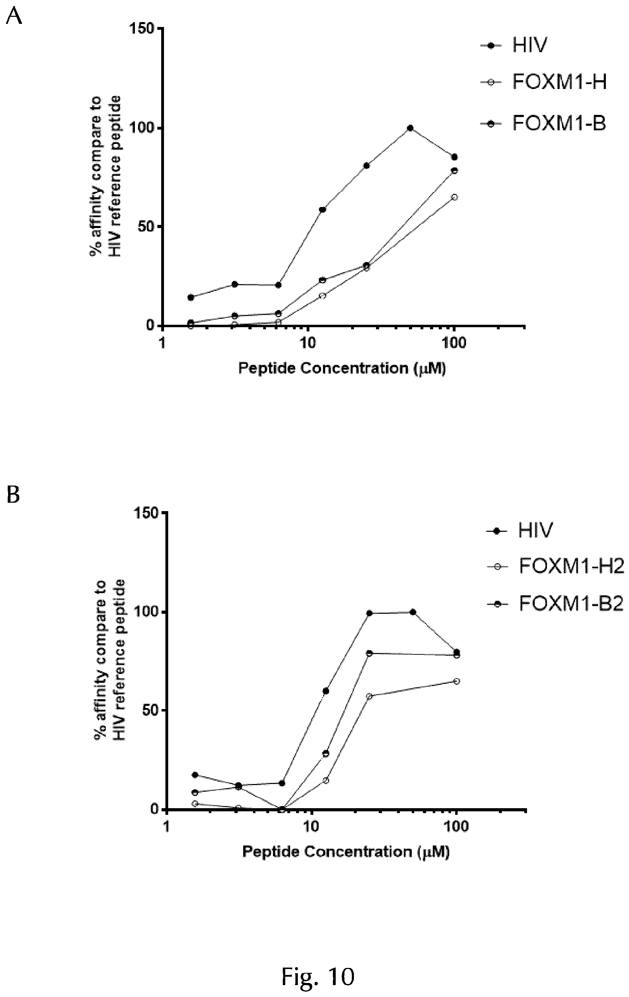

In addition, Figures 8 - 10 illustrate the results for selected examples, namely for antigenic peptides IL13RA2-B and IL13RA2-L in comparison to the corresponding human IL13RA2 fragment IL13RA2-H (Fig. 8), for antigenic peptides BIRC5-B1, BIRC5-B2 and BIRC5-B3 in comparison to the corresponding human BIRC5 fragment BIRC5-H (Fig. 9), and for antigenic peptide FOXM1-B in comparison to the corresponding human FOXM1 fragment FOXM1-H (Fig. 10A) and antigenic peptide FOXM1-B2 in comparison to the corresponding human FOXM1 fragment FOXM1-H2 (Fig. 10B).

In summary, the results show that the exemplified IL13RA2, BIRC5 and FOXM1 peptides show at least similar binding affinity to HLA-A*0201 as the corresponding human tumor antigen fragments. In most cases, the binding affinity observed for the antigenic peptides disclosed herein was stronger than that of the corresponding human epitopes. Without being bound to any theory it is assumed that such a strong binding affinity of the antigenic peptides disclosed herein reflects their ability to raise an immune response (i.e., their immunogenicity).

### EXAMPLE 9: Vaccination of mice with antigenic peptides as disclosed herein induces improved T cell responses in ELISPOT-IFNγ assay.

### A. Materials and Methods

### A.1 Mouse model

The immunization scheme is the same as described above and shown in Figure 2. Briefly, HLA-A2 humanized mice (HLA-A2 (CB6F1-Tg(HLA-A*0201/H2-K^{b})A*0201) were assigned randomly (based on mouse sex and age) to experimental groups, wherein each group was immunized with a specific vaccination peptide (vacc-pAg) combined to a common helper peptide (h-pAg T13L; sequence: TPPAYRPPNAPIL; SEQ ID NO: 280; Bhasin M, Singh H, Raghava GP (2003) MHCBN: a comprehensive database of MHC binding and non-binding peptides. Bioinformatics 19: 665-666) (as outlined in Table 15 below). The vacc-pAg were compared in couples (group 1 vs. group 2, group 1 vs. group 3; group 1 vs. group 4; group 5 vs. group 6; group 7 vs. group 8). Thereby, both native and optimized versions of a single peptide were compared in each wave.

**Table 15. Experimental group composition. h-pAg: 'helper' peptide; vacc-pAg: vaccination peptide. The number of boost injections is indicated into brackets.**

| Group | Peptide (vacc-pAg) | Helper (h-Ag) | Prime | Boost | Animal Number |
|---|---|---|---|---|---|
| 1 | BIRC5-H (100µg) | T13L (150 µg) | + | +(1X) | 5 |
| 2 | BIRC5-B1 (100µg) | T13L (150 µg) | + | +(1X) | 5 |
| 3 | BIRC5-B2 (100µg) | T13L (150 µg) | + | +(1X) | 5 |
| 4 | BIRC5-B3 (100µg) | T13L (150 µg) | + | +(1X) | 5 |
| 5 | FOXM1-H2 (100µg) | T13L (150 µg) | + | +(1X) | 5 |
| 6 | FOXM 1-B2 (100µg) | T13L (150 µg) | + | +(1X) | 5 |
| 7 | IL13RA2-H (100µg) | T13L (150 µg) | + | +(1X) | 5 |
| 8 | IL13RA2-B (100µg) | T13L (150 µg) | + | +(1X) | 5 |

The peptides were provided as follows:
- vacc-pAg: BIRC5-H, BIRC5-B1, BIRC5-B2, BIRC5-B3, FOXM1-H2, FOXM1-B2, IL13RA2-H and IL13RA2-B; all produced and provided at a 4 mg/ml (4mM) concentration;
- h-pAg: T13L; Eurogentec batch 1713334 re-suspended in pure distilled water at a 10 mg / mL concentration

The animals were immunized on day 0 (d0) with a prime injection, and on d14 with a boost injection. Each mouse was injected s.c. at tail base with 100 µL of an oil-based emulsion that contained :
- 100 µg of vacc-pAg (25 µL of 4 mg/mL stock per mouse);
- 150 µg of h-pAg (15 µL of 10 mg/mL stock per mouse);
- 10 µL of PBS to reach a total volume of 50 µL (per mouse);
- Incomplete Freund's Adjuvant (IFA) added at 1:1 (v:v) ratio (50 µL per mouse).

A separate emulsion was prepared for each vacc-pAg, as follows: IFA reagent was added to the vacc-pAg/h-pAg/PBS mixture in a 15 mL tube and mixed on vortex for repeated cycles of 1 min until forming a thick emulsion.

### A.2 Analysis

Seven days after the boost injection (i.e. on d21), the animals were euthanized and the spleen was harvested. Splenocytes were prepared by mechanical disruption of the organ followed by 70 µm-filtering and Ficoll density gradient purification.

The splenocytes were immediately used in an ELISPOT-IFNγ assay (Table 16). Experimental conditions were repeated in triplicates, using 2*10⁵ total splenocytes per well, and were cultured in presence of vacc-pAg (10 µM), lonomycin (0.1µM) plus PMA (1µM) or medium-only to assess for their capacity to secrete IFNy. The commercial ELISPOT-IFNγ kit (Diaclone Kit Mujrine IFNy ELISpot) was used following the manufacturer's instructions, and the assay was performed after about 19h of incubation.

**Table 16. Setup of the ELISPOT-IFNγ assay.**

| Group | Stimulus | Wells | Animal | Total |
|---|---|---|---|---|
| 1 | BIRC5-H (10µM) | 3 | 5 | 15 |
| | lonomycin (0,1µM) PMA 1µM) | 3 | 5 | 15 |
| | Medium | 3 | 5 | 15 |
| 2 | BIRC5-B1 (10µM) | 3 | 5 | 15 |
| | lonomycin (0,1µM) PMA 1µM) | 3 | 5 | 15 |
| | Medium | 3 | 5 | 15 |
| 3 | BIRC5-B2 (10µM) | 3 | 5 | 15 |
| | lonomycin (0,1µM) PMA 1µM) | 3 | 5 | 15 |
| | Medium | 3 | 5 | 15 |
| 4 | BIRC5-B3 (10µM) | 3 | 5 | 15 |
| | lonomycin (0,1µM) PMA 1µM) | 3 | 5 | 15 |
| | Medium | 3 | 5 | 15 |
| 5 | FOXM1-H2 (10µM) | 3 | 5 | 15 |
| | lonomycin (0,1µM) PMA 1µM) | 3 | 5 | 15 |
| | Medium | 3 | 5 | 15 |
| 6 | FOXM1-B2 (10µM) | 3 | 5 | 15 |
| | lonomycin (0,1µM) PMA 1µM) | 3 | 5 | 15 |
| | Medium | 3 | 5 | 15 |
| 7 | IL13RA2-H (10µM) | 3 | 5 | 15 |
| | lonomycin (0,1µM) PMA 1µM) | 3 | 5 | 15 |
| | Medium | 3 | 5 | 15 |
| 8 | IL13RA2-B (10µM) | 3 | 5 | 15 |
| | lonomycin (0,1µM) PMA 1µM) | 3 | 5 | 15 |
| | Medium | 3 | 5 | 15 |

Spots were counted on a CTL ELISpot reader. Data plotting and statistical analysis were performed with the Prism-5 software (GraphPad Software Inc.).

### B. Results

A total of 40 HLA-A2 (CB6F1-Tg(HLA-A*0201/H2-K^{b})A*0201) mice were used for these experiment. All mice were aged of 6 to 9 weeks at the experiment starting date. Both males and females were used in the study. Animals have been housed in groups of 5 per cage at maximum. At time of sacrifice, the spleen T cell population was analysed by flow cytometry, showing that the large majority belonged to the CD4+ T cell subset.

After plating and incubation with the appropriate stimuli, the IFNy-producing cells were revealed and counted. The data were then normalized as a number of specific spots (the average counts obtained in the 'medium only' condition being subtracted) per 50*10³ total T cells.

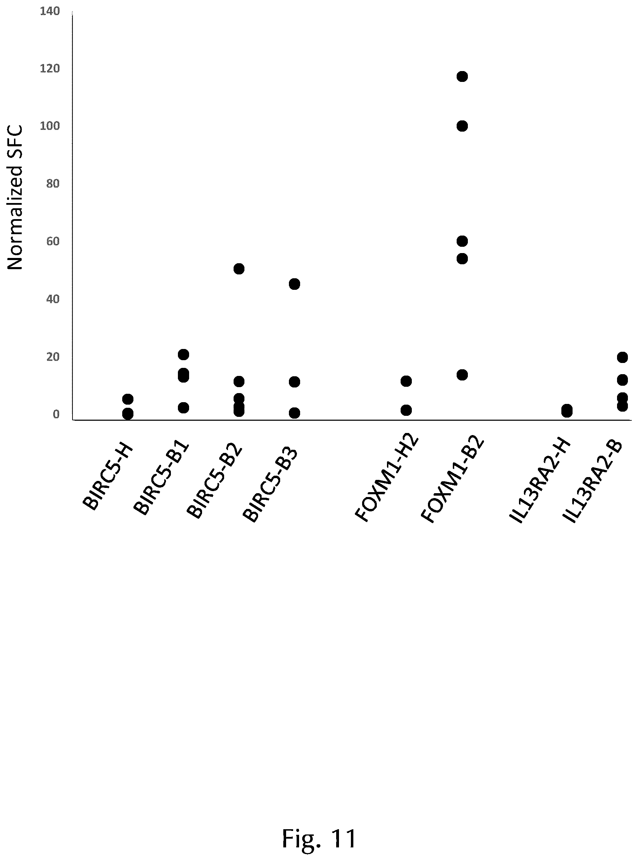

The individual average values (obtained from the triplicates) were next used to plot the group average values. As the functional capacity of T cells might vary from individual to individual, the data were also expressed as the percentage of the ionomycin plus PMA response per individual (see Figure 11).

Overall, vaccination with the antigenic peptides BIRC5-B1, BIRC5-B2, BIRC5-B3, FOXM1-B2 and IL13RA2-B induced improved T cell responses in the ELISPOT-IFNγ assay, as compared to the respective human reference epitopes (BIRC5-H, FOXM1-H2 and IL13RA2-H).

### EXAMPLE 10: Immunogenicity of BIRC5-B1 in HLA-A2 transgenic mice and cross-reactivity with the corresponding human peptide.

### A. Materials and Methods

The antigenic peptide BIRC5-B1 (SEQ ID NO: 289) and the corresponding human peptide BIRC5-H (SEQ ID NO: 286) were tested in distinct groups of male and female HHD DR3 mice expressing human HLA-A2 and HLA-DR3 MHC and lacking the murine H-2 class I and class II MHCs. Groups of 5 mice (male and female) were subcutaneously injected on days 0 and 14 with 100 µg of BIRC5-B1 or BIRC5-H, 150 µg of helper peptide (DR3) and IFA. On day 21, the mice were euthanized and splenocytes were prepared and stimulated in vitro with BIRC5-B1 or the human peptide BIRC5-H to assess their capacity to secrete IFN-y as assessed by ELISpot. ConA was used as a positive control.

### B. Results

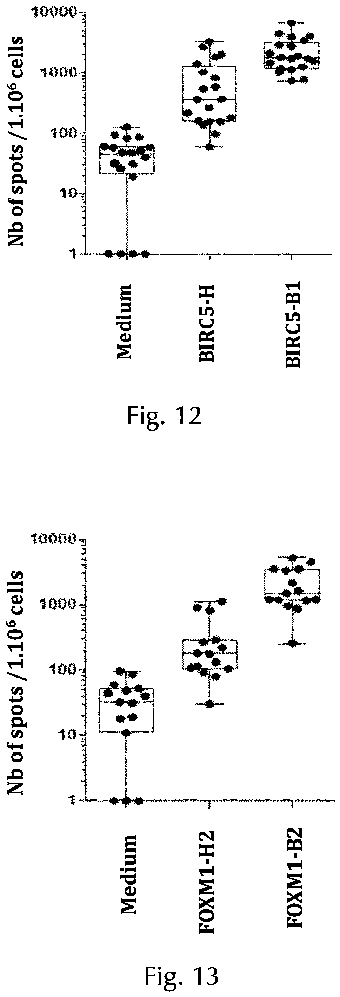

The number of SFC (normalized to the number of CD8 cells) are depicted in Figure 12. Results are shown for mice immunized with BIRC5-B1. The results show that immunisation of mice with BIRC5-B1 allows to induce T-cells that are able to react strongly after challenge with either BIRC5-B1 or the human corresponding peptide BIRC5-H. Thus, BIRC5-B1 is strongly immunogenic and is able to drive an effective immune response against human corresponding peptide. Immunisation of mice with the human corresponding peptide BIRC5-H does not induce any immune response against BIRC5-B1 or the human corresponding peptide (data not shown).

These results were confirmed in HHD DR1 mice expressing human HLA-A2 and HLA-DR1 MHC and lacking the murine H-2 class I and class II MHCs (groups of 5 mice).

### EXAMPLE 11: Immunogenicity of FOXM1-B2 in HLA-A2 transgenic mice and cross-reactivity with the corresponding human peptide.

### A. Materials and Methods

The antigenic peptide FOXM1-B2 (SEQ ID NO: 312) and the corresponding human peptide FOXM1-H2 (SEQ ID NO: 293) were tested in distinct groups of male and female HHD DR3 mice expressing human HLA-A2 and HLA-DR3 MHC and lacking the murine H-2 class I and class II MHCs. Groups of 5 mice (male and female) were subcutaneously injected on days 0 and 14 with 100 µg of FOXM1-B2 or FOXM1-H2, 150 µg of helper peptide (DR3) and IFA. On day 21, the mice were euthanized and splenocytes were prepared and stimulated *in vitro* with FOXM1-B2 or the human corresponding peptide FOXM1-H2 to assess their capacity to secrete IFN-y as assessed by ELISpot. ConA was used as a positive control.

### B. Results

The number of SFC (normalized to the number of CD8 cells) are depicted in Figure 13. Results are shown for mice immunized with FOXM1-B2. The results show that immunisation of mice with FOXM1-B2 allows to induce T-cells that are able to react strongly after challenge with either FOXM1-B2 or human corresponding peptide. Thus, FOXM1-B2 is strongly immunogenic and is able to drive an effective immune response against human corresponding peptide FOXM1-H2. Immunisation of mice with the human corresponding peptide FOXM1-H2 does not induce immune response against FOXM1-B2 or the human corresponding peptide (data not shown).

These results were confirmed in HHD DR1 mice expressing human HLA-A2 and HLA-DR1 MHC and lacking the murine H-2 class I and class II MHCs (groups of 5 mice).

Altogether, these immunogenicity studies described in Examples 6, 10 and 11 performed in HHD DR3 and HHD DR1 mice showed that the three antigenic peptides: IL13RA2-L, BIRC5-B1 and FOXM1-B2, induced strong immune responses. Cross-reactivity of the T cells generated against IL13RA2-L, BIRC5-B1 and FOXM1-B2 for the corresponding human peptides was shown in HHD DR3 and HHD DR1 mice.

Accordingly, those results provide experimental evidence that antigen-based immunotherapy is able to improve T cell response *in vivo* and that the antigenic peptides disclosed herein are particularly efficient for that purpose.

### EXAMPLE 12: BIRC5-B1 has superior affinity to the HLA-A*0201 allele.

This Example provides evidence that the peptide disclosed herein as set forth in SEQ ID NO: 289 (also referred to herein as BIRC5-B1) has a higher affinity than the corresponding reference human peptide derived from BIRC5 (BIRC5-H, SEQ ID NO: 286) and a comparative sequence variant thereof ("2M"; SEQ ID NO: 330). In this experiment, the peptide of sequence SEQ ID NO: 289 (BIRC5-B1) was compared to
- the peptide "2M" (LMLGEFLKL; SEQ ID NO: 330), in which the threonine at position 2 of SEQ ID NO: 593 was substituted by methionine (2M);
- the corresponding reference human peptide BIRC5-H (SEQ ID NO: 286); and
- a positive control (HIV).

### A. Materials and Methods

The experimental protocol, materials and methods correspond to those outlined in Example 8, with the only difference that the above mentioned (poly)peptides were used.

### B. Results

The following *in vitro* binding affinities were obtained:

**(Table 17)**

| **Peptide** | **SEQ ID NO** | **Concentration of peptide that induces 20% of HLA-A2 expression (µM)** | **In vitro binding affinity** |
|---|---|---|---|
| BIRC5-H | 286 | 95,9 | 112,82 |
| BIRC5-B1 | 289 | 1,24 | 1,46 |
| 2M | 330 | 2,87 | 3,38 |
| HIV | | 0,85 | 1,00 |

**(Table 18)**

| **Peptide** | | **Peptide concentration (µM)** | | | |
|---|---|---|---|---|---|
| **Name** | **SEQ ID NO.** | **100** | **10** | **1** | **0,1** |
| HIV | | 100 | 84,725 | 22,14 | 2,405 |
| BIRC5-H | 286 | 20,545 | 3,515 | 0 | 0 |
| BIRC5-B | 289 | 101,845 | 65,06 | 17,42 | 1,07 |
| 2M | 330 | 75,22 | 48,465 | 8,37 | 0,76 |

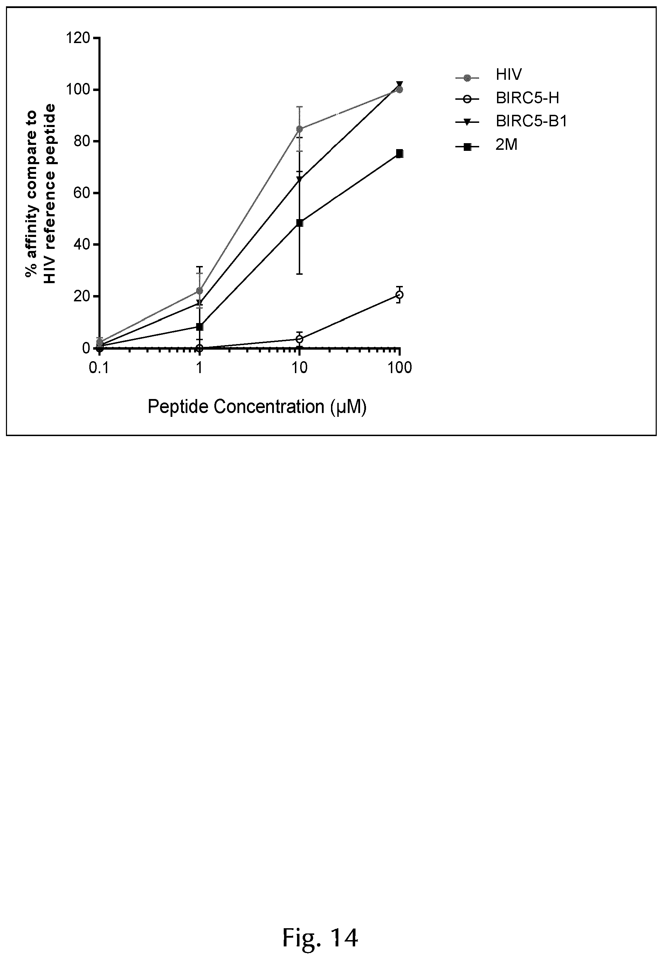

In summary, the antigenic peptide BIRC5-B1 (SEQ ID NO: 289) showed considerably higher *in vitro* binding affinity to HLA-A*0201 than the corresponding human epitope (BIRC5-H) and the comparative peptide "2M". Results are also shown in Figure 14.

### EXAMPLE 13: Candidate (poly)peptides has superior affinity to the HLA-A*0201 allele.

This Example provides evidence that the (poly)peptides of sequence SEQ ID NO: 31 (« FLPFGFILV », also referred to herein as IL13RA2-B), SEQ ID NO: 279 (« FLPFGFILPV », also referred to herein as IL13RA2-L), SEQ ID NO: 64 (« FMPFGFILV », also referred to herein as IL13RA2-B2), SEQ ID NO: 336 (« FMPFGFILGV », also referred to herein as IL13RA2-B3), SEQ ID NO: 212 (« YMPFGFILV », also referred to herein as IL13RA2-B4), SEQ ID NO: 178 (« YLPFGFILV », also referred to herein as IL13RA2-B5) and SEQ ID NO: 335 (« FMPFGFILPI », also referred to herein as IL13RA2-B6) have higher affinity to the HLA-A*0201 allele than other sequence variants of the corresponding reference human peptide derived from IL13RA2 (« WLPFGFILI », SEQ ID N° 263, also referred to herein as IL13RA2-H). Synthetic peptide "1A9V" (SEQ ID NO: 282), as described above and by Eguchi Junichi et al., 2006 was used as comparative peptide.

### A. Materials and Methods

The experimental protocol, materials and methods correspond to those outlined in Example 3, with the only difference that the above mentioned (poly)peptides were used.

### B. Results

The following *in vitro* binding affinities were obtained:

**(Table 19)**

| **Peptide** | **SEQ ID NO** | **Concentration of peptide that induces 20% of HLA-A2 expression (µM)** | **In vitro binding affinity** |
|---|---|---|---|
| IL13RA2-B | 31 | 1,9 | 2,48 |
| IL13RA2-L | 279 | 0,4 | 0,53 |
| IL13RA2-H | 263 | 62,3 | 78,77 |
| 1A9V | 282 | 20,9 | 27,68 |
| IL13RA2-B2 | 64 | 4,5 | 5,97 |
| IL13RA2-B3 | 336 | 3,3 | 4,39 |
| IL13RA2-B4 | 212 | 4,5 | 5,24 |
| IL13RA2-B5 | 178 | 1,5 | 1,76 |
| IL13RA2-B6 | 335 | 5,9 | 6,86 |
| HIV | | | 1 |

Accordingly, the (poly)peptides IL13RA2-B (SEQ ID NO: 31), IL13RA2-L (SEQ ID NO: 279), IL13RA2-B2 (SEQ ID NO: 64), IL13RA2-B3 (SEQ ID NO: 336), IL13RA2-B4 (SEQ ID NO: 212), IL13RA2-B5 (SEQ ID NO: 178) and IL13RA2-B6 (SEQ ID NO: 335) showed considerably higher binding affinity to HLA-A*0201 than the corresponding reference human peptide or the peptide variant "1A9V" described by Eguchi Junichi et al., 2006.

### EXAMPLE 14: Identification of IL13RA2-L, BIRC5-B1 and FOXM1-B2 peptide-specific CD8 T cells in humans and ex vivo cytotoxic effects of IL13RA2-L peptide specific CD8 human T cells.

Multiple investigations support the notion of presence of a repertoire of specific T cells against microbial peptides. The number of microbial specific T-cells against peptides is expected to be low, but sufficient to be re-activated by a vaccine challenge.

To identify, amplify and functionally characterize circulating IL13RA2-L, BIRC5-B1 and FOXM1-B2 peptide-specific T cells in humans, an *in vitro* amplification protocol has been developed in order to detect T cells specific for each antigenic peptide and investigate their cytotoxic capacity.

### 3.1 Identification of antigenic peptide-specific CD8 T cells in human

Peripheral blood mononuclear cells (PBMC) from several HLA-A*02 healthy donors were subjected to multiple rounds of *in vitro* stimulation with cells presenting IL13RA2-L, BIRC5-B1 and FOXM1-B2 peptides in HLA-A2 dependent context (as peptide loaded T2 cells). Identification and quantification of T cells specific clones were performed using peptide MHC multimers (pMHC). pMHC multimers are recombinantly produced and coupled with fluorescent labels. Binding of pMHC multimers to T cells allows identification and sorting by flow cytometry of specific T cells harboring TCRs able to specifically recognize selected pMHC complexes.

*In vitro* amplification method and specific pMHC multimers have been used for identification of IL13RA2-L-, BIRC5-B1- and FOXM1-B2-specific T cells. pMHC multimers were generated for all the bacteria peptides and their respective human counterpart. PBMCs from several HLA-A*02 healthy donors (up to 19 donors) were collected, enriched after CD137 and CD8 selection and subjected to multiple rounds of in vitro amplification with bacteria peptides loaded T2 cells to increase the number of specific T cell clones. Detection of bacteria and human peptide-specific CD8 T cells using cytometry analysis with the fluorescent multimer was performed on enriched CD8 T cell populations.

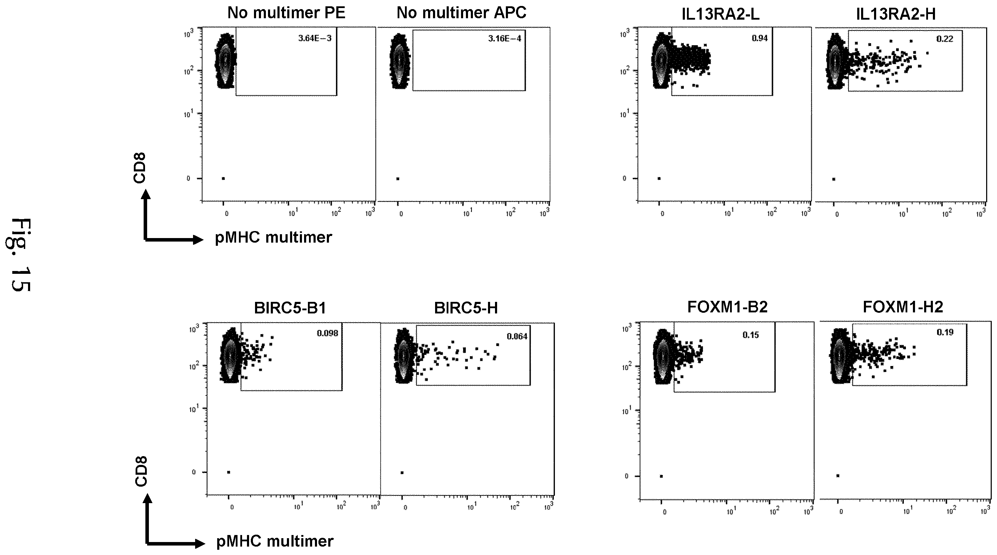

Figure 15 exemplifies results obtained with one HLA-A2 healthy donor. For this donor, cell amplification allows detection of IL13RA2-L specific cells (0.94%), BIRC5-B1 specific cells (0.098%) and FOXM1-B2 specific cells (0.15%). We also detect IL13RA2-H specific cells (0.22%), BIRC5-H1 specific cells (0.064%) and FOXM1-H2 specific cells (0.19%) CD8 T cells.

These results demonstrate the presence of CD8 T cells in the blood of healthy HLA-A2 donors that can recognize the microbiome-derived peptides, and importantly also the human counterpart peptides.

### 3.2 Antigenic peptide-specific CD8 T cytotoxicity functions

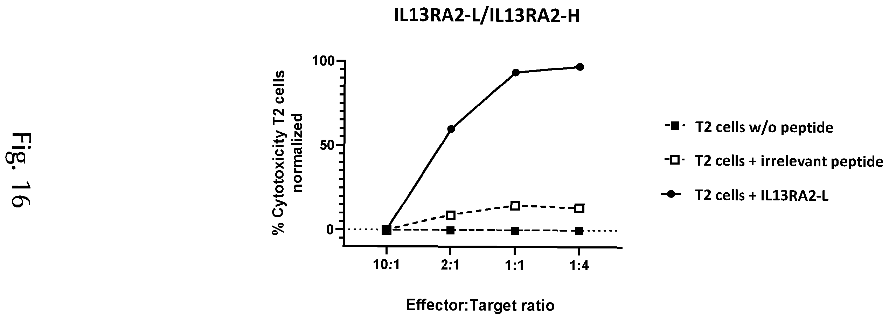

CD8+ T cells expanded per above were used to perform cytotoxic assays in presence of different ratios of target and effector cells to assess their cytotoxic capacity, using flow cytometry readout. Target cells were T2 cell lines loaded with bacterial peptide. Negative control was T2 cells unloaded and T2 cells loaded with irrelevant peptide. As shown in Figure 16, IL13RA2-L peptide-specific human T cell clones expanded *in vitro* have the capacity to kill T2 cells loaded with the bacteria peptide, IL13RA2-L.

Overall, these results demonstrate the presence of T cell clones in healthy volunteers able to recognize microbial peptide and to kill target with microbial peptides and human counterparts. These data are particularly encouraging as T cell clones have been obtained in healthy donors, therefore we could expect that specific T cell clones could be efficiently amplified in patients exposed to the immunization by antigenic peptides as disclosed herein.

## Claims

1. A combination of
(i) a peptide consisting of an amino acid sequence as set forth in SEQ ID NO: 279; and
(ii) a peptide consisting of an amino acid sequence as set forth in SEQ ID NO: 289;
for use in prevention and/or treatment of an adrenal cancer.

2. A combination of
(i) an immunogenic compound comprising a peptide as defined in claim 1 (i); and
(ii) an immunogenic compound comprising a peptide as defined in claim 1 (ii);
for use in prevention and/or treatment of an adrenal cancer.

3. A combination of
(i) a nanoparticle loaded with a peptide as defined in claim 1 (i); and
(ii) a nanoparticle loaded with a peptide as defined in claim 1 (ii));
for use in prevention and/or treatment of an adrenal cancer.

4. A combination of
(i) a cell loaded with a peptide as defined in claim 1 (i); and
(ii) a cell loaded with a peptide as defined in claim 1 (ii));
for use in prevention and/or treatment of an adrenal cancer.

5. A pharmaceutical composition comprising
- a peptide as defined in claim 1(i); and,
- a peptide as defined in claim 1(ii);
and, optionally, one or more pharmaceutically acceptable excipients
for use in prevention and/or treatment of an adrenal cancer.

6. A pharmaceutical composition comprising
- the immunogenic compound as defined in claim 2(i); and,
- the immunogenic compound as defined in claim 2(ii);
and, optionally, one or more pharmaceutically acceptable excipients
for use in prevention and/or treatment of an adrenal cancer.

7. A pharmaceutical composition comprising
- the nanoparticle as defined in claim 3(i); and,
- the nanoparticle as defined in claim 3(ii);
and, optionally, one or more pharmaceutically acceptable excipients
for use in prevention and/or treatment of an adrenal cancer.

8. A pharmaceutical composition comprising
- the cell as defined in claim 4(i); and,
- the cell as defined in claim 4(ii);
and, optionally, one or more pharmaceutically acceptable excipients
for use in prevention and/or treatment of an adrenal cancer.

9. The pharmaceutical composition for use according to claim 5, wherein the composition further comprises a (poly)peptide comprising an amino acid sequence as set forth in SEQ ID NO: 312.

10. The pharmaceutical composition for use according to any one of claims 5 and 9, wherein the pharmaceutical composition further comprises the peptide consisting of an amino acid sequence as set forth in SEQ ID NO: 281.

11. The combination of peptides, for use according to claim 1, the combination of immunogenic compounds for use according to claim 2, the combination of nanoparticles for use according to claim 3, the combination of cells for use according to claim 4, the pharmaceutical composition for use according to any one of claims 5-10, wherein the adrenal cancer is malignant pheochromocytoma/paraganglioma (MPP) or an adrenocortical carcinoma (ACC).

## Patentansprüche

1. Kombination aus
(i) einem Peptid, das aus einer Aminosäuresequenz besteht, wie in der SEQ ID NR: 279 dargelegt; und
(ii) einem Peptid, das aus einer Aminosäuresequenz besteht, wie in der SEQ ID NR: 289 dargelegt;
zur Verwendung bei der Prävention und/oder Behandlung von Nebennierenkrebs.

2. Kombination aus
(i) einer immunogenen Verbindung, die ein Peptid nach Anspruch 1 umfasst (i); und
(ii) einer immunogenen Verbindung, die ein Peptid nach Anspruch 1 umfasst (ii);
zur Verwendung bei der Prävention und/oder Behandlung von Nebennierenkrebs.

3. Kombination aus
(i) einem Nanopartikel, das mit einem Peptid nach Anspruch 1 geladen ist (i); und
(ii) einem Nanopartikel, das mit einem Peptid nach Anspruch 1 geladen ist (ii);
zur Verwendung bei der Prävention und/oder Behandlung von Nebennierenkrebs.

4. Kombination aus
(i) einer Zelle, die mit einem Peptid nach Anspruch 1 geladen ist (i); und
(ii) einer Zelle, die mit einem Peptid nach Anspruch 1 geladen ist (ii);
zur Verwendung bei der Prävention und/oder Behandlung von Nebennierenkrebs.

5. Pharmazeutische Zusammensetzung, umfassend
- ein Peptid nach Anspruch 1 (i); und
- ein Peptid nach Anspruch 1 (ii);
und wahlweise einen oder mehrere pharmazeutisch annehmbare Hilfsstoffe zur Verwendung bei der Prävention und/oder Behandlung von Nebennierenkrebs.

6. Pharmazeutische Zusammensetzung, umfassend
- die immunogene Verbindung nach Anspruch 2 (i); und
- die immunogene Verbindung nach Anspruch 2 (ii);
und wahlweise einen oder mehrere pharmazeutisch annehmbare Hilfsstoffe zur Verwendung bei der Prävention und/oder Behandlung von Nebennierenkrebs.

7. Pharmazeutische Zusammensetzung, umfassend
- das Nanopartikel nach Anspruch 3 (i); und
- das Nanopartikel nach Anspruch 3 (ii);
und wahlweise einen oder mehrere pharmazeutisch annehmbare Hilfsstoffe zur Verwendung bei der Prävention und/oder Behandlung von Nebennierenkrebs.

8. Pharmazeutische Zusammensetzung, umfassend
- die Zelle nach Anspruch 4 (i); und
- die Zelle nach Anspruch 4 (ii);
und wahlweise einen oder mehrere pharmazeutisch annehmbare Hilfsstoffe zur Verwendung bei der Prävention und/oder Behandlung von Nebennierenkrebs.

9. Pharmazeutische Zusammensetzung zur Verwendung nach Anspruch 5, wobei die Zusammensetzung ferner ein (Poly)peptid umfasst, das eine Aminosäuresequenz umfasst, wie in der SEQ ID NR: 312 dargelegt.

10. Pharmazeutische Zusammensetzung zur Verwendung nach einem der Ansprüche 5 und 9, wobei die pharmazeutische Zusammensetzung ferner das Peptid umfasst, das aus der Aminosäuresequenz besteht, wie in der SEQ ID NR: 281 dargelegt.

11. Kombination aus Peptiden zur Verwendung nach Anspruch 1, Kombination aus immunogenen Verbindungen zur Verwendung nach Anspruch 2, Kombination aus Nanopartikeln zur Verwendung nach Anspruch 3, Kombination aus Zellen zur Verwendung nach Anspruch 4, pharmazeutische Zusammensetzung zur Verwendung nach einem der Ansprüche 5 bis 10, wobei der Nebennierenkrebs malignes Phäochromozytom/Paragangliom (MPP) oder ein adrenokortikales Karzinom (ACC) ist.

## Revendications

1. Une combinaison de
• un peptide consistant en une séquence d'acides aminés telle que définie dans SEQ ID NO : 279 ; et
• un peptide consistant en une séquence d'acides aminés telle que définie dans SEQ ID NO : 289 ;
pour utilisation dans la prévention et/ou le traitement d'un cancer des glandes surrénales.

2. Une combinaison de
• un composé immunogène comprenant un peptide tel que défini dans la revendication 1 (i) ; et
• un composé immunogène comprenant un peptide tel que défini dans la revendication 1 (ii);
pour utilisation dans la prévention et/ou le traitement d'un cancer des glandes surrénales.

3. Une combinaison de
• une nanoparticule chargée d'un peptide tel que défini dans la revendication 1 (i) ; et
• une nanoparticule chargée d'un peptide tel que défini dans la revendication 1 (ii) ;
pour utilisation dans la prévention et/ou le traitement d'un cancer des glandes surrénales.

4. Une combinaison de
• une cellule chargée d'un peptide tel que défini dans la revendication 1 (i) ; et
• une cellule chargée d'un peptide tel que défini dans la revendication 1 (ii)) ;
pour utilisation dans la prévention et/ou le traitement d'un cancer des glandes surrénales.

5. Composition pharmaceutique comprenant
• un peptide tel que défini dans la revendication 1 (i) ; et
• un peptide tel que défini dans la revendication 1 (ii) ;
et, facultativement, un ou plusieurs excipients pharmaceutiquement acceptables,
pour utilisation dans la prévention et/ou le traitement d'un cancer des glandes surrénales.

6. Composition pharmaceutique comprenant
• le composé immunogène tel que défini dans la revendication 2(i) ; et
• le composé immunogène tel que défini dans la revendication 2(ii) ;
et, facultativement, un ou plusieurs excipients pharmaceutiquement acceptables,
pour utilisation dans la prévention et/ou le traitement d'un cancer des glandes surrénales.

7. Composition pharmaceutique comprenant
• la nanoparticule telle que définie dans la revendication 3(i) ; et
• la nanoparticule telle que définie dans la revendication 3(ii) ;
et, facultativement, un ou plusieurs excipients pharmaceutiquement acceptables pour utilisation dans la prévention et/ou le traitement d'un cancer des glandes surrénales.

8. Composition pharmaceutique comprenant
• la cellule telle que définie dans la revendication 4(i) ; et
• la cellule telle que définie dans la revendication 4(ii) ;
et, facultativement, un ou plusieurs excipients pharmaceutiquement acceptables pour utilisation dans la prévention et/ou le traitement d'un cancer des glandes surrénales.

9. Composition pharmaceutique pour son utilisation selon la revendication 5, dans laquelle la composition comprend en outre un (poly)peptide comprenant une séquence d'acides aminés telle que définie dans SEQ ID NO : 312.

10. Composition pharmaceutique pour son utilisation selon l'une quelconque des revendications 5 et 9, dans laquelle la composition pharmaceutique comprend en outre le peptide consistant en une séquence d'acides aminés telle que définie dans SEQ ID NO : 281.

11. La combinaison de peptides pour son utilisation selon la revendication 1, la combinaison de composés immunogènes pour son utilisation selon la revendication 2, la combinaison de nanoparticules pour son utilisation selon la revendication 3, la combinaison de cellules pour son utilisation selon la revendication 4, la composition pharmaceutique pour son utilisation selon l'une quelconque des revendications 5-10, dans laquelle le cancer des glandes surrénales est un phéochromocytome/paragangliome malin ou un carcinome corticosurrénalien.
